# EUROPEAN PATENT APPLICATION

(11) **EP 1 180 513 A1**
(43) Date of publication of application: **20.02.2002**
(21) Application number: 00921055.0
(22) Date of filing: 27.04.2000
(51) Int. Cl.: C07D 207/277, C07D 401/06, C07D 401/14, C07D 403/06, C07D 405/14, C07D 413/06, A61K 31/4015, A61K 31/454, A61K 31/4545, A61K 31/496, A61K 31/5377, A61K 31/55, A61P 31/18, A61P 43/00

(54) **CYCLIC AMIDE COMPOUNDS, PROCESS FOR THE PREPARATION OF THE SAME AND USES THEREOF**

(30) Priority: 28.04.1999 JP 12254999
(71) Applicant: Takeda Chemical Industries, Ltd., Osaka-shi, Osaka 541-0045 (JP)
(72) Inventor: ISHIHARA, Yuji, Tsukuba-shi, Ibaraki 305-0051 (JP); IMAMURA, Shinichi, Osaka-shi, Osaka 531-0063 (JP); HASHIGUCHI, Shohei, Toyonaka-shi, Osaka 560-0881 (JP); NISHIMURA, Osamu, Tsukuba-shi, Ibaraki 305-0812 (JP); KANZAKI, Naoyuki, Ibaraki-shi, Osaka 567-0867 (JP); BABA, Masanori, Kagoshima-shi, Kagoshima 891-0103 (JP)
(74) Representative: Lewin, John Harvey
(86) International application number: JP0002765
(87) International publication number: WO0066551

(57) **Abstract**

A compound of the formula: 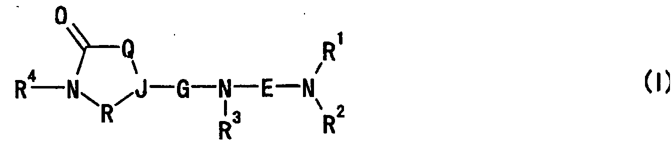 wherein R¹ is a hydrocarbon group, R² is a hydrocarbon group having 2 or more carbon atoms, where R¹ and R² may in combination form, together with an adjacent nitrogen atom, a ring optionally having a substituent or substituents, R³ is a hydrocarbon group optionally having a substituent or substituents or a heterocyclic group optionally having a substituent or substituents, R⁴ is a hydrogen atom, a hydrocarbon group, a heterocyclic group and the like, E is a divalent chain hydrocarbon group and the like, G is CO or SO₂, J is a nitrogen atom, a methine group and the like, and Q and R are each a divalent chain C₁₋₃ hydrocarbon group and the like, and a salt thereof show a superior CCR5 antagonistic activity and are useful as agents for the prophylaxis or treatment of HIV infection of human peripheral blood mononuclear cells, particularly AIDS.

## Description

### Technical Field

The present invention relates to cyclic amide compounds, which are useful for the treatment of acquired immunodeficiency syndrome, and their production and use.

### Background Art

HIV (human immunodeficiency virus) protease inhibitors have been developed in recent years for the treatment of AIDS (acquired immunodeficiency syndrome), and use of the protease inhibitors in combination with two conventional HIV reverse transcriptase inhibitors has provided dramatic progress in the treatment of AIDS. However, it is not sufficient for the eradication of AIDS, and the development of new anti-AIDS drugs having different activities and mechanisms are therefore required.

CD4 has long been known as a receptor from which HIV invades a target cell. Recently, CCR5 has been discovered as a second receptor of macrophage-tropic HIV and CXCR4 has been discovered as a second receptor for T-cell tropic HIV. These are G protein-coupled chemokine receptors having seven transmembrane domains. These chemokine receptors are thought to play an essential role in establishment and spread of HIV infection. In fact, it is reported that a person who is resistant to HIV infection in spite of several exposures retains mutation of homo deletion of CCR5 gene. Therefore, a CCR5 antagonist is expected to be a new anti-HIV drug.

As chemokine receptor antagonists, at present, there are known aromatic urea derivatives (J. Biol. Chem., 1998, 273, 10095-10098.), benzodiazepine derivatives (Japanese unexamined patent publication No.9-249570), cyclam derivatives (Nat. Med., 1998, 4, 72-77.), spiro piperidine derivatives (WO98/25604,25605,), acridine derivatives (WO98/30218), xanthene derivatives (WO98/04554), haloperidol derivatives (J.Biol.Chem.,1998,273,15687-15692., WO98/24325, 02151.), benzazocine-type compound (Japanese unexamined patent publication No.9-25572), benzimidazole derivatives (WO98/06703), piperazine and diazepine derivatives (WO97/44329), 3-disubstituted piperidine derivatives (Japanese unexamined patent publication No.9-249566), 4-substituted piperidine derivatives (WO99/04794), substituted pyrrolidine derivatives (WO99/09984), etc. However, so far, there has been no report that a CCR5 antagonist is developed as a therapeutic agent of AIDS.

Of the cyclic compounds containing a heteroatom and with regard to the physiological activity of pyrrolidinone derivatives, a compound having the structure expressed by the following formula (I) wherein Q=CH₂, R=CH₂, J=CH, G=CO, R³=H was reported to have a plant growth controlling or herbicide activity some time ago (JP-A-51-125745), an analgesic, antiinflammatory activity (Chim. Ther., 1972, 7, 398-403) and the like. However, there is not any report on a chemokine receptor antagonistic activity or a description of the present compound wherein R³≠H.

### Disclosure of the Invention

The present inventors diligently made extensive studies on compounds having CCR5 antagonistic activity and, as a result, they found that a compound shown by the formula (I) or a salt thereof shows superior CCR5 antagonistic activity and is useful as an agent for the prophylaxis or treatment of HIV infection of human peripheral blood mononuclear cells (especially AIDS), and also that the compound has superior absorbability when orally administered. Based on the finding, the present invention was accomplished.

Accordingly, the present invention provides the following.
[1] A compound of the formula: 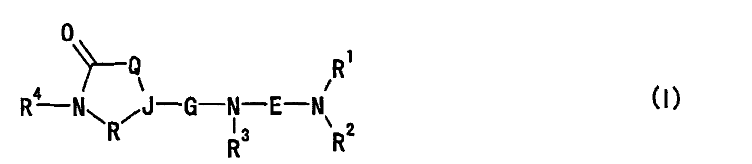 wherein
   - R¹: is a hydrocarbon group;
   - R²: is a hydrocarbon group having 2 or more carbon atoms, or R¹ and R² may in combination form, together with an adjacent nitrogen atom, a ring optionally having a substituent or substituents;
   - R³: is a hydrocarbon group optionally having a substituent or substituents or a heterocyclic group optionally having a substituent or substituents;
   - R⁴: is a hydrogen atom, a hydrocarbon group optionally having a substituent or substituents or a heterocyclic group optionally having a substituent or substituents;
   - E: is a divalent chain hydrocarbon group optionally having a substituent or substituents other than an oxo group;
   - G: is CO or SO₂;
   - J: is a nitrogen atom or a methine group optionally having a substituent or substituents; and
   - Q and R: are each a bond or a divalent chain C₁₋₃ hydrocarbon group optionally having a substituent or substituents,
   or a salt thereof.
[2] The compound of [1] above, wherein R¹ is a C₁₋₆ alkyl group or a C₃₋₈ cycloalkyl group; R² is a C₂₋₆ alkyl group or a C₃₋₈ cycloalkyl group, or R¹ and R² in combination form, together with an adjacent nitrogen atom, a ring optionally having a substituent or substituents; R³ is a C₁₋₆ alkyl group optionally having a substituent or substituents, a C₃₋₈ cycloalkyl group optionally having a substituent or substituents, an aryl group optionally having a substituent or substituents or a heterocyclic group optionally having a substituent or substituents; R⁴ is a hydrogen atom, alkyl group optionally having a substituent or substituents, a C₃₋₈ cycloalkyl group optionally having a substituent or substituents, an aryl group optionally having a substituent or substituents or a heterocyclic group optionally having a substituent or substituents; E is a C₂₋₅ alkylene group optionally having a substituent or substituents other than oxo group; G is CO or SO₂; J is a nitrogen atom or a methine group optionally having a substituent or substituents; and Q and R are each a bond or a C₁₋₃ alkylene group optionally having a substituent or substituents.
[3] The compound of [1] or [2] above, wherein R¹ and R² in combination form, together with an adjacent nitrogen atom, a ring optionally having a substituent or substituents.
[4] The compound of [3] above, wherein the ring optionally having a substituent or substituents is a 1-piperidinyl group or a 1-piperazinyl group each optionally having a substituent or substituents.
[5] The compound of [4] above, wherein the substituent of the 1-piperidinyl group or 1-piperazinyl group is (1) phenyl-C₁₋₄ alkyl optionally having halogen on a benzene ring, (2) diphenylmethyl optionally having hydroxy, (3) benzoyl optionally having halogen on a benzene ring, (4) 2-phenylethen-1-yl, (5) phenyl optionally having halogen, (6) hydroxy, (7) phenoxy or (8) benzyloxy.
[6] The compound of [3] above, wherein the ring optionally having a substituent or substituents is a 1-piperidinyl group optionally having a substituent or substituents.
[7] The compound of [6] above, wherein the substituent of the 1-piperidinyl group is a benzyl group optionally having halogen on a benzene ring.
[8] The compound of [1] or [2] above, wherein R³ is (1) a C₁₋₆ alkyl group, (2) a C₃₋₈ cycloalkyl group, (3) a benzyl group optionally having a hydroxy group, (4) a naphthylmethyl group, (5) a phenyl group optionally having, as a substituent, (a) C₁₋ ₄ alkyl optionally having halogen, (b) C₁₋₄ alkoxy optionally having halogen, (c) phenyl, (d) cyano, (e) benzyloxy or (f) a halogen atom, (6) a naphthyl group, (7) an indanyl group or (8) a tetrahydronaphthyl group.
[9] The compound of [1] or [2] above, wherein R³ is a phenyl group optionally having, as a substituent, C₁₋₄ alkyl or halogen.
[10] The compound of [1] or [2] above, wherein E is C₂₋₆ polymethylene optionally having hydroxy.
[11] The compound of [1] or [2] above, wherein R⁴ is (1) a hydrogen atom, (2) C₁₋₆ alkyl optionally having (a) halogen, (b) pyridyl, (c) morpholino, (d) furyl, (e) ethynyl or (f) C₃₋₈ cycloalkyl, (3) phenyl-C₁₋₄ alkyl optionally having (a) halogen, (b) C₁₋₄ alkyl, (c) halogeno-C₁₋₄ alkyl or (d) C₁₋₄ alkoxy on a benzene ring, or (4) C₃₋₈ cycloalkyl.
[12] The compound of [1] or [2] above, wherein R⁴ is (a) C₁₋₄ alkyl group optionally having, as a substituent, halogen or furyl or (b) a benzyl group optionally having halogen on a benzene ring.
[13] The compound of [1] above, wherein -N(R¹)R² is a 1-piperidinyl group optionally having a substituent or substituents, E is a trimethylene group, R³ is a phenyl group optionally having a substituent or substituents, G is CO, J is CH, and Q and R are each a methylene group.
[14] A compound selected from *N*-[3-(4-benzyl-1-piperidinyl)propyl]-*N*-(3,4-dichlorophenyl)-1-methyl-5-oxo-3-pyrrolidinecarboxamide, 1-benzyl-*N*-[3-(4-benzyl-1-piperidinyl)propyl]-5-oxo-*N*-phenyl-3-pyrrolidinecarboxamide, *N*-[3-(4-benzyl-1-piperidinyl)propyl]-1-(2-chlorobenzyl)-5-oxo-*N*-phenyl-3-pyrrolidinecarboxamide, *N*-(3,4-dichlorophenyl)-*N*-{3-[4-(4-fluorobenzyl)-1-piperidinyl]propyl}-1-methyl-5-oxo-3-pyrrolidinecarboxamide and *N*-[3-(4-benzyl-1-piperidinyl)propyl]-5-oxo-*N*-phenyl-1-(2,2,2-trifluoroethyl)-3-pyrrolidinecarboxamide, or a salt thereof.
[15] A prodrug of the compound of [1] above.
[16] A pharmaceutical composition containing the compound of [1] above or a prodrug thereof.
[17] The composition of [16] above, which is a chemokine receptor antagonist.
[18] The composition of [16] above, which is a CCR5 antagonist.
[19] The composition of [16] above, which is an agent for the prophylaxis or treatment of HIV infectious diseases.
[20] The composition of [16] above, which is an agent for the prophylaxis or treatment of AIDS.
[21] The composition of [16] above, which is an agent for suppressing the progress of a disease state of AIDS.
[22] The composition of [19] above, which further contains a protease inhibitor and/or a reverse transcriptase inhibitor in combination.
[23] The composition of [22] above, wherein the reverse transcriptase inhibitor is zidovudine, didanosine, zalcitabine, lamivudine, stavudine, abacavir, nevirapine, delavirdine or efavirenz.
[24] The composition of [22] above, wherein the protease inhibitor is saquinavir, ritonavir, indinavir, amprenavir or nelfinavir.
[25] Use of the compound of [1] above or a prodrug thereof, and a protease inhibitor and/or a reverse transcriptase inhibitor for the prophylaxis or treatment of HIV infectious diseases.
[26] A method for producing a compound of the formula (I) or a salt thereof, which method comprises reacting a compound of the formula: 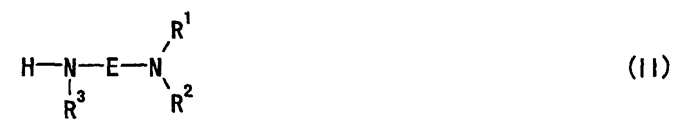 wherein each symbol is as defined above, or a salt thereof, and a compound of the formula: 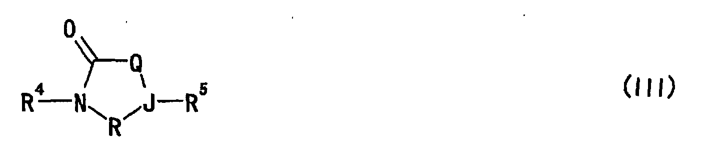 wherein R⁵ is a carboxyl group or a sulfonic acid group, a salt thereof or a reactive derivative thereof, and other symbols are as defined above, or a salt thereof.
[27] A method for producing a compound of the formula (I) or a salt thereof, which method comprises reacting, in the presence of a base, a compound of the formula: 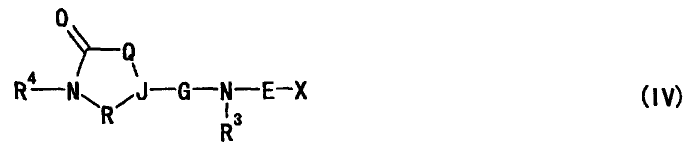 wherein X is a leaving group, and other symbols are as defined above, or a salt thereof and a compound of the formula: 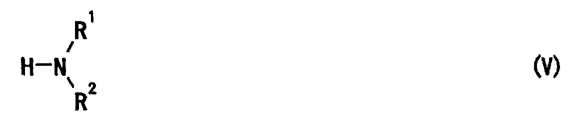 wherein each symbol is as defined above, or a salt thereof.
[28] A method for suppressing a chemokine receptor activity, which method comprises administering an effective amount of the compound of [1] above to a mammal.
[29] Use of a compound of [1] above for the production of a pharmaceutical agent that suppresses a chemokine receptor activity.

The hydrocarbon group represented by R¹ includes, for example, a chain aliphatic hydrocarbon group, an alicyclic hydrocarbon group, an aryl group and the like. Preferably, it is a chain aliphatic hydrocarbon group or an alicyclic hydrocarbon group.

The chain aliphatic hydrocarbon group includes, for example, a linear or branched aliphatic hydrocarbon group such as alkyl group, alkenyl group, alkynyl group and the like, with preference given to alkyl group. Examples of the alkyl group include C₁₋₁₀ alkyl groups, such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like (preferably C₁₋₆ alkyl etc.). Examples of the alkenyl group include C₂₋₆ alkenyl groups, such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Examples of the alkynyl group include C₂₋₆ alkynyl groups, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

Examples of the alicyclic hydrocarbon group include saturated or unsaturated alicyclic hydrocarbon groups, such as cycloalkyl group, cycloalkenyl group, cycloalkanedienyl group and the like, with preference given to cycloalkyl group. Examples of the cycloalkyl group include C₃₋₉ cycloalkyl (preferably C₃₋₈ cycloalkyl etc.), such as cyclopropyl, cyclobutyl, cyclopentyl, cyclohexyl, cycloheptyl, cyclooctyl, cyclononyl and the like, and condensed rings such as 1-indanyl, 2-indanyl and the like. Examples of the cycloalkenyl group include C₃₋₆ cycloalkenyl groups, such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl and the like. Examples of the cycloalkanedienyl group include C₄₋₆ cycloalkanedienyl groups, such as 2,4-cyclopentanedien-1-yl, 2,4-cyclohexanedien-1-yl, 2,5-cyclohexanedien-1-yl and the like.

Examples of the aryl group include monocyclic or condensed polycyclic aromatic hydrocarbon groups, such as C₆₋₁₄ aryl groups, which are preferably phenyl, naphthyl, anthryl, phenanthryl, acenaphthylenyl, 4-indanyl, 5-indanyl etc., and the like, with particular preference given to phenyl, 1-naphthyl, 2-naphthyl and the like.

The hydrocarbon group having 2 or more carbon atoms at R² includes, for example, the hydrocarbon groups at R¹ having 2 or more carbon atoms. Of those recited with regard to R¹, preferred are C₂₋₆ alkyl and C₃₋₈ cycloalkyl.

When R¹ and R² in combination form, together with an adjacent nitrogen atom, a ring optionally having a substituent or substituents, the ring may contain, besides one nitrogen atom, a different nitrogen atom, an oxygen atom and a sulfur atom. Examples thereof include monocyclic groups, such as 1-azetidinyl, 1-pyrrolidinyl, 1-piperidinyl, 1-homopiperidinyl, heptamethyleneimino, 1-piperazinyl, 1-homopiperazinyl, morpholino, thiomorpholino and the like, condensed rings such as 2-isoindolinyl, 1,2,3,4-tetrahydro-2-isoquinolyl, 1,2,4,5-tetrahydro-3H-3-benzodiazepin-3-yl and the like, cyclic amino groups such as spiro ring and the like (e.g., indene-1-spiro-4'-piperidin-1'-yl etc.), said cyclic amino group optionally having 1 to 5, preferably 1 to 3, substituent(s) at a chemically permitted position on the ring.

Examples of the substituent include hydroxy group, cyano group, nitro group, oxo group, halogen atom (e.g., fluorine atom, chlorine atom, bromine atom, iodine atom etc.), a group of the formula: -YR^{a} (wherein R^{a} is a hydrocarbon group optionally having a substituent or substituents or a heterocyclic group optionally having a substituent or substituents, Y is a bond (single bond), -CR^{b}R^{c}-, -COO-, -CO-, -CO-NR^{b}-, -CS-NR^{b}-, -CO-S-, -CS-S-, -CO-NR^{b}-CO-NR^{c}-, -C(=NH)-NR^{b}-, -NR^{b}-, -NR^{b}-CO-, -NR^{b}-CS-, -NR^{b}-CO-NR^{c}-, -NR^{b}-CS-NR^{c}-, -NR^{b}-CO-O-, -NR^{b}-CS-O-, -NR^{b}-CO-S-, -NR^{b}-CS-S-, -NR^{b}-C(=NH)-NR^{c}-, -NR^{b}-SO₂-, -NR^{b}-NR^{c}-, -O-, -O-CO-, -O-CS-, -O-CO-O, -O-CO-NR^{b}-, -O-C(=NH)-NR^{b}-, -S-, -SO-, -SO₂-, -SO₂-NR^{b}-, -S-CO-, -S-CS-, -S-CO-NR^{b}-, -S-CS-NR^{b}-, -S-C(=NH)-NR^{b}- and the like, wherein R^{b} and R^{c} are each a hydrogen atom, alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, acyl group derived from sulfonic acid, acyl group derived from carboxylic acid etc.), and the like.

The "hydrocarbon group" of the hydrocarbon group optionally having a substituent or substituents at R^{a} is exemplified by chain aliphatic hydrocarbon group, alicyclic hydrocarbon group, aryl group and the like. As these chain aliphatic hydrocarbon group, alicyclic hydrocarbon group, aryl group, those exemplified as the chain aliphatic hydrocarbon group, alicyclic hydrocarbon group and aryl group at R¹ can be used. Examples of the substituent of the hydrocarbon group include those exemplified as the substituents for the "hydrocarbon group optionally having a substituent or substituents" at R³ to be mentioned later.

As the "heterocyclic group optionally having a substituent or substituents" at the aforementioned R^{a}, those exemplified as the "heterocyclic group optionally having a substituent or substituents" at R³ to be mentioned later can be recited. As the alkyl group optionally having a substituent or substituents, alkenyl group optionally having a substituent or substituents, alkynyl group optionally having a substituent or substituents, aryl group optionally having a substituent or substituents, cycloalkyl group or cycloalkenyl group optionally having a substituent or substituents, heterocyclic group optionally having a substituent or substituents, acyl group derived from carboxylic acid, alkyl sulfonyl group optionally having a substituent or substituents, and arylsulfonyl group optionally having a substituent or substituents, as expressed by the aforementioned R^{b} and R^{c}, those exemplified as the substituent of the hydrocarbon group optionally having a substituent or substituents at R³ to be mentioned later are exemplified.

It is preferable that R¹ and R² in combination form, together with an adjacent nitrogen atom, a heterocycle optionally having a substituent or substituents.

More preferably, NR¹R² is a group of the formula: 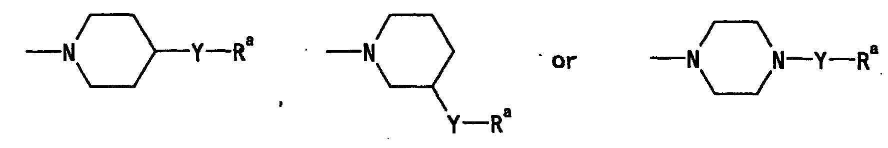 wherein Y and R^{a} are as defined above. As used here, Y and R^{a} are as defined above, and R^{a} is particularly preferably an aryl group optionally having a substituent or substituents or a heterocyclic group optionally having a substituent or substituents.

YR^{a} is particularly preferably a benzyl group optionally having a substituent or substituents.

NR¹R² is particularly preferably a 4-benzyl-1-piperidinyl group optionally having a substituent or substituents.

As the hydrocarbon group of the hydrocarbon group optionally having a substituent or substituents at R³, there are mentioned, for example, those similar to the hydrocarbon groups at R¹, with particular preference given to C₁₋₆ alkyl group, C₃₋₈ cycloalkyl group and aryl group. These are exemplified by those recited for R¹.

The heterocyclic group of the heterocyclic group optionally having a substituent or substituents at R³ is, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom, a nitrogen atom and the like.

Examples of the aromatic heterocyclic group include aromatic monocyclic heterocyclic group (e.g., 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.); condensed aromatic heterocyclic group [e.g., 8 to 12-membered condensed aromatic heterocyclic group (preferably a heterocycle wherein the aforementioned 5-or 6-membered aromatic monocyclic heterocyclic group is condensed with a benzene ring or a heterocycle wherein the same or different two heterocycles of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the non-aromatic heterocyclic group include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

Examples of the substituent of the hydrocarbon group optionally having a substituent or substituents as expressed by R³ and the substituent of the heterocyclic group optionally having a substituent or substituents as expressed by R³ include alkyl group optionally having a substituent or substituents, alkenyl group optionally having a substituent or substituents, alkynyl group optionally having a substituent or substituents, an aryl group optionally having a substituent or substituents, cycloalkyl group or cycloalkenyl group optionally having a substituent or substituents, a heterocyclic group optionally having a substituent or substituents, amino group optionally having a substituent or substituents, imidoyl group optionally having a substituent or substituents, amidino group optionally having a substituent or substituents, hydroxy group optionally having a substituent or substituents, thiol group optionally having a substituent or substituents, optionally esterified carboxyl group, carbamoyl group optionally having a substituent or substituents, thiocarbamoyl group optionally having a substituent or substituents, sulfamoyl group optionally having a substituent or substituents, halogen atom (e.g., fluorine, chlorine, bromine, iodine etc., preferably chlorine, bromine etc.), cyano group, nitro group, acyl group derived from carboxylic acid, alkyl sulfinyl group optionally having a substituent or substituents, alkyl sulfonyl group optionally having a substituent or substituents, arylsulfinyl group optionally having a substituent or substituents, arylsulfonyl group optionally having a substituent or substituents and the like, wherein 1 to 5 (preferably 1 to 3) of these optional substituents may be present at a substitutable position.

The aryl group of the "aryl group optionally having a substituent or substituents" as a substituent may be, for example, C₆₋₁₄ aryl group such as phenyl, naphthyl, anthryl, phenanthryl, acenaphthylenyl etc., and the like. Here, the substituent of the aryl group includes, for example, lower alkoxy group (e.g., C₁₋₆ alkoxy group such as methoxy, ethoxy, propoxy etc., and the like), halogen atom (e.g., fluorine, chlorine, bromine, iodine etc.), lower alkyl group (e.g., C₁₋₆ alkyl group such as methyl, ethyl, propyl etc., etc.), amino group, hydroxy group, cyano group, amidino group and the like, wherein one or two of these optional substituents may be present at a substitutable position.

The cycloalkyl group of the "cycloalkyl group optionally having a substituent or substituents" as a substituent may be, for example, C₃₋₇ cycloalkyl group, such as cyclopropyl, cyclobutyl, cyclopentyl, cyclohexyl, cycloheptyl etc., and the like. As used herein, examples of the substituent of the "cycloalkyl group" are similar in the kind and the number to those exemplified for the substituent of the aforementioned "aryl group optionally having a substituent or substituents".

The cycloalkenyl group of the "cycloalkenyl group optionally having substituents" as a substituent may be, for example, C₃₋₆ cycloalkenyl group such as cyclopropenyl, cyclobutenyl, cyclopentenyl, cyclohexenyl etc., and the like. As used herein, examples of the substituent of the "cycloalkenyl group optionally having a substituent or substituents" are similar in the kind and the number to those exemplified for the substituent of the aforementioned "aryl group optionally having a substituent or substituents".

The alkyl group of the "alkyl group optionally having a substituent or substituents" as a substituent may be, for example, C₁₋₆ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl etc., and the like. As used herein, examples of the substituent of the alkyl group are similar in the kind and the number to those exemplified for the substituent of the aforementioned "aryl group optionally having a substituent or substituents".

The alkenyl group of the "alkenyl group optionally having a substituent or substituents" as a substituent may be, for example, C₂₋₆alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc., and the like. As used herein, examples of the substituent of the alkenyl group are similar in the kind and the number to those exemplified for the substituent of the aforementioned "aryl group optionally having a substituent or substituents".

The alkynyl group of the "alkynyl group optionally having a substituent or substituents" as a substituent may be, for example, C₂₋₆ alkynyl group, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. As used herein, examples of the substituent of the alkynyl group are similar in the kind and the number to those exemplified for the substituent of the aforementioned "aryl group optionally having a substituent or substituents".

The heterocyclic group of the "heterocyclic group optionally having a substituent or substituents" as a substituent may be, for example, an aromatic heterocyclic group, a saturated or unsaturated non-aromatic heterocyclic group (aliphatic heterocyclic group) and the like, containing, as an atom (cyclic atom) constituting the ring system, at least one (preferably 1 to 4, more preferably 1 or 2) of 1 to 3 kinds (preferably 1 or 2 kinds) of the hetero atom selected from an oxygen atom, a sulfur atom and a nitrogen atom, and the like.

Examples of the "aromatic heterocyclic group" include aromatic monocyclic heterocyclic group (e.g., 5- or 6-membered aromatic monocyclic heterocyclic group, such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.) and condensed aromatic heterocyclic group [e.g., 8 to 12-membered condensed aromatic heterocycle (preferably a heterocycle wherein the aforementioned 5- or 6-membered aromatic monocyclic heterocyclic group is condensed with a benzene ring or a heterocycle wherein the same or different two heterocycle of the aforementioned 5- or 6-membered aromatic monocyclic heterocyclic group are condensed), such as benzofuranyl, isobenzofuranyl, benzothienyl, indolyl, isoindolyl, 1H-indazolyl, benzindazolyl, benzooxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, Phenoxathiinyl, thianthrenyl, phenanthridinyl, phenanthrolinyl, indolizinyl, pyrro[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl etc.] and the like.

Examples of the "non-aromatic heterocyclic group" include 3 to 8-membered (preferably 5- or 6-membered) saturated or unsaturated (preferably saturated) non-aromatic heterocyclic group (aliphatic heterocyclic group), such as oxiranyl, azetidinyl, oxetanyl, thietanyl, pyrrolidinyl, tetrahydrofuryl, thioranyl, piperidinyl, tetrahydropyranyl, morpholinyl, thiomorpholinyl, piperazinyl etc., and the like.

The substituent that the "heterocyclic group optionally having a substituent or substituents" as a substituent may have is exemplified by lower alkyl group (e.g., C₁₋₆ alkyl group, such as methyl, ethyl, propyl etc., and the like), acyl group (e.g., C₁₋₆ alkanoyl, such as formyl, acetyl, propionyl, pivaloyl etc., benzoyl etc.), and the like.

The substituent of the "amino group optionally having a substituent or substituents", "imidoyl group optionally having a substituent or substituents", "amidino group optionally having a substituent or substituents", "hydroxy group optionally having a substituent or substituents" and "thiol group optionally having a substituent or substituents" as a substituent may be, for example, lower alkyl group (e.g., C₁₋₆ alkyl group, such as methyl, ethyl, propyl, isopropyl, butyl, isobutyl, t-butyl, pentyl, hexyl etc., and the like), acyl group (e.g., C₁₋₆ alkanoyl (e.g., formyl, acetyl, propionyl, pivaloyl etc.), benzoyl etc.), C₁₋₆ alkyl sulfonyl (e.g., methanesulfonyl, ethanesulfonyl etc.), C₃₋₁₄ arylsulfonyl (e.g., benzenesulfonyl, p-toluenesulfonyl etc.), optionally halogenated C₁₋₆ alkoxy-carbonyl (e.g., trifluoromethoxycarbonyl, 2,2,2-trifluoroethoxycarbonyl, trichloromethoxycarbonyl, 2,2,2-trichloroethoxycarbonyl etc.), and the like. The "amino group" of the "amino group optionally having a substituent or substituents" as the substituent may be substituted by imidoyl group optionally having a substituent or substituents (e.g., C₁₋₆ alkyl imidoyl, formylimidoyl, amidino etc.), and the like. In addition, two substituents may form a cyclic amino group together with a nitrogen atom. In this case, examples of the cyclic amino group include 3 to 8-membered (preferably 5- or 6-membered) cyclic amino, such as 1-azetidinyl, 1-pyrrolidinyl, 1-piperidinyl, morpholino, 1-piperazinyl and 1-piperazinyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀aralkyl group, such as benzyl, phenethyl etc., and the like), aryl group (e.g., C₆₋₁₀ aryl group, such as phenyl, 1-naphthyl, 2-naphthyl etc., and the like), and the like.

Examples of the "carbamoyl group optionally having a substituent or substituents" include unsubstituted carbamoyl, N-monosubstituted carbamoyl group and N,N-disubstituted carbamoyl group.

The "N-monosubstituted carbamoyl group" is a carbamoyl group having one substituent on the nitrogen atom. Examples of the substituent include lower alkyl group (e.g., C₁₋₆ alkyl group, such as methyl, ethyl, propyl, isopropyl, butyl, isobutyl, t-butyl, pentyl, hexyl etc., and the like), cycloalkyl group (e.g., C₃₋₆ cycloalkyl group, such as cyclopropyl, cyclobutyl, cyclopentyl, cyclohexyl etc., and the like), aryl group (e.g., C₆₋₁₀ aryl group, such as phenyl, 1-naphthyl, 2-naphthyl etc., and the like), aralkyl group (e.g., C₇₋₁₀ aralkyl group, such as benzyl, phenethyl etc., preferably phenyl-C₁₋₄ alkyl group etc.), heterocyclic group (e.g., those exemplified as the "heterocyclic group" as a substituent of "hydrocarbon group optionally having a substituent or substituents" at R³ and the like). The lower alkyl group, cycloalkyl group, aryl group, aralkyl group and heterocyclic group may have substituents, which substituents are, for example, hydroxy group, amino group optionally having a substituent or substituents [which amino group optionally having 1 or 2 from lower alkyl group (e.g., C₁₋₆ alkyl group such as methyl, ethyl, propyl, isopropyl, butyl, isobutyl, t-butyl, pentyl, hexyl etc., and the like), acyl group (e.g., C₁₋₆ alkanoyl such as formyl, acetyl, propionyl, pivaloyl etc., benzoyl, etc.), and the like as substituents], halogen atom (e.g., fluorine, chlorine, bromine, iodine etc.), nitro group, cyano group, lower alkoxy group optionally having 1 to 5 halogen atoms as substituents (e.g., fluorine, chlorine, bromine, iodine etc.) lower alkyl group optionally having 1 to 5 halogen atoms as substituents (e.g., fluorine, chlorine, bromine, iodine etc.), and the like. Examples of the lower alkyl group include C₁₋₆ alkyl group, such as methyl, ethyl, n-propyl, isopropyl, n-butyl, isobutyl, sec-butyl, tert-butyl, pentyl, hexyl etc., and the like, particularly preferably methyl, ethyl and the like. Examples of the lower alkoxy group include C₁₋₆ alkoxy group, such as methoxy, ethoxy, n-propoxy, isopropoxy, n-butoxy, isobutoxy, sec-butoxy, tert-butoxy etc., and the like, particularly preferably methoxy, ethoxy and the like. These substituents preferably have the same or different, 1 or 2 or 3 (preferably 1 or 2) substituents.

The "N,N-disubstituted carbamoyl group" is a carbamoyl group having 2 substituents on a nitrogen atom. Examples of one of the substituents are those similar to the substituents of the aforementioned "N-monosubstituted carbamoyl group" and examples of the other include lower alkyl group (e.g., C₁₋₆ alkyl group, such as methyl, ethyl, propyl, isopropyl, butyl, t-butyl, pentyl, hexyl etc., and the like), C₃₋₆ cycloalkyl group (e.g., cyclopropyl, cyclobutyl, cyclopentyl, cyclohexyl etc.), C₇₋₁₀ aralkyl group (e.g., benzyl, phenethyl etc., preferably phenyl-C₁₋₄ alkyl group etc.) and the like. Two substituents may form a cyclic amino group together with a nitrogen atom. In this case, examples of the cyclic aminocarbamoyl group include 3 to 8-membered (preferably 5- or 6-membered) cyclic amino such as 1-azetidinylcarbonyl, 1-pyrrolidinylcarbonyl, 1-piperidinylcarbonyl, morpholinocarbonyl, 1-piperazinylcarbonyl and 1-piperazinylcarbonyl optionally having, at the 4-position, lower alkyl group (e.g., C₁₋₆ alkyl group, such as methyl, ethyl, propyl, isopropyl, butyl, t-butyl, pentyl, hexyl etc., and the like), aralkyl group (e.g., C₇₋₁₀ aralkyl group, such as benzyl, phenethyl etc., and the like), aryl group (e.g., C₆₋₁₀ aryl group, such as phenyl, 1-naphthyl, 2-naphthyl etc., and the like), and the like.

Examples of the substituent of the "thiocarbamoyl group optionally having a substituent or substituents" are similar to those exemplified for the substituent of the aforementioned "carbamoyl group optionally having a substituent or substituents".

Examples of the "sulfamoyl group optionally having a substituent or substituents" include unsubstituted sulfamoyl, N-monosubstituted sulfamoyl group and N,N-disubstituted sulfamoyl group.

The "N-monosubstituted sulfamoyl group" means sulfamoyl group having one substituent on a nitrogen atom. Examples of the substituent are those similar to the substituent of the "N-monosubstituted carbamoyl group".

The "N,N-disubstituted sulfamoyl group" means sulfamoyl group having 2 substituents on a nitrogen atom. Examples of the substituent are those similar to the substituent of the "N,N-disubstituted carbamoyl group".

Examples of the "optionally esterified carboxyl group" include, besides free carboxyl group, lower alkoxy carbonyl group, aryloxycarbonyl group, aralkyloxycarbonyl group and the like.

Examples of the "lower alkoxy carbonyl group" include C₁₋ ₆ alkoxy-carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl etc., and the like. Of these, C₁₋₃ alkoxy-carbonyl group, such as methoxycarbonyl, ethoxycarbonyl, propoxycarbonyl etc., and the like are preferable.

Examples of the "aryloxycarbonyl group" preferably include C₇₋₁₂ aryloxy-carbonyl group, such as phenoxycarbonyl, 1-naphthoxycarbonyl, 2-naphthoxycarbonyl etc., and the like.

Examples of the "aralkyloxycarbonyl group" preferably include C₇₋₁₀ aralkyloxy-carbonyl group such as benzyloxycarbonyl, phenethyloxycarbonyl etc., and the like (preferably C₆₋₁₀ aryl-C₁₋₄ alkoxy-carbonyl etc.).

The "aryloxycarbonyl group" and "aralkyloxycarbonyl group" may have substituents. Examples of the substituent are similar in the kind and the number to those exemplified for the substituent of aryl group and aralkyl group as the substituents of the aforementioned N-monosubstituted carbamoyl group.

The "acyl group derived from carboxylic acid" as the substituent is exemplified by one wherein a hydrogen atom or the single substituent that the aforementioned "N-monosubstituted carbamoyl group" has on a nitrogen atom is bonded to carbonyl, and the like. Preferred are acyl, such as benzoyl and C₁₋₆ alkanoyl, e.g., formyl, acetyl, propionyl, pivaloyl etc., and the like.

The alkyl of the "alkyl sulfinyl group optionally having a substituent or substituents" and "alkyl sulfonyl group optionally having a substituent or substituents" as the substituent may be, for example, lower alkyl group such as C₁₋₆ alkyl group (e.g., methyl, ethyl, propyl, isopropyl, butyl, isobutyl, t-butyl, pentyl, hexyl etc.), and the like.

The aryl of the "arylsulfinyl group optionally having a substituent or substituents" and "arylsulfonyl group optionally having a substituent or substituents" as the substituent may be, for example, C₆₋₁₄ aryl group, such as phenyl, naphthyl, anthryl, phenanthryl, acenaphthylenyl etc., and the like.

The substituent of these alkyl and aryl may be, for example, lower alkoxy group (e.g., C₁₋₆ alkoxy group, such as methoxy, ethoxy, propoxy etc., and the like), halogen atom (e.g., fluorine, chlorine, bromine, iodine etc.), lower alkyl group (e.g., C₁₋₆ alkyl group, such as methyl, ethyl, propyl etc., and the like), amino group, hydroxy group, cyano group, amidino group and the like, wherein one or two of these optional substituents may be present at a substitutable position.

The hydrocarbon group optionally having a substituent or substituents as expressed by R⁴ is exemplified by those shown with regard to hydrocarbon group optionally having a substituent or substituents as expressed by R³, and the heterocyclic group optionally having a substituent or substituents as expressed by R⁴ is exemplified by those shown with regard to the heterocyclic group optionally having a substituent or substituents as expressed by R³.

The divalent chain hydrocarbon group of the divalent chain hydrocarbon group optionally having a substituent or substituents other than oxo group, as expressed by E, is exemplified by C₁₋₆ alkylene, such as methylene, ethylene etc., C₂₋₆ alkenylene, such as ethenylene etc., C₂₋₆ alkynylene, such as ethynylene etc., and the like. Preferred is C₁₋₅ alkylene and more preferred is trimethylene.

The substituent of the divalent hydrocarbon group may be any as long as it is not an oxo group. Examples thereof include alkyl group optionally having a substituent or substituents, an aryl group optionally having a substituent or substituents, cycloalkyl group or cycloalkenyl group optionally having a substituent or substituents, optionally esterified carboxyl group, carbamoyl group or thiocarbamoyl group optionally having a substituent or substituents, amino group optionally having a substituent or substituents, hydroxy group optionally having a substituent or substituents, thiol (mercapto) group optionally having a substituent or substituents, acyl group derived from carboxylic acid, alkyl sulfonyl group optionally having a substituent or substituents, arylsulfonyl group optionally having a substituent or substituents, halogen (e.g., fluorine, chlorine, bromine etc.), nitro, cyano and the like. The number of the substituents may be 1 to 3. The alkyl group optionally having a substituent or substituents, an aryl group optionally having a substituent or substituents, cycloalkyl group or cycloalkenyl group optionally having a substituent or substituents, carboxyl group optionally having an esterified group, carbamoyl group or thiocarbamoyl group optionally having a substituent or substituents, amino group optionally having a substituent or substituents, hydroxy group optionally having a substituent or substituents, thiol (mercapto) group optionally having a substituent or substituents, acyl group derived from carboxylic acid, alkyl sulfonyl group optionally having a substituent or substituents, arylsulfonyl group optionally having a substituent or substituents are those similar to the substituent of the heterocyclic group optionally having a substituent or substituents as expressed by the aforementioned R³.

Examples of the substituent of the methine group optionally having a substituent or substituents expressed by J are those similar to the substituent of the heterocyclic group optionally having a substituent or substituents expressed by the aforementioned R³.

The divalent chain C₁₋₃ hydrocarbon group of the divalent chain C₁₋₃ hydrocarbon group optionally having a substituent or substituents, as expressed by Q and R, is exemplified by one having 1 to 3 carbon atoms from the divalent chain hydrocarbon group of the divalent chain hydrocarbon group optionally having a substituent or substituents other than oxo group, as expressed by E.

The substituent of the divalent chain C₁₋₃ hydrocarbon group optionally having a substituent or substituents, as expressed by Q and R, is exemplified by those exemplified as the substituent of the divalent chain hydrocarbon group optionally having a substituent or substituents other than oxo group, as expressed by E.

The salt of the carboxyl group or sulfonic acid group, as expressed by R⁵, is exemplified by salts with alkali metal, such as sodium, potassium, lithium etc., salts with alkaline earth metal, such as calcium, magnesium, strontium etc., ammonium salt and the like.

As the reactive derivative of the carboxyl group, as expressed by R⁵, a reactive derivative, such as acid halide, acid azide, acid anhydride, mixed acid anhydride, active amide, active ester, active thio ester and the like, is subjected to an acylation reaction. The acid halide is exemplified by acid chloride, acid bromide etc., mixed acid anhydride is exemplified by mono C₁₋₆ alkyl carbonate mixed acid anhydride (e.g., mixed acid anhydride of free acid and monomethyl carbonate, monoethyl carbonate, monoisopropyl carbonate, monoisobutyl carbonate, mono tert-butyl carbonate, monobenzyl carbonate, mono(p-nitrobenzyl) carbonate, monoallyl carbonate etc.), C₁₋₆ aliphatic carboxylic mixed acid anhydride (e.g., mixed acid anhydride of free acid and acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid etc.), C₇₋₁₂ aromatic carboxylic mixed acid anhydride (e.g., mixed acid anhydride of free acid and benzoic acid, p-toluic acid, p-chlorobenzoic acid etc.), organic sulfonic mixed acid anhydride (e.g., mixed acid anhydride of free acid and methanesulfonic acid, ethanesulfonic acid, benzenesulfonic acid, p-toluenesulfonic acid etc.) and the like, active amide is exemplified by amide with heterocyclic compound containing nitrogen [e.g., acid amide of free acid and pyrazole, imidazole, benzotriazole etc., these heterocyclic compounds containing nitrogen being optionally substituted by C₁₋₆ alkyl group (e.g., methyl, ethyl etc.), C₁₋₆ alkoxy group (e.g., methoxy, ethoxy etc.), halogen atom (e.g., fluorine, chlorine, bromine etc.), oxo group, thioxo group, C₁₋₆ alkylthio group (e.g., methylthio, ethylthio etc.), etc.] and the like.

As the active ester, any can be used as long as it is used for this purpose in the field of β-lactam and peptide synthesis. For example, organic phosphates (e.g., diethoxyphosphate, diphenoxy phosphate etc.), p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester and the like are mentioned. Examples of the active thio ester include esters with aromatic heterocyclic thiol compound, such as 2-pyridylthiol ester, 2-benzothiazolylthiol ester and the like, wherein these heterocycles may be substituted by C₁₋₆ alkyl group (e.g., methyl, ethyl etc.), C₁₋₆ alkoxy group (e.g., methoxy, ethoxy etc.), halogen atom (e.g., fluorine, chlorine, bromine etc.), C₁₋₆ alkyl thio group (e.g., methylthio, ethylthio etc.) and the like.

Examples of the reactive derivative of the sulfonic acid group expressed by R⁵ include sulfonyl halide (e.g., sulfonyl chloride, sulfonyl bromide etc.), sulfonyl azide, acid anhydride thereof, and the like.

Examples of the leaving group expressed by X include halogen atom (e.g., chlorine atom, bromine atom, iodine atom etc.), alkyl or arylsulfonyloxy group (e.g., methanesulfonyloxy, ethanesulfonyloxy, benzenesulfonyloxy, p-toluenesulfonyloxy etc.), and the like.

Examples of the salt of the compound of the formula (I) of the present invention include acid addition salt, such as inorganic acid salts (e.g., hydrochloride, sulfate, hydrobromate, phosphate etc.), organic acid salts (e.g., acetate, trifluoroacetate, succinate, maleate, fumarate, propionate, citrate, tartrate, lactate, oxalate, methanesulfonate, p-toluenesulfonate etc.) and the like. The compound may form salts with a base (e.g., alkali metal salts such as potassium salt, sodium salt, lithium salt etc., alkaline earth metal salts, such as calcium salt, magnesium salt etc. and salts with organic base such as ammonium salt, trimethylamine salt, triethylamine salt, tert-butyldimethylamine salt, dibenzylmethylamine salt, benzyldimethylamine salt, N,N-dimethylaniline salt, pyridine salt, quinoline salt etc).

The compound of the formula (I) and a salt thereof may be a hydrate, all of which including salts and hydrates, are to be referred to as compound (I) in the following.

The prodrug of the compound (I) means a compound that is converted to compound (I) in the body by reaction with an enzyme, gastric acid and the like.

Examples of the prodrug of compound (I) when the compound (I) has an amino group include compounds wherein the amino group is acylated, alkylated or phosphorated (e.g., compound wherein the amino group of compound (I) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated etc.); when compound (I) has a hydroxy group, a compound wherein the hydroxy group is acylated, alkylated, phosphorated or borated [e.g., compound wherein the hydroxy group of compound (I) is acetylated, palmitoylated, propanoylated, pivaloylated, succinylated, fumarylated, alanylated, dimethylaminomethylcarbonylated etc.]; when compound (I) has a carboxyl group, a compound wherein the carboxyl group is esterified, amidated (e.g., carboxyl group of compound (I) ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, methylamidated etc.); and the like. These compounds can be produced by a method known *per se*.

The prodrug of compound (I) may be of a kind that changes to compound (I) under physiological conditions, as described in *Iyakuhin no Kaihatsu,* vol. 7, Molecular Design pp. 163-198, Hirokawa Shoten (1990).

The prodrug of compound (I) may be as it is or a pharmacologically acceptable salt. Examples of such salt include, when the prodrug of compound (I) has an acidic group, such as carboxyl group etc., salts with inorganic base (e.g., alkali metal such as sodium, potassium etc., alkaline earth metal such as calcium, magnesium etc., transition metal such as zinc, iron, copper etc., and the like), salts with organic base (e.g., organic amines such as trimethylamine, triethylamine, pyridine, picoline, ethanolamine, diethanolamine, triethanolamine, dicyclohexylamine, N,N'-dibenzylethylenediamine etc., basic amino acids such as arginine, lysine, ornithine etc., etc.), and the like.

When the prodrug of compound (I) has a basic group, such as amino group and the like, the salt is exemplified by salts with inorganic acid and organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), salts with acidic amino acid, such as aspartic acid, glutamic acid etc., and the like.

The prodrug of compound (I) may be a hydrate or a non-hydrate.

While it has one or more asymmetric carbon(s) in a molecule, both an R configuration compound and an S configuration compound due to the asymmetric carbons are encompassed in the present invention.

In the present specification, the "lower" of the lower alkyl group, lower alkoxy group and the like means chain, branched or cyclic carbon chain having 1 to 6 carbon atoms, unless particularly specified.

The compounds of the formulas (II) to (VI), a compound having a basic group or an acidic group can form a salt with an acid addition salt or a salt with a base. The salts with these acid addition salts and bases are exemplified by those recited with regard to the aforementioned compound (I). In the following, the compounds of the respective formulas, inclusive of salts thereof, are to be briefly referred to as a compound (symbol of the formula). For example, a compound of the formula (II) and a salt thereof are simply referred to as compound (II).

The compound (I) can be produced by, for example, the following method and the like.

### Production Method 1

As shown in the following formulas, compound (II) and compound (III) are reacted to produce compound (I). 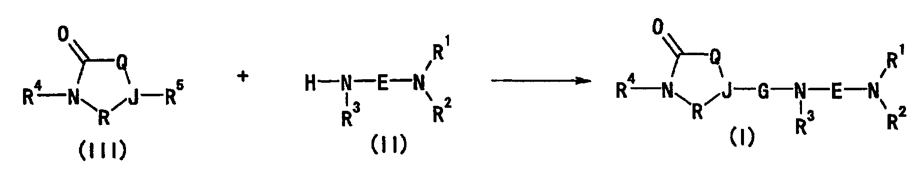 wherein each symbol is as defined above.

This reaction generally proceeds in a solvent inert to the reaction. Examples of the solvent include ether solvents (e.g., ethyl ether, diisopropyl ether, dimethoxyethane, tetrahydrofuran, dioxane etc.), halogen solvents (e.g., dichloromethane, dichloroethane, chloroform etc.), aromatic solvents (e.g., toluene, chlorobenzene, xylene etc.), acetonitrile, N,N-dimethylformylamide (DMF), acetone, methyl ethyl ketone, dimethyl sulfoxide (DMSO), water and the like, which are used alone or in combination. Of these, acetonitrile, dichloromethane, chloroform and the like are preferable. This reaction is generally carried out by reacting 1 to 5 equivalents, preferably 1 to 3 equivalents, of compound (III) with compound (II). The reaction temperature is from -20°C to 50°C, preferably 0°C to room temperature, and the reaction time is generally from 5 min to 100 h. In this reaction, a co-presence of a base sometimes affords smooth progress of the reaction. As the base, both inorganic bases and organic bases are effective. Examples of the inorganic base include hydroxide, hydride, carbonate, hydrogencarbonate, organic acid salt and the like of alkali metals and alkaline earth metals. Particularly, potassium carbonate, sodium carbonate, sodium hydroxide, potassium hydroxide, sodium bicarbonate and potassium bicarbonate are preferable. As the organic base, tertiary amines such as triethylamine and the like are preferable. Examples of the reactive derivative include acid anhydride, acid halide (e.g., acid chloride and acid bromide), active ester and the like, with preference given to acid halide. The amount of use of the base is generally 1 to 10 equivalents, preferably 1 to 3 equivalents, relative to compound (II).

In the case of acylation from carboxylic acid, 1 equivalent of compound (II) is reacted with 1 to 1.5 equivalents of carboxylic acid in an inert solvent (e.g., halogen solvent and acetonitrile) in the presence of 1 to 1.5 equivalents of a dehydrative condensing agent such as dicyclohexylcarbodiimide (DCC) and the like. This reaction generally proceeds at room temperature where the reaction time is 0.5 to 24 h.

In compound (II) to be used for this method, the divalent chain hydrocarbon group optionally substituted by a group other than oxo group, as expressed by E, is a group of the formula: 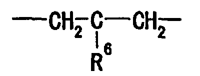 wherein R⁶ is a substituent other than oxo group, for example, the compound can be produced by a method described in Synthetic Comm., 1991, 20, 3167-3180. That is, utilizing the addition reaction of amineamides to unsaturated bond, the following method is employed for the production. 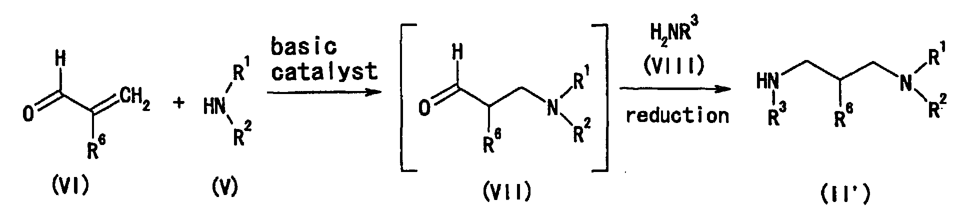 wherein each symbol is a as defined above.

The substituent other than oxo group expressed by R⁶ means the substituent other than oxo group of the divalent chain hydrocarbon group optionally having a substituent or substituents other than oxo group, as expressed by E.

The compound can be obtained by reacting acrolein derivative (VI) and compound (V) and then reacting the obtained product with compound (VIII) under reducing conditions. The reaction between compound (VI) and compound (V) is generally carried out in a solvent inert to the reaction in the presence of a base. Examples of the base include 1) strong base such as hydride of alkali metal or alkaline earth metal (e.g., lithium hydride, sodium hydride, potassium hydride, calcium hydride etc.), amide of alkali metal or alkaline earth metal (e.g., lithiumamide, sodiumamide, lithium diisopropylamide, lithium dicyclohexylamide, lithium hexamethylsilazide, sodium hexamethylsilazide, potassium hexamethylsilazide etc.), lower alkoxide of alkali metal or alkaline earth metal (e.g., sodium methoxide, sodium ethoxide, potassium t-butoxide etc.) and the like, 2) inorganic base such as hydroxide of alkali metal or alkaline earth metal (e.g., sodium hydroxide, potassium hydroxide, lithium hydroxide, barium hydroxide etc.), carbonate of alkali metal or alkaline earth metal (e.g., sodium carbonate, potassium carbonate, cesium carbonate etc.), hydrogencarbonate of alkali metal or alkaline earth metal (e.g., sodium hydrogencarbonate, potassium hydrogencarbonate etc.) and the like, 3) organic base and the like such as amines [e.g., triethylamine, diisopropylethylamine, N-methylmorpholine, dimethylaminopyridine, DBU (1,8-diazabicyclo[5.4.0]-7-undecen), DBN (1,5-diazabicyclo[4.3.0]-non-5-en) etc.] and basic heterocyclic compound (e.g., pyridine, imidazole, 2,6-lutidine etc.), and the like. Examples of the solvent include those recited for the reaction of the aforementioned compound (II) and compound (III), which can be used alone or in combination. By this reaction, compound (VII) is obtained.

Examples of the reducing agent to be used for the reaction of compound (VII) and compound (VIII) include sodium borohydride, lithium borohydride, cyanosodium borohydride and the like. These reducing agents are used in an amount of generally 1 to 10 equivalents, preferably 1 to 4 equivalents, relative to compound (VII). The reaction temperature is from -20°C to 50°C, preferably 0°C - room temperature and the reaction time is 0.5 - 24 h.

The catalytic reduction is conducted by a reaction with a catalytic amount of a metal catalyst, such as Raney Nickel, platinum oxide, metal palladium, palladium-carbon etc. in an inert solvent (e.g., alcohol solvent such as methanol, ethanol, isopropanol, t-butanol etc.) at room temperature to 100°C at a hydrogen pressure of 1 atm to 100 atm for 1 to 48 h.

The compound (II) used for this method can be produced by a method described in, for example, Chem. Pharm. Bull. 47(1) 28-36 (1999), JP-A-56-53654 and the like or a method analogous thereto.

The compound (III) to be used for this method can be produced by a method described in, for example, J. Am. Chem. Soc., 1950, 72, 1415., J. Am. Chem. Soc., 1952, 74, 4549, J. Org. Chem., 1956, 21, 1087 and the like or a method analogous thereto.

### Production Method 2

As shown in the following formulas, compound (IV) and compound (V) are reacted to produce compound (I). 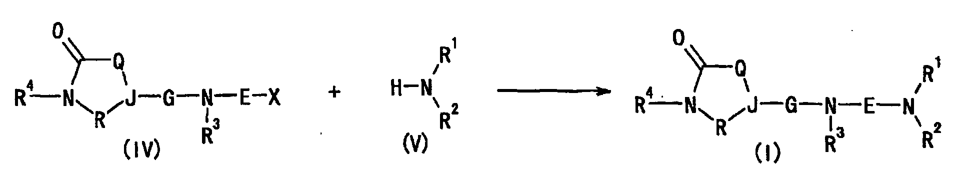 wherein each symbol is as defined above.

This reaction can be carried out according to the method described in, for example, ORGANIC FUNCTIONAL GROUP PREPARATIONS, 2nd printing, ACADEMIC PRESS, INC.

This reaction is generally carried out in a solvent inert to the reaction. Examples of the solvent include alcohol solvents, ether solvents, halogen solvents, aromatic solvents, acetonitrile, N,N-dimethylformamide (DMF), acetone, methyl ethyl ketone, dimethyl sulfoxide (DMSO) and the like, which may be used alone or in combination. Of these, acetonitrile, dimethylformamide, acetone, ethanol and the like are preferable. The reaction temperature is generally from room temperature to 100°C, preferably from room temperature to 50°C and the reaction time is generally from 0.5 to one day. For this reaction, 1 to 3 equivalents of a base is generally added relative to compound (IV), but it is not essential. Examples of the base include the base used for the reaction of the above-mentioned compound (II) and compound (III).

The compound (IV) used as a starting material for this reaction can be synthesized by a known method using compound (III) as a starting material.

### Production Method 3

Of the compound (I), a compound wherein E is represented by the formula: 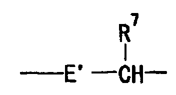 wherein E' is a group E having less one carbon atoms, R⁷ is a hydrogen atom or a hydrocarbon group, can be produced as shown in the following formulas, wherein compound of the formula (IX) and compound of the formula (V) are reacted under reducing conditions to give the compound. 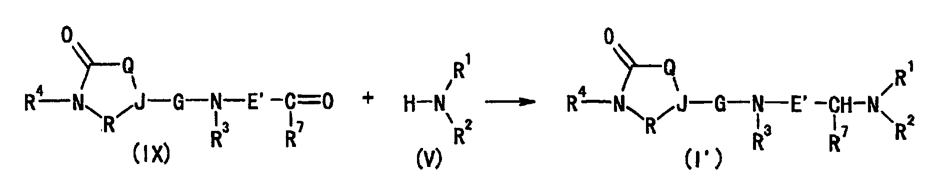 wherein each symbol is as defined above.

The group expressed by E' which has less one carbon atoms as compared to E is a divalent chain hydrocarbon group optionally having a substituent or substituents other than oxo group and has carbon atoms of E less one. Examples of the hydrocarbon group expressed by R⁷ include unsubstituted alkyl group, aryl group, cycloalkyl group and cycloalkenyl group from the alkyl group optionally having a substituent or substituents, aryl group optionally having a substituent or substituents, cycloalkyl group optionally having a substituent or substituents and cycloalkenyl group optionally having a substituent or substituents, which have been exemplified as the substituents other than oxo group of a divalent chain hydrocarbon group optionally having a substituent or substituents other than oxo group, as expressed by E.

This reaction is carried out generally by reacting compound (IX) and compound (V) in a suitable solvent (e.g., water, alcohol, ether, halogen, acetonitrile, mixed solvent of two or more kinds of these etc.), adding an acidic substance where necessary, such as acetic acid, trifluoroacetic acid and the like, in the presence of a compound (1 - 5 equivalents, preferably 1 - 1.5 equivalents), wherein carbonyl group is added to alkyl group, and a reducing agent. The reducing agent and other conditions are the same as those described for the method of Production Method 1.

The compound (IV) used as a starting material for this reaction can be produced by a known method using compound (III) as a starting material.

### Production Method 4

Of the compound (I), a compound wherein E is represented by the formula: 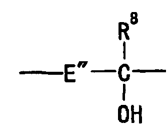 wherein E" is a group E having less two carbon atoms and R⁸ is a hydrocarbon group, can be produced as shown by reacting compound of the formula (X) and compound of the formula (V). 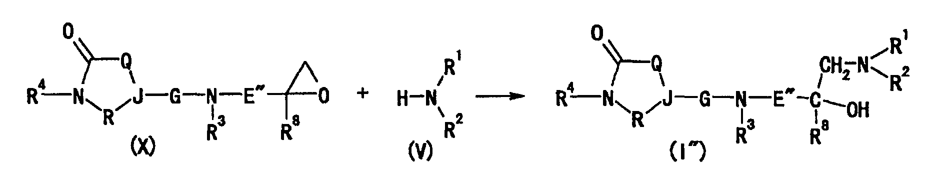 wherein each symbol is as defined above.

The group expressed by E" which has less two carbon atoms as compared to E is a divalent chain hydrocarbon group optionally having a substituent or substituents other than oxo group and has carbon atoms of E less two. Examples of the hydrocarbon group expressed by R⁸ include hydrocarbon groups exemplified for R⁷.

This reaction is carried out in the presence or absence of a solvent. Examples of the solvent include those recited for the reaction of the aforementioned compound (II) and compound (III). For this reaction, a Lewis acid such as anhydrous zinc chloride, anhydrous aluminum chloride, anhydrous iron(II) chloride, titanium tetrachloride, tin tetrachloride, cobalt chloride, copper(II) chloride, boron trifluoride etherate etc. or the aforementioned base can be used as a catalyst to accelerate the reaction. The reaction temperature is generally from -40°C to 180°C.

The compound (X) used as a starting material for this reaction can be synthesized by a known method using compound (III) as a starting material.

### Production Method 5

The compound (XI) and compound (XII) are reacted to produce compound (I). 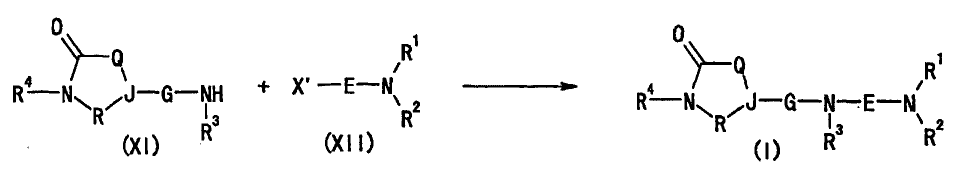 wherein X' is a leaving group and other symbols are as defined above.

Examples of the leaving group expressed by X' include those exemplified as the leaving group expressed by X.

This reaction can be carried out according to the method of Production Method 2.

The compound (XII) used as a starting material for this reaction can be produced from compound (V) by a known method.

The compound (XI) used as a starting material for this reaction can be synthesized by reacting compound (III) and compound (VIII) according to the method of Production Method 1.

### Production Method 6

As shown in the following formulas, the compound and compound (XIV) are reacted to produce compound (I). 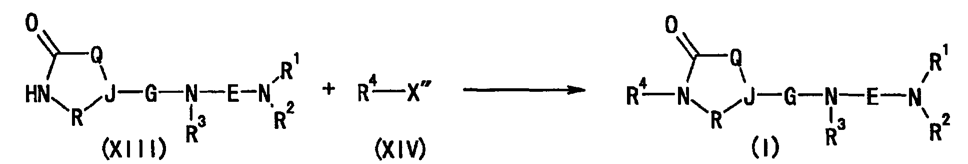 wherein X" is a leaving group and other symbols are as defined above.

This reaction can be carried out according to the method of Production Method 2. Examples of the leaving group expressed by X" include those exemplified as the leaving group expressed by X.

The compound (I) of the present invention can be combined with different agents for the prophylaxis or treatment of HIV infectious diseases (particularly, agent for the prophylaxis or treatment of AIDS). In this case, these drugs are separately or simultaneously mixed with pharmacologically acceptable carriers, excipients, binders, diluents and the like and formulated into preparations, which can be administered orally or parenterally as pharmaceutical compositions for the prophylaxis or treatment of HIV infectious diseases. When the drugs are separately formulated into preparations, respective preparations may be mixed when in use by the use of a diluent and the like before administration. It is also possible to administer respective preparations formulated separately at the same time or separately at certain time intervals to the same subject. A kit product to administer separately formulated preparations by mixing, when in use, by the use of a diluent and the like (e.g., injection kit including ampoules containing respective powder drugs, a diluent to mix and dissolve two or more kinds of drugs when in use, and the like), a kit product to administer separately formulated preparations at the same time or separately at certain time intervals to the same subject (e.g., tablet kit for administering two or more tablets at the same time or separately at certain time intervals, which includes tablets containing respective drugs placed in the same bag or different bags having, where necessary, a description column to note the time of administration of the drug etc.), and the like are also encompassed in the pharmaceutical composition of the present invention.

Specific examples of other agents for the prophylaxis or treatment of HIV infectious diseases, which are used in combination with the compound (I) of the present invention, include nucleoside reverse transcriptase inhibitors such as zidovudine, didanosine, zalcitabine, lamivudine, stavudine, abacavir, adefovir, adefovir dipivoxil, fozivudine tidoxil and the like; non-nucleoside reverse transcriptase inhibitors such as nevirapine, delavirdine, efavirenz, loviride, immunocal, oltipraz and the like, inclusive of pharmaceutical agents having antioxidant action such as immunocal, oltipraz and the like; protease inhibitors such as saquinavir, ritonavir, indinavir, nelfinavir, amprenavir, palinavir, lasinavir and the like; and the like.

As the nucleoside reverse transcriptase inhibitors, zidovudine, didanosine, zalcitabine, lamivudine, stavudine and the like are preferable, as the non-nucleoside reverse transcriptase inhibitors, nevirapine, delavirdine and the like are preferable, and as the protease inhibitor, saquinavir, ritonavir, indinavir, nelfinavir and the like are preferable.

The compound (I) of the present invention can be used in combination with the aforementioned protease inhibitors, nucleoside reverse transcriptase inhibitors and the like, as well as, for example, CXCR4 antagonists (e.g., AMD-3100 etc.), which are second receptors of T-cell tropic HIV-1, antibodies against HIV-1 surface antigens, and HIV-1 vaccines.

The compound (I) of the present invention has a CCR antagonistic action, particularly a potent CCR5 antagonistic action. Therefore, the compound is used for the prophylaxis or treatment of various HIV infectious diseases in human, such as AIDS. The compound (I) of the present invention is low toxic and can be used safely.

The compound (I) of the present invention can be used as a CCR5 antagonist for, for example, an agent for the prophylaxis or treatment of AIDS and an agent for suppressing the progress of the disease state of AIDS.

While the daily dose of the compound (I) varies depending on the condition and body weight of patients and administration route, it is about 5 to 1000 mg, preferably about 10 to 600 mg, more preferably about 10 to 300 mg, particularly preferably about 15 to 150 mg, in the amount of the active ingredient [compound (I)] in the case of oral administration to an adult (body weight 50 Kg), which is administered once or two to three times a day.

When the compound (I) and a reverse transcriptase inhibitor and/or a protease inhibitor are used in combination, the dose of the reverse transcriptase inhibitor or the protease inhibitor is appropriately determined within the range of not less than about 1/200 to 1/2 and not more than about 2 to 3 times the typical dose. Moreover, when two or more kinds of pharmaceutical agents are used in combination, and when one pharmaceutical agent affects metabolism of a different pharmaceutical agent, the dose of each pharmaceutical agent is adjusted as appropriate. In general, a dose for a single administration of each pharmaceutical agent is employed.

For example, the general doses of typical reverse transcriptase inhibitors and protease inhibitors are as follows.
zidovudine: 100 mg
didanosine: 125 - 200 mg
zalcitabine: 0.75 mg
lamivudine: 150 mg
stavudine: 30 - 40 mg
saquinavir: 600 mg
ritonavir: 600 mg
indinavir: 800 mg
nelfinavir: 750 mg

Specific embodiments, wherein the compound (I) and a reverse transcriptase inhibitor and/or a protease inhibitor are combined, are shown in the following.
(a) The compound (I) (about 10 - 300 mg) and zidovudine (about 50 - 200 mg) per an adult (body weight 50 Kg) are combined and administered to the same subject. The respective drugs may be administered simultaneously or at a time difference of within 12 hours.
(b) The compound (I) (about 10 - 300 mg) and saquinavir (about 300 - 1200 mg) per an adult (body weight 50 Kg) are combined and administered to the same subject per an adult (body weight 50 Kg). The respective drugs may be administered simultaneously or at a time difference of within 12 hours.

### Most Preferable Embodiment of The Invention

The present invention is explained in detail in the following by referring to Examples, Reference Examples, Experimental Examples and Formulation Examples. However, these are mere examples and do not limit the present invention in any way.

The gene manipulation methods described below followed the method described in a textbook (Maniatis et al, Molecular Cloning, Cold Spring Harbor Laboratory, 1989) or a method described in the attached protocol of reagent.

### Example 1

### N-[3-(4-benzyl-1-piperidinyl)propyl]-1-methyl-5-oxo-N-phenyl-3-pyrrolidinecarboxamide hydrochloride

A mixture of the compound (400 mg, purity 80% from ¹H NMR) obtained in Reference Example 3, 4-benzylpiperidine (0.239 ml, 1.4 mmol), potassium iodide (225 mg, 1.4 mmol), potassium carbonate (282 mg, 2.0 mmol), acetonitrile (20 ml) was stirred at 100°C for 24 h. The reaction mixture was concentrated under reduced pressure and water (15 ml) was added to the residue. The mixture was extracted with ethyl acetate (30 mlx3). The organic layer was dried over anhydrous magnesium sulfate and concentrated under reduced pressure. The residue was subjected to column chromatography (silica gel 10 g, ethyl acetate/methanol=1/0→9/1). The objective fraction was concentrated under reduced pressure and the residue was dissolved in diethyl ether. 1N Hydrogen chloride (diethyl ether solution, 2 ml) was added and the precipitate was filtrated. The precipitate was washed with diethyl ether and dried under reduced pressure to give the title compound (282 mg, 0.6 mmol, yield 44%) as a hygroscopic pale-yellow amorphous.
¹H NMR (D₂O) 8 1.35-1.65 (2H, m), 1.75-2.1 (5H, m), 2.45 (1H, dd, J=8.7, 17.7Hz), 2.55-2.75 (1H, m), 2.63 (2H, d, J=6.8Hz), 2.77 (3H, s), 2.8-3.0 (2H, m), 3.0-3.7 (7H, m), 3.75-3.9 (2H, m), 7.2-7.45 (7H, m), 7.45-7.65 (3H, m).
Anal. Calcd for C₂₇H₃₅N₃O₂·HCl·0.5H₂O: C, 67.69; H, 7.78; Cl, 7.40; N, 8.77. Found: C, 67.58; H, 7.75; Cl, 7.17; N, 8.59.

### Example 2

### 1-methyl-5-oxo-N-phenyl-N-[3-(1-piperidinyl)propyl]-3-pyrrolidinecarboxamide hydrochloride

By reactions and purification similar to those in Example 1 using piperidine, the title compound was obtained, yield 48%.
¹H NMR (D₂O) δ 1.3-2.1 (8H, m), 2.46 (1H, dd, J=9.0, 17.2Hz), 2.66 (1H, dd, J=6.0, 17.2Hz), 2.75-3.2 (4H, m), 2.78 (3H, s), 3.2-3.65 (3H, m), 3.42 (1H, t, J=10.0Hz), 3.57 (1H, dd, J=5.5, 10.0Hz), 3.75-3.95 (2H, m), 7.3-7.4 (2H, m), 7.5-7.7 (3H, m).
Anal. Calcd for C₂₀H₂₉N₃O₂·HCl·0.2H₂O: C, 62.63; H, 7.99; Cl, 9.24; N, 10.96. Found: C, 62.63; H, 7.80; Cl, 9.19; N, 10.99.

### Example 3

### N-{3-[cyclohexyl (methyl) amino]propyl}-1-methyl-5-oxo-N-phenyl-3-pyrrolidinecarboxamide hydrochloride

By reactions and purification similar to those in Example 1 using *N*-methylcyclohexylamine, the title compound was obtained, yield 12%.
¹H NMR (D₂O) δ 1.0-2.1 (12H, m), 2.47 (1H, dd, J=9.7, 17.1Hz), 2.65 (1H, dd, J=6.1, 17.1Hz), 2.78 (3H+3H, s), 3.0-3.5 (4H, m), 3.43 (1H, t, J=9.7Hz), 3.57 (1H, dd, J=5.4, 9.7Hz), 3.7-4.0 (2H, m), 7.3-7.45 (2H, m), 7.5-7.65 (3H, m).
Anal. Calcd for C₂₂H₃₃N₃O₂·HCl·0.8H₂O: C, 62.56; H, 8.50; Cl, 8.39; N, 9.95. Found: C, 62.46; H, 8.48; Cl, 8.34; N, 9.86.

### Example 4

### 1-methyl-5-oxo-N-phenyl-N-[3-(1,2,3,4-tetrahydro-2-isoquinolyl)propyl]-3-pyrrolidinecarboxamide hydrochloride

By reactions and purification similar to those in Example 1 using 1,2,3,4-tetrahydroisoquinoline, the title compound was obtained, yield 39%.
¹H NMR (D₂O) δ 2.0-2.2 (2H, m), 2.44 (1H, dd, J=9.8, 16.8Hz), 2.55-2.75 (1H, m), 2.77 (3H, s), 3.1-3.7 (9H, m), 3.75-4.0 (2H, m), 4.45 (2H, s), 7.15-7.45 (6H, m), 7.45-7.7 (3H, m).
Anal. Calcd for C₂₄H₂₉N₃O₂·HCl·1.1H₂O: C, 64.37; H, 7.25; Cl, 7.92; N, 9.38. Found: C, 64.35; H, 7.08; Cl, 7.49; N, 9.33.

### Example 5

### 1-methyl-5-oxo-N-phenyl-N-[3-(1,2,4,5-tetrahydro-3H-3-benzoazepin-3-yl)propyl]-3-pyrrolidinecarboxamide fumarate

By reactions and purification similar to those in Example 1 using 1,2,4,5-tetrahydro-3*H*-3-benzoazepine, the title compound was obtained, yield 33%.
¹H NMR (D₂O) δ 1.9-2.15 (2H, m) , 2.45 (1H, dd, J=9.5, 17.9Hz), 2.65 (1H, dd, J=5.7, 17.9Hz), 2.76 (3H, s), 2.95-3.4 (9H, m), 3.41 (1H, t, J=9.8Hz), 3.56 (1H, dd, J=5.3, 9.8Hz), 3.6-3.95 (4H, m), 6.62 (2H, s), 7.28 (4H, s), 7.3-7.4 (2H, m), 7.45-7.65 (3H, m).
Anal. Calcd for C₂₅H₃₁N₃O₂·C₄H₄O₄·0.2H₂O: C, 66.32; H, 6.79; N, 8.00. Found: C, 66.23; H, 6.71; N, 7.95.

### Example 6

### 1-methyl-5-oxo-N-phenyl-N-[3-(4-phenyl-1-piperidinyl)propyl]-3-pyrrolidinecarboxamide fumarate

By reactions and purification similar to those in Example 1 using 4-phenylpiperidine hydrochloride, the title compound was obtained, yield 42%.
¹H NMR (D₂O) δ 1.7-2.3 (6H, m), 2.45 (1H, dd, J=9.0, 17.3Hz), 2.65 (1H, dd, J=5.7, 17.3Hz), 2.77 (3H, s), 2.8-4.0 (12H, m), 6.67 (2H, s), 7.25-7.65 (10H, m).
Anal. Calcd for C₂₆H₃₃N₃O₂·C₄H₄O₄·0.8H₂O: C, 65.51; H, 7.07; N, 7.64. Found: C, 65.53; H, 6.97; N, 7.65.

### Example 7

### N-[3-(4-acetamide-4-phenyl-1-piperidinyl)propyl]-1-methyl-5-oxo-N-phenyl-3-pyrrolidinecarboxamide hydrochloride

By reactions and purification similar to those in Example 1 using 4-acetamide-4-phenylpiperidine hydrochloride, the title compound was obtained, yield 40%.
¹H NMR (D₂O) δ 1.85-2.8 (8H, m), 2.07 (3H, s), 2.77 (3H, s), 3.1-3.7 (9H, m), 3.7-4.0 (2H, m), 7.25-7.7 (10H, m).
Anal. Calcd for C₂₈H₃₆N₄O₃·HCl·1.4H₂O: C, 62.48; H, 7.45; Cl, 6.59; N, 10.41. Found: C, 62.56; H, 7.23; Cl, 7.02; N, 10.11.

### Example 8

### N-[3-(indene-1-spiro-4'-piperidin-1'-yl)propyl]-1-methyl-5-oxo-N-phenyl-3-pyrrolidinecarboxamide fumarate

By reactions and purification similar to those in Example 1 using indene-1-spiro-4'-piperidine, the title compound was obtained, yield 43%.
¹H NMR (D₂O) δ 1.45-1.65 (2H, m), 1.95-2.2 (2H, m), 2.3-2.55 (3H, m), 2.67 (1H, dd, J=6.2, 17.2Hz), 2.77 (3H, s), 3.2-3.45 (5H, m), 3.42 (1H, t, J=9.8Hz), 3.59 (1H, dd, J=5.4, 9.8Hz), 3.65-3.8 (2H, m), 3.8-3.95 (2H, m), 6.63 (2H, s), 6.97 (1H, d, J=5.8Hz), 7.02 (1H, d, J=5.8Hz), 7.25-7.7 (9H, m).
Anal. Calcd for C₂₈H₃₃N₃O₂·C₄H₄O₄·1.0H₂O: C, 66.53; H, 6.80; N, 7.27. Found: C, 66.60; H, 6.62; N, 7.30.

### Example 9

### N-(3-{4-[hydroxy(diphenyl)methyl]-1-piperidinyl}propyl)-1-methyl-5-oxo-N-phenyl-3-pyrrolidinecarboxamide

By reactions and purification similar to those in Example 1 using 4-[hydroxy(diphenyl)methyl]piperidine, the title compound was obtained, yield 51%.
¹H NMR (CDCl₃) δ 1.35-2.55 (12H, m), 2.6-2.8 (1H, m), 2.76 (3H, s), 2.8-3.15 (3H, m), 3.17 (1H, t, J=9.1Hz), 3.55-3.8 (3H, m), 7.05-7.55 (15H, m).
Anal. Calcd for C₃₃H₃₉N₃O₃·0.6H₂O: C, 73.88; H, 7.55; N, 7.83. Found: C, 73.81; H, 7.58; N, 7.83.

### Example 10

### N-[3-(4-benzyl-1-piperazinyl)propyl]-1-methyl-5-oxo-N-phenyl-3-pyrrolidinecarboxamide dihydrochloride

By reactions and purification similar to those in Example 1 using 1-benzylpiperazine, the title compound was obtained, yield 51%.
¹H NMR (D₂O) δ 1.9-2.1 (2H, m), 2.44 (1H, dd, J=9.2, 17.1Hz), 2.64 (1H, dd, J=6.5, 17.1Hz), 2.76 (3H, s) , 3.15-3.7 (13H, m), 3.7-4.0 (2H, m), 4.38 (2H, s), 7.3-7.4 (2H, m), 7.45-7.65 (8H, m).
Anal. Calcd for C₂₆H₃₄N₄O₂·2HCl·1.2H₂O: C, 59.02; H, 7.31; Cl, 13.40; N, 10.59. Found: C, 59.00; H, 7.34; Cl, 13.36; N, 10.49.

### Example 11

### 1-methyl-5-oxo-N-phenyl-N-[3-(1-piperazinyl)propyl] -3-pyrrolidinecarboxamide

*N*-[3-(4-Benzyl-1-piperazinyl)propyl]-1-methyl-5-oxo-*N*-phenyl-3-pyrrolidinecarboxamide (463 mg, 1.1 mmol) was dissolved in methanol (10 ml) and palladium hydroxide - carbon (20%, 93 mg) was added and the mixture was stirred at room temperature for 16 h under a hydrogen atmosphere. An insoluble material was filtrated and the insoluble material was washed with methanol. The filtrate was concentrated under reduced pressure to give the title compound (364 mg, 1.1 mmol, yield 99%) as a colorless oil.
¹H NMR (CDCl₃) δ 1.6-1.85 (2H, m), 2.15-2.6 (9H, m), 2.6-2.9 (3H, m), 2.77 (3H, s) , 2.95-3.2 (1H, m), 3.19 (1H, t, J=8.9Hz), 3.64 (1H, dd, J=6.8, 8.9Hz), 3.65-3.8 (2H, m), 7.1-7.2 (2H, m), 7.3-7.55 (3H, m).

### Example 12

### N- [3-(4-benzoyl-1-piperazinyl)propyl]-1-methyl-5-oxo-N-phenyl-3-pyrrolidinecarboxamide fumarate

The compound (192 mg, 0.56 mmol) obtained in Example 11 and triethylamine (0.101 ml, 0.72 mmol) were dissolved in THF (5 ml) and benzoyl chloride (0.078 ml, 0.67 mmol) was added under ice-cooling and the mixture was stirred at the same temperature for 1 h. The reaction mixture was concentrated under reduced pressure and a saturated aqueous sodium hydrogencarbonate solution (15 ml) was added. The mixture was extracted with ethyl acetate (30 ml×3). The organic layer was dried over anhydrous magnesium sulfate and concentrated under reduced pressure. The residue was subjected to column chromatography (silica gel 10 g, ethyl acetate/methanol = 1/0→9/1→4/1). The objective fraction was concentrated under reduced pressure to give *N*-[3-(4-benzoyl-1-piperazinyl)propyl]-1-methyl-5-oxo-*N*-phenyl-3-pyrrolidinecarboxamide (221 mg, 0.49 mmol). The obtained compound was dissolved in methanol and fumaric acid (57 mg, 0.49 mmol) was added. The reaction mixture was concentrated under reduced pressure and diethyl ether was added. The precipitate was collected by filtration. The precipitate was washed with diethyl ether and dried under reduced pressure to give the title compound (228 mg, 0.40 mmol, yield 72%) as a hygroscopic pale-yellow amorphous.
¹H NMR (D₂O) δ 1.9-2.15 (2H, m), 2.44 (1H, dd, J=9.0, 17.6Hz), 2.65 (1H, dd, J=6.0, 17.6Hz), 2.76 (3H, s), 3.1-4.0 (15H, m), 6.63 (2H, s), 7.3-7.4 (2H, m), 7.4-7.65 (8H, m).
Anal. Calcd for C₂₆H₃₂N₄O₃·C₄H₄O₄·0.9H₂O: C, 62.03; H, 6.56; N, 9.65. Found: C, 61.97; H, 6.36; N, 9.35.

### Example 13

### N-{3-[4-(4-fluorobenzoyl) -1-piperidinyl]propyl}-1-methyl-5-oxo-N-phenyl-3-pyrrolidinecarboxamide

By reactions and purification similar to those in Example 1 using 4-(4-fluorobenzoyl)piperidine hydrochloride, the title compound was obtained.
¹H NMR (CDCl₃) δ 1.56-1.90 (6H, m), 1.97-2.44 (5H, m), 2.60-2.80 (4H, m), 2.85-3.26 (5H, m), 3.58-3.80 (3H, m), 7.06-7.20 (4H, m), 7.34-7.53 (3H, m), 7.95 (2H, dd, J=5.1, 8.8Hz).

### Example 14

### N-{3-[4-(4-chlorophenyl)-4-hydroxy-1-piperidinyl]propyl}-1-methyl-5-oxo-N-phenyl-3-pyrrolidinecarboxamide

By reactions and purification similar to those in Example 1 using 4-(4-chlorophenyl)-4-hydroxypiperidine, the title compound was obtained.
¹H NMR (CDCl₃) δ 1.44-1.95 (7H, m), 2.03-2.91 (10H, m), 2.97-3.25 (3H, m), 3.60-3.84 (3H, m), 7.13-7.54 (9H, m).

### Example 15

### N-{3-[4-(4-fluorophenyl)-1-piperazinyl]propyl}-1-methyl-5-oxo-N-phenyl-3-pyrrolidinecarboxamide

By reactions and purification similar to those in Example 1 using 1-(4-fluorophenyl)piperazine, the title compound was obtained.
¹H NMR (CDCl₃) δ 1.56-1.87 (2H, m), 2.16-2.84 (11H, m), 2.93-3.26 (6H, m), 3.56-3.84 (3H, m), 6.69-7.21 (6H, m), 7.29-7.52 (3H, m) .

### Example 16

### N-{3-[4-(diphenylmethyl)-1-piperazinyl]propyl}-1-methyl-5-oxo-N-phenyl-3-pyrrolidinecarboxamide

By reactions and purification similar to those in Example 1 using 1-(diphenylmethyl)piperazine, the title compound was obtained.
¹H NMR (CDCl₃) δ 1.60-1.86 (2H, m), 2.12-2.50 (11H, m) , 2.58-2.80 (4H, m), 2.94-3.21 (2H, m), 3.55-3.77 (3H, m), 4.19 (1H, s), 7.07-7.30 (8H, m), 7.33-7.50 (7H, m).

### Example 17

### N-{4-[4-(4-fluorobenzoyl)-1-piperidinyl]butyl}-1-methyl-5-oxo-N-phenyl-3-pyrrolidinecarboxamide

By reactions and purification similar to those in Example 1 using the compound obtained in Reference Example 4 and 4-(4-fluorobenzoyl)piperidine hydrochloride, the title compound was obtained.
¹H NMR (CDCl₃) δ 1.39-1.64 (4H, m), 1.71-2.43 (9H, m), 2.60-2.80 (4H, m), 2.86-3.27 (5H, m), 3.59-3.68 (3H, m), 7.06-7.20 (4H, m), 7.35-7.53 (3H, m), 7.97 (2H, dd, J=5.5, 8.9Hz).

### Example 18

### N-{4-[4-(4-chlorophenyl)-4-hydroxy-1-piperidinyl]butyl}-1-methyl-5-oxo-N-phenyl-3-pyrrolidinecarboxamide

By reactions and purification similar to those in Example 1 using the compound obtained in Reference Example 4 and 4-(4-chlorophenyl)-4-hydroxypiperidine, the title compound was obtained.
¹H NMR (CDCl₃) δ 1.42-1.93 (7H, m), 1.97-2.52 (7H, m), 2.56-2.89 (6H, m), 2.95-3.25 (2H, m) , 3.55-3.81 (3H, m) , 7.07-7.20 (2H, m), 7.23-7.56 (7H, m).

### Example 19

### N-{4-[4- (4-fluorophenyl)-1-piperazinyl]butyl}-1-methyl-5-oxo-N-phenyl-3-pyrrolidinecarboxamide

By reactions and purification similar to those in Example 1 using the compound obtained in Reference Example 4 and 1-(4-fluorophenyl)piperazine, the title compound was obtained.
¹H NMR (CDCl₃) δ 1.46-1.64 (4H, m) , 2.23 (1H, dd, J=9.2, 16.9Hz), 2.33-2.46 (2H, m), 2.53-2.80 (8H, m), 3.00-3.24 (6H, m), 3.60-3.80 (3H, m), 6.81-7.02 (4H, m), 7.11-7.20 (2H, m), 7.35-7.53 (3H, m).

### Example 20

### N-{4-[4-(diphenylmethyl)-1-piperazinyl]butyl}-1-methyl-5-oxo-N-phenyl-3-pyrrolidinecarboxamide

By reactions and purification similar to those in Example 1 using the compound obtained in Reference Example 4 and 1-(diphenylmethyl)piperazine, the title compound was obtained.
¹H NMR (CDCl₃) δ 1.35-1.62 (4H, m), 2.08-2.53 (11H, m), 2.58-2.80 (4H, m), 2.93-3.22 (2H, m), 3.54-3.77 (3H, m), 4.20 (1H, s), 7.06-7.51 (15H, m).

### Example 21

### N-{5-[4-(4-fluorobenzoyl)-1-piperidinyl]pentyl}-1-methyl-5-oxo-N-phenyl-3-pyrrolidinecarboxamide

By reactions and purification similar to those in Example 1 using the compound obtained in Reference Example 5 and 4-(4-fluorobenzoyl)piperidine hydrochloride, the title compound was obtained.
¹H NMR (CDCl₃) δ 1.22-1.63 (6H, m) , 1.68-1.92 (4H, m), 1.97-2.40 (5H, m), 2.60-2.80 (4H, m), 2.91-3.28 (5H, m), 3.58-3.76 (3H, m), 7.06-7.21 (4H, m), 7.35-7.53 (3H, m), 7.96 (2H, dd, J=5.5, 8.8Hz).

### Example 22

### N-{2-[4-(4-fluorobenzoyl)-1-piperidinyl]ethyl}-1-methyl-5-oxo-N-phenyl-3-pyrrolidinecarboxamide fumarate

By reactions and purification similar to those in Example 1 using the compound obtained in Reference Example 6-4 and 4-(4-fluorobenzoyl)piperidine hydrochloride, the title compound was obtained, yield 20%.
¹H NMR (D₂O) δ 1.75-2.3 (4H, m), 2.43 (1H, dd, J=9.4, 17.6Hz), 2.55-2.75 (1H, m), 2.76 (3H, s), 3.05-4.0 (10H, m), 4.05-4.3 (2H, m) , 6.66 (2H, s), 7.29 (2H, t, J=8.8Hz), 7.3-7.45 (2H, m), 7.45-7.65 (3H, m), 8.06 (2H, dd, J=5.5, 8.7Hz).
Anal. Calcd for C₂₆H₃₀FN₃O₃·C₄H₄O₄·1.5H₂O: C, 60.60; H, 6.27; N, 7.07. Found: C, 60.68; H, 6.13; N, 7.15.

### Example 23

### N-[3-(4-benzyl-1-piperidinyl)propyl]-N-(3,4-dichlorophenyl)-1-methyl-5-oxo-3-pyrrolidinecarboxamide hydrochloride

By reactions and purification similar to those in Example 1 using the compound obtained in Reference Example 7, the title compound was obtained, yield 69%.
¹H NMR (D₂O) 8 1.35-1.65 (2H, m), 1.75-2.1 (5H, m), 2.47 (1H, dd, J=9.4, 18.0Hz), 2.55-2.75 (1H, m), 2.65 (2H, d, J=7.2Hz), 2.75-3.2 (4H, m), 2.79 (3H, s), 3.2-3.7 (5H, m), 3.7-3.9 (2H, m), 7.25-7.45 (6H, m), 7.63 (1H, d, J=2.2Hz), 7.72 (1H, d, J=8.4Hz).
Anal. Calcd for C₂₇H₃₃Cl₂N₃O₂·HCl·0.7H₂O: C, 58.80; H, 6.47; Cl, 19.28; N, 7.62. Found: C, 58.77; H, 6.41; Cl, 18.91; N, 7.56.

### Example 24

### N-(3,4-dichlorophenyl)-N-{3-[4-(4-fluorobenzoyl)-1-piperidinyl]propyl}-1-methyl-5-oxo-3-pyrrolidinecarboxamide hydrochloride

By reactions and purification similar to those in Example 1 using the compound obtained in Reference Example 7 and 4-(4-fluorobenzoyl)piperidine hydrochloride, the title compound was obtained, yield 68%.
¹H NMR (D₂O) δ 1.7-2.3 (6H, m), 2.4-2.75 (2H, m), 2.79 (3H, s), 3.0-4.0 (12H, m), 7.2-7.4 (3H, m), 7.6-7.8 (2H, m), 8.0-8.15 (2H, m).
Anal. Calcd for C₂₇H₃₀Cl₂FN₃O₃·HCl·0.4H₂O: C, 56.09; H, 5.54; Cl, 18.40; N, 7.27. Found: C, 56.14; H, 5.66; Cl, 17.80; N, 7.22.

### Example 25

### N-[3-(4-benzylidene-1-piperidinyl)propyl]-1-methyl-5-oxo-N-phenyl-3-pyrrolidinecarboxamide hydrochloride

To a mixture of the compound (274 mg, 1.0 mmol) obtained in Reference Example 8-2, 4-benzylidenepiperidine hydrochloride (231 mg, 1.10 mmol) and THF (10 ml) were successively added triethylamine (0.209 ml, 1.5 mmol) and sodium triacetoxy boro hydride (318 mg, 1.5 mmol), and the mixture was stirred at room temperature for 6 h. A saturated aqueous sodium hydrogencarbonate solution (15 ml) and water (10 ml) were added and the mixture was extracted with ethyl acetate (20 ml×3). The organic layer was dried over anhydrous magnesium sulfate and concentrated under reduced pressure. The residue was subjected to column chromatography (silica gel 10 g, ethyl acetate/methanol = 1/0→9/1→6/1). The objective fraction was concentrated under reduced pressure and the residue was dissolved in methanol, and 1N hydrogen chloride (diethyl ether solution, 2 ml) was added. The mixture was concentrated under reduced pressure and diethyl ether was added to the residue and the precipitate was collected by filtration. The precipitate was washed with diethyl ether and dried under reduced pressure to give the title compound (380 mg, 0.81 mmol, yield 81%) as a hygroscopic pale-yellow amorphous.
¹H NMR (D₂O) δ 1.9-2.15 (2H, m), 2.3-4.0 (17H, m), 2.78 (3H, s), 6.61 (1H, s), 7.25-7.65 (10H, m).
Anal. Calcd for C₂₇H₃₃N₃O₂·HCl·0.7H₂O: C, 67.47; H, 7.42; Cl, 7.38; N, 8.74. Found: C, 67.48; H, 7.44; Cl, 7.40; N, 8.70.

### Example 26

### 1-methyl-5-oxo-N-[3-(4-phenoxy-1-piperidinyl)propyl]-N-phenyl-3-pyrrolidinecarboxamide hydrochloride

By reactions and purification similar to those in Example 25 using 4-phenoxypiperidine hydrochloride, the title compound was obtained, yield 78%.
¹H NMR (DMSO-*d*₆) δ 1.7-2.35 (7H, m), 2.35-2.55 (1H, m), 2.63 (3H, s), 2.85-3.85 (11H, m), 4.4-4.8 (1H, m) , 6.9-7.1 (3H, m), 7.2-7.6 (7H, m).
Anal. Calcd for C₂₆H₃₃N₃O₃·HCl·0.8H₂O: C, 64.20; H, 7.38; Cl, 7.29; N, 8.64. Found: C, 64.17; H, 7.50; Cl, 7.99; N, 8.66.

### Example 27

### 1-methyl-5-oxo-N-phenyl-N-(3-{4-[(E)-2-phenylethenyl]-1-piperidinyl}propyl)-3-pyrrolidinecarboxamide hydrochloride

By reactions and purification similar to those in Example 25 using 4-[(*E*)-2-phenylethenyl]piperidine hydrochloride, the title compound was obtained, yield 89%.
¹H NMR (D₂O) δ 1.55-1.9 (2H, m), 1.9-2.2 (5H, m), 2.46 (1H, dd, J=9.3, 17.2Hz), 2.66 (1H, dd, J=6.3, 17.2Hz), 2.78 (3H, s), 2.85-3.75 (9H, m), 3.75-3.95 (2H, m), 6.30 (1H, dd, J=6.5, 16.0Hz), 6.56 (1H, d, J=16.0Hz), 7.25-7.65 (10H, m).
Anal. Calcd for C₂₈H₃₅N₃O₂·HCl·0.6H₂O: C, 68.23; H, 7.61; Cl, 7.19; N, 8.53. Found: C, 68.18; H, 7.44; Cl, 7.20; N, 8.52.

### Example 28

### 1-methyl-5-oxo-N-[3-(4-phenethyl-1-piperidinyl)propyl]-N-phenyl-3-pyrrolidinecarboxamide hydrochloride

By reactions and purification similar to those in Example 25 using 4-phenethylpiperidine hydrochloride, the title compound was obtained, yield 62%.
¹H NMR (D₂O) δ 1.3-1.85 (5H, m), 1.85-2.15 (4H, m), 2.45 (1H, dd, J=8.7, 17.7Hz), 2.55-3.65 (12H, m), 2.77 (3H, s), 3.75-3.95 (2H, m), 7.2-7.45 (7H, m), 7.5-7.65 (3H, m).
Anal. Calcd for C₂₈H₃₇N₃O₂·HCl·1.0H₂O: C, 66.98; H, 8.03; Cl, 7.06; N, 8.37. Found: C, 66.99; H, 8.10; Cl, 7.52; N, 8.31.

### Example 29

### N-{3-[4-(benzyloxy)-1-piperidinyl]propyl}-1-methyl-5-oxo-N-phenyl-3-pyrrolidinecarboxamide hydrochloride

By reactions and purification similar to those in Example 25 using 4-(benzyloxy)piperidine hydrochloride, the title compound was obtained, yield 75%.
¹H NMR (D₂O) δ 1.7-2.4 (6H, m), 2.46 (1H, dd, J=8,8, 17.4Hz), 2.66 (1H, dd, J=6.1, 17.4Hz), 2.78 (3H, s), 3.0-3.65 (9H, m), 3.75-4.0 (3H, m), 4.64 (2H, s), 7.3-7.45 (2H, m), 7.45 (5H, s), 7.5-7.65 (3H, m).
Anal. Calcd for C₂₇H₃₅N₃O₃·HCl·0.6H₂O: C, 65.27; H, 7.55; Cl, 7.14; N, 8.46. Found: C, 65.27; H, 7.63; Cl, 7.14; N, 8.51.

### Example 30

### N-{3-[4-(diphenylmethyl)-1-piperidinyl]propyl}-1-methyl-5-oxo-N-phenyl-3-pyrrolidinecarboxamide fumarate

By reactions and purification similar to those in Example 25 using 4-(diphenylmethyl)piperidine hydrochloride, the title compound was obtained, yield 70%.
¹H NMR (DMSO-*d*₆) δ 1.0-1.3 (2H, m), 1.3-1.75 (4H, m), 1.95-2.55 (5H, m), 2.62 (3H, s), 2.8-3.1 (3H, m), 3.13 (1H, t, J=9.2Hz), 3.37 (1H, dd, J=6.1, 9.2Hz), 3.5-3.7 (4H, m), 3.54 (1H, d, J=11.0Hz), 6.57 (2H, s), 7.05-7.55 (15H, m).
Anal. Calcd for C₃₃H₃₉N₃O₂·C₄H₄O₄·0.3H₂O: C, 70.41; H, 6.96; N, 6.66. Found: C, 70.48; H, 7.06; N, 6.67.

### Example 31

### N-[3-(4-benzyl-1-piperidinyl)propyl]-1-methyl-N-(4-methylphenyl)-5-oxo-3-pyrrolidinecarboxamide hydrochloride

To a mixture of 1-methyl-5-oxo-3-pyrrolidinecarboxylic acid (358 mg, 2.5 mmol), DMF (0.023 ml) and dichloromethane (10 ml) was added oxalyl chloride (0.256 ml, 3.0 mmol) under ice-cooling and the mixture was stirred at the same temperature for 15 min and 1 h until it reached room temperature. The obtained solution was added to a mixture of the compound (395 mg, 1.0 mmol) obtained in Reference Example 9, triethylamine (1.39 ml, 10 mmol) and dichloromethane (15 ml) at -20°C with stirring and 1 h until it reached 0°C. A saturated aqueous sodium hydrogencarbonate solution (15 ml) was added. The organic solvent was evaporated under reduced pressure and the residue was extracted with ethyl acetate (15 ml×3). The organic layer was washed successively with saturated aqueous sodium hydrogencarbonate solution (5 ml×3) and saturated brine (5 ml), dried over anhydrous magnesium sulfate and concentrated under reduced pressure. The residue was subjected to column chromatography (silica gel 10 g, ethyl acetate/methanol=1/0→9/1). The objective fraction was concentrated under reduced pressure and the residue was dissolved in methanol. 1N Hydrogen chloride (diethyl ether solution, 2 ml) was added and the mixture was concentrated under reduced pressure. Diethyl ether was added to the residue and the precipitate was collected by filtration. The precipitate was washed with diethyl ether and dried under reduced pressure to give the title compound (409 mg, 0.84 mmol, yield 85%) as a hygroscopic pale-yellow amorphous.
¹H NMR (DMSO-*d*₆) δ 1.3-1.95 (7H, m) , 2.11 (1H, dd, J=9.9, 16.5Hz), 2.3-2.6 (3H, m) , 2.35 (3H, s) , 2.6-3.5 (9H, m), 2.63 (3H, s), 3.5-3.75 (2H, m) , 7.1-7.4 (9H, m).
Anal. Calcd for C₂₈H₃₇N₃O₂·HCl·0.6H₂O: C, 67.96; H, 7.98; Cl, 7.16; N, 8.49. Found: C, 67.99; H, 7.94; Cl, 7.45; N, 8.28.

### Example 32

### N-[3-(4-benzyl-1-piperidinyl)propyl]-N-(4-tert-butylphenyl)-1-methyl-5-oxo-3-pyrrolidinecarboxamide hydrochloride

By reactions and purification similar to those in Example 31 using the compound obtained in Reference Example 11, the title compound was obtained, yield 75%.
¹H NMR (DMSO-*d*₆) δ 1.31 (9H, s), 1.35-1.95 (7H, m), 2.11 (1H, dd, J=9.6, 16.4Hz), 2.35-2.6 (3H, m), 2.6-3.5 (9H, m), 2.63 (3H, s), 3.55-3.75 (2H, m) , 7.1-7.4 (7H, m), 7.51 (2H, d, J=8.4Hz).
Anal. Calcd for C₃₁H₄₃N₃O₂·HCl·0.6H₂O: C, 69.34; H, 8.48; Cl, 6.60; N, 7.83. Found: C, 69.27; H, 8.52; Cl, 6.40; N, 7.82.

### Example 33

### N-[3-(4-benzyl-1-piperidinyl)propyl]-N-(5-indanyl)-1-methyl-5-oxo-3-pyrrolidinecarboxamide hydrochloride

By reactions and purification similar to those in Example 31 using the compound obtained in Reference Example 12, the title compound was obtained, yield 69%.
¹H NMR (D₂O) δ 1.44-1.58 (2H, m), 1.88-2.14 (7H, m), 2.44-2.49 (1H, m), 2.60-2.69 (3H, m), 2.77 (3H, s), 2.81-2.98 (6H, m), 3.06-3.14 (2H, m), 3.28-3.53 (5H, m), 3.76-3.82 (2H, m), 7.08 (1H, d, J=8.2Hz), 7.22-7.43 (7H, m).
Anal. Calcd for C₃₀H₃₉N₃O₂·HCl·1.5H₂O: C, 67.08; H, 8.07; N, 7.82. Found: C, 67.19; H, 7.97; N, 8.01.

### Example 34

### N-[3-(4-benzyl-1-piperidinyl)propyl]-N-(4-methoxyphenyl)-1-methyl-5-oxo-3-pyrrolidinecarboxamide hydrochloride

By reactions and purification similar to those in Example 31 using the compound obtained in Reference Example 13, the title compound was obtained, yield 88%.
¹H NMR (D₂O) δ 1.35-1.65 (2H, m), 1.75-2.1 (5H, m), 2.45 (1H, dd, J=9.7, 17.7Hz), 2.55-2.75 (1H, m), 2.63 (2H, d, J=7.0Hz), 2.75-3.0 (2H, m), 2.78 (3H, s), 3.0-3.2 (2H, m), 3.2-3.65 (5H, m), 3.7-3.9 (2H, m), 3.89 (3H, s), 7.13 (2H, d, J=8.8Hz), 7.2-7.45 (7H, m).
Anal. Calcd for C₂₈H₃₇N₃O₃·HCl·0.6H₂O: C, 65.83; H, 7.73; Cl, 6.94; N, 8.22. Found: C, 65.79; H, 7.70; Cl, 6.98; N, 8.06.

### Example 35

### N-[3-(4-benzyl-1-piperidinyl)propyl]-N-(3,4-dimethoxyphenyl)-1-methyl-5-oxo-3-pyrrolidinecarboxamide hydrochloride

By reactions and purification similar to those in Example 31 using the compound obtained in Reference Example 14, the title compound was obtained, yield 78%.
¹H NMR (D₂O) δ 1.35-1.7 (2H, m), 1.7-2.1 (5H, m), 2.46 (1H, dd, J=8.6, 17.4Hz), 2.55-2.75 (1H, m), 2.63 (2H, d, J=6.0Hz), 2.75-4.1 (11H, m), 2.79 (3H, s), 3.89 (3H, s), 3.92 (3H, s), 6.9-7.1 (2H, m), 7.15 (1H, d, J=8.2Hz), 7.2-7.5 (5H, m).
Anal. Calcd for C₂₉H₃₉N₃O₄·HCl·0.7H₂O: C, 64.18; H, 7.69; Cl, 6.53; N, 7.74. Found: C, 64.21; H, 7.69; Cl, 6.65; N, 7.77.

### Example 36

### N-[3-(4-benzyl-1-piperidinyl)propyl]-N-(3,4-diethoxyphenyl)-1-methyl-5-oxo-3-pyrrolidinecarboxamide hydrochloride

By reactions and purification similar to those in Example 31 using the compound obtained in Reference Example 15, the title compound was obtained, yield 78%.
¹H NMR (D₂O) δ 1.40-1.52 (8H, m), 1.82-2.00 (5H, m), 2.46-2.64 (5H, m), 2.70-2.95 (5H, m), 3.07-3.14 (2H, m), 3.30-3.56 (6H, m), 4.10-4.22 (4H, m), 6.91-7.02 (2H, m), 7.13-7.17 (1H, m), 7.25-7.38 (5H, m).
Anal. Calcd for C₃₁H₄₃N₃O₄·HCl·1.0H₂O: C, 64.62; H, 8.05; N, 7.29. Found: C, 64.39; H, 8.11; N, 7.42.

### Example 37

### N-[3-(4-benzyl-1-piperidinyl)propyl]-N-(4-chlorophenyl)-1-methyl-5-oxo-3-pyrrolidinecarboxamide hydrochloride

By reactions and purification similar to those in Example 31 using the compound obtained in Reference Example 16, the title compound was obtained, yield 86%.
¹H NMR (D₂O) δ 1.35-1.65 (2H, m), 1.8-2.1 (5H, m), 2.45 (1H, dd, J=9.6, 17.6Hz), 2.55-2.75 (1H, m), 2.64 (2H, d, J=7.2Hz), 2.75-3.65 (9H, m), 2.78 (3H, s), 3.65-3.95 (2H, m), 7.2-7.45 (7H, m), 7.59 (2H, d, J=8.6Hz).
Anal. Calcd for C₂₇H₃₄ClN₃O₂·HCl·0.6H₂O: C, 62.93; H, 7.08; Cl, 13.76; N, 8.15. Found: C, 63.04; H, 7.14; Cl, 13.60; N, 8.16.

### Example 38

### N-[3-(4-benzyl-1-piperidinyl)propyl]-N-(3-chlorophenyl)-1-methyl-5-oxo-3-pyrrolidinecarboxamide hydrochloride

By reactions and purification similar to those in Example 31 using the compound obtained in Reference Example 17, the title compound was obtained, yield 79%.
¹H NMR (D₂O) δ 1.40-1.55 (2H, m), 1.85-2.03 (5H, m), 2.47-2.95 (9H, m), 3.06-3.59 (7H, m), 3.71-3.85 (2H, m), 7.25-7.55 (9H, m).
Anal. Calcd for C₂₇H₃₄ClN₃O₂·HCl·0.7H₂O: C, 62.71; H, 7.10; N, 8.13. Found: C, 62.77; H, 7.05; N, 8.24.

### Example 39

### N-[3-(4-benzyl-1-piperidinyl)propyl]-N-(3,4-difluorophenyl)-1-methyl-5-oxo-3-pyrrolidinecarboxamide hydrochloride

By reactions and purification similar to those in Example 31 using the compound obtained in Reference Example 19, the title compound was obtained, yield 80%.
¹H NMR (D₂O) δ 1.40-1.55 (2H, m), 1.89-2.00 (5H, m), 2.48-2.64 (4H, m), 2.77-2.94 (5H, m), 3.06-3.14 (2H, m), 3.30-3.55 (5H, m), 3.73-3.79 (2H, m), 7.20-7.46 (8H, m).
Anal. Calcd for C₂₇H₃₃F₂N₃O₂·HCl·0.6H₂O: C, 62.74; H, 6.86; N, 8.13. Found: C, 62.44; H, 6.88; N, 8.27.

### Example 40

### N-[3-(4-benzyl-1-piperidinyl)propyl]-N-(2,4-difluorophenyl)-1-methyl-5-oxo-3-pyrrolidinecarboxamide hydrochloride

By reactions and purification similar to those in Example 31 using the compound obtained in Reference Example 20, the title compound was obtained, yield 63%.
¹H NMR (D₂O) δ 1.43-1.58 (2H, m), 1.88-1.95 (5H, m), 2.47-2.65 (4H, m), 2.77-2.91 (5H, m), 3.07-3.11 (2H, m), 3.26 (1H, m), 3.36-3.55 (4H, m), 3.66-3.82 (2H, m), 7.10-7.49 (8H, m).
Anal. Calcd for C₂₇H₃₃F₂N₃O₂·HCl·1.0H₂O: C, 61.88; H, 6.92; N, 8.02. Found: C, 62.14; H, 6.95; N, 8.26.

### Example 41

### N-[3-(4-benzyl-1-piperidinyl)propyl]-N-(2,6-difluorophenyl)-1-methyl-5-oxo-3-pyrrolidinecarboxamide hydrochloride

By reactions and purification similar to those in Example 31 using the compound obtained in Reference Example 21, the title compound was obtained, yield 88%.
¹H NMR (D₂O) δ 1.40-1.58 (2H, m), 1.76-2.07 (5H, m), 2.50-2.64 (4H, m), 2.71-2.94 (5H, m), 3.08-3.29 (3H, m), 3.42-3.56 (4H, m), 3.76-3.81 (2H, m), 7.19-7.38 (7H, m), 7.53-7.58 (1H, m) .
Anal. Calcd for C₂₇H₃₃F₂N₃O₂·HCl·1.1H₂O: C, 61.67; H, 6.94; N, 7.99. Found: C, 61.52; H, 6.92; N, 8.29.

### Example 42

### N-[3-(4-benzyl-1-piperidinyl)propyl]-N-(3-chloro-4-fluorophenyl)-1-methyl-5-oxo-3-pyrrolidinecarboxamide hydrochloride

By reactions and purification similar to those in Example 31 using the compound obtained in Reference Example 22, the title compound was obtained, yield 68%.
¹H NMR (D₂O) δ 1.40-1.58 (2H, m), 1.89-1.96 (5H, m), 2.47-2.64 (4H, m), 2.77-2.95 (5H, m), 3.01-3.13 (2H, m), 3.32-3.56 (5H, m), 3.73-3.79 (2H, m), 7.25-7.40 (6H, m), 7.55-7.60 (2H, m).
Anal. Calcd for C₂₇H₃₃ClFN₃O₂·HCl·0.75H₂O: C, 60.50; H, 6.39; N, 7.84. Found: C, 60.70; H, 6.71; N, 8.16.

### Example 43

### N-[3-(4-benzyl-1-piperidinyl)propyl]-1-methyl-5-oxo-N- (4-trifluoromethylphenyl)-3-pyrrolidinecarboxamide hydrochloride

By reactions and purification similar to those in Example 31 using the compound obtained in Reference Example 23, the title compound was obtained, yield 70%.
¹H NMR (DMSO-*d*₆) δ 1.44-1.57 (2H, m), 1.70-1.85 (5H, m), 2.10-2.21 (2H, m), 2.39-2.54 (3H, m), 2.64 (3H, s), 2.70-3.05 (4H, m), 3.13-3.45 (4H, m), 3.65-3.75 (2H, m), 7.16-7.34 (5H, m), 7.65-7.69 (2H, m) , 7.85-7.90 (2H, m).
Anal. Calcd for C₂₈H₃₄F₃N₃O₂·HCl·0.5H₂O: C, 61.47; H, 6.63; N, 7.68. Found: C, 61.43; H, 6.73; N, 7.97.

### Example 44

### N-[3-(4-benzyl-1-piperidinyl)propyl]-N-[3,5-bis(trifluoromethyl)phenyl]-1-methyl-5-oxo-3-pyrrolidinecarboxamide hydrochloride

By reactions and purification similar to those in Example 31 using the compound obtained in Reference Example 24, the title compound was obtained, yield 50%.
¹H NMR (D₂O) δ 1.44-1.51 (2H, m), 1.89-2.01 (5H, m), 2.45-2.63 (4H, m), 2.69-2.96 (5H, m), 3.08-3.85 (9H, m), 7.25-7.38 (5H, m), 8.06 (2H, s), 8.26 (1H, s).

### Example 45

### N-[3-(4-benzyl-1-piperidinyl)propyl]-1-methyl-5-oxo-N-(4-trifluoromethoxyphenyl)-3-pyrrolidinecarboxamide hydrochloride

By reactions and purification similar to those in Example 31 using the compound obtained in Reference Example 25, the title compound was obtained, yield 60%.
¹H NMR (D₂O) δ 1.45-1.58 (2H, m), 1.69-1.85 (5H, m), 2.06-2.19 (2H, m), 2.39-2.54 (3H, m), 2.64 (3H, s), 2.70-3.05 (4H, m), 3.12-3.46 (4H, m), 3.63-3.71 (2H, m), 7.16-7.34 (5H, m), 7.47-7.61 (4H, m).
Anal. Calcd for C₂₈H₃₄F₃N₃O₃·HCl·0.6H₂O: C, 59.53; H, 6.46; N, 7.44. Found: C, 59.31; H, 6.54; N, 7.70.

### Example 46

### N-[3-(4-benzyl-1-piperidinyl)propyl]-1-methyl-N-(1-naphthyl)-5-oxo-3-pyrrolidinecarboxamide hydrochloride

By reactions and purification similar to those in Example 31 using the compound obtained in Reference Example 26, the title compound was obtained, yield 67%.
¹H NMR (D₂O) δ 1.43-1.56 (2H, m), 1.86-2.10 (5H, m), 2.58-2.80 (6H, m), 2.86-3.40 (8H, m), 3.47-3.57 (4H, m), 7.23-7.40 (5H, m), 7.54-7.82 (5H, m), 8.09-8.13 (2H, m).
Anal. Calcd for C₃₁H₃₇N₃O₂·HCl·1.5H₂O: C, 68.05; H, 7.55; N, 7.68. Found: C, 67.79; H, 7.47; N, 7.62.

### Example 47

### N-[3-(4-benzyl-1-piperidinyl)propyl]-N-(3-biphenyl)-1-methyl-5-oxo-3-pyrrolidinecarboxamide hydrochloride

By reactions and purification similar to those in Example 31 using the compound obtained in Reference Example 27, the title compound was obtained, yield 85%.
¹H NMR (DMSO-*d*₆) δ 1.3-2.0 (7H, m) , 2.14 (1H, dd, J=9.5, 17.3Hz), 2.4-2.6 (3H, m), 2.6-3.5 (9H, m), 2.63 (3H, s), 3.6-3.85 (2H, m), 7.1-7.8 (14H, m).
Anal. Calcd for C₃₃H₃₉N₃O₂·HCl·0.5H₂O: C, 71.40; H, 7.44; Cl, 6.39; N, 7.57. Found: C, 71.31; H, 7.49; Cl, 6.37; N, 7.53.

### Example 48

### N-[3-(benzyloxy)phenyl]-N-[3-(4-benzyl-1-piperidinyl)propyl]-1-methyl-5-oxo-3-pyrrolidinecarboxamide hydrochloride

By reactions and purification similar to those in Example 31 using the compound obtained in Reference Example 28, the title compound was obtained, yield 82%.
¹H NMR (DMSO-*d*₆) 8 1.3-1.95 (7H, m), 2.09 (1H, dd, J=10.0, 17.2Hz), 2.35-2.6 (3H, m), 2.6-3.5 (9H, m), 2.63 (3H, s), 3.55-3.75 (2H, m) , 5.17 (2H, s) , 6.9-7.55 (14H, m).
Anal. Calcd for C₃₄H₄₁N₃O₃·HCl·0.5H₂O: C, 69.78; H, 7.41; Cl, 6.06; N, 7.18. Found: C, 69.72; H, 7.42; Cl, 5.94; N, 7.16.

### Example 49

### N-[4-(benzyloxy)phenyl]-N-[3-(4-benzyl-1-piperidinyl)propyl]-1-methyl-5-oxo-3-pyrrolidinecarboxamide hydrochloride

By reactions and purification similar to those in Example 31 using the compound obtained in Reference Example 29, the title compound was obtained, yield 78%.
¹H NMR (DMSO-*d*₆) δ 1.3-1.95 (7H, m) , 2.10 (1H, dd, J=9.4, 16.8Hz), 2.35-2.6 (3H, m), 2.6-3.5 (9H, m), 2.63 (3H, s), 3.5-3.75 (2H, m), 5.13 (2H, s), 7.05-7.55 (14H, m).
Anal. Calcd for C₃₄H₄₁N₃O₃·HCl·0.6H₂O: C, 69.57; H, 7.42; Cl, 6.04; N, 7.16. Found: C, 69.60; H, 7.38; Cl, 6.14; N, 7.18.

### Example 50

### N-[3-(4-benzyl-1-piperidinyl)propyl]-N-phenyl-trans-4-cotininecarboxamide dihydrochloride

By reactions and purification similar to those in Example 31 using the compound obtained in Reference Example 10 and trans-4-cotininecarboxylic acid, the title compound was obtained, yield 93%.
¹H NMR (D₂O) δ 1.42-1.48 (2H, m), 1.83-1.95 (5H, m), 2.60-2.63 (5H, m), 2.69-2.92 (5H, m), 3.02-3.60 (6H, m), 5.04 (1H, d, J=6.0Hz), 7.24-7.41 (10H, m), 7.97 (1H, t, J=7.4Hz), 8.24 (1H, d, J=8.4Hz), 8.55 (1H, d, J=1.8Hz), 8.77 (1H, d, J=5.2Hz).
Anal. Calcd for C₃₂H₃₈N₄O₂·2HCl·1.5H₂O: C, 62.94; H, 7.10; N, 9.18. Found: C, 62.80; H, 7.29; N, 8.88.

### Example 51

### 1-benzyl-N-[3-(4-benzyl-1-piperidinyl)propyl]-5-oxo-N-phenyl-3-pyrrolidinecarboxamide

By reactions and purification similar to those in Example 31 using the compounds obtained in Reference Example 10 and Reference Example 44, the title compound was obtained, yield 68% (oil).
¹H NMR (CDCl₃) δ 1.15-1.33 (2H, m), 1.40-1.86 (7H, m), 2.23-2.36 (3H, m), 2.50 (2H, d, *J* = 6.6 Hz), 2.68-2.90 (3H, m), 2.92-3.12 (2H, m), 3.53 (1H, dd, J = 7.6, 5.4 Hz), 3.64-3.72 (2H, m), 4.33 (1H, d, J = 14.6 Hz), 4.43 (1H, d, J = 14.6 Hz), 7.00-7.30 (15H, m).
Anal. Calcd for C₃₃H₃₉N₃O₂·0.5H₂O: C, 76.41; H, 7.77; N, 8.10. Found: C, 76.37; H, 7.63; N, 8.23.

### Example 52

### N-[3-(4-benzyl-1-piperidinyl)propyl]-5-oxo-N,1-diphenyl-3-pyrrolidinecarboxamide

By reactions and purification similar to those in Example 31 using the compounds obtained in Reference Example 10 and Reference Example 43, the title compound was obtained, yield 62% (oil).
¹H NMR (CDCl₃) δ 1.10-2.00 (9H, m) , 2.27-2.45 (3H, m), 2.51 (2H, d, J = 6.6 Hz), 2.81-2.99 (3H, m), 3.10-3.27 (1H, m), 3.62 (1H, t, J = 9.0 Hz), 3.71-3.79 (2H, m), 4.18 (1H, t, J = 9.0 Hz), 7.09-7.53 (15H, m).
Anal. Calcd for C₃₂H₃₇N₃O₂·0.5H₂O: C, 76.16; H, 7.59; N, 8.33. Found: C, 75.91; H, 7.85; N, 8.35.

### Example 53

### N-[3-(4-benzyl-1-piperidinyl)propyl]-1-cyclohexyl-5-oxo-N-phenyl-3-pyrrolidinecarboxamide

By reactions and purification similar to those in Example 31 using the compounds obtained in Reference Example 10 and Reference Example 45, the title compound was obtained, yield 57% (oil) .
¹H NMR (CDCl₃) δ 1.00-1.86 (19H, m), 2.15-2.32 (3H, m), 2.50 (2H, d, J = 6.6 Hz), 2.58-2.70 (1H, m), 2.67-3.06 (3H, m), 3.18 (1H, t, J = 9.0 Hz), 3.56-3.94 (4H, m), 7.10-7.50 (10H, m).
Anal. Calcd for C₃₂H₄₃N₃O₂·0.5H₂O: C, 75.26; H, 8.68; N, 8.23. Found: C, 75.19; H, 8.37; N, 8.32.

### Example 54

### N-[3-(4-benzyl-1-piperidinyl)propyl]-1-butyl-5-oxo-N-phenyl-3-pyrrolidinecarboxamide

By reactions and purification similar to those in Example 31 using the compounds obtained in Reference Example 10 and Reference Example 46, the title compound was obtained, yield 46% (oil).
¹H NMR (CDCl₃) δ 0.88 (3H, t, J = 7.2 Hz), 1.05-1.90 (13H, m), 2.22 (1H, dd, J = 16.8, 8.8 Hz), 2.28 (2H, t, J = 7.4 Hz), 2.50 (2H, d, J = 6.6 Hz), 2.66 (1H, dd, J = 16.8, 8.8 Hz), 2.75-2.90 (2H, m), 2.94-3.45 (4H, m), 3.62-3.75 (3H, m), 7.10-7.50 (10H, m).
Anal. Calcd for C₃₀H₄₁N₃O₂·0.5H₂O: C, 74.34; H, 8.73; N, 8.67. Found: C, 74.60; H, 8.77; N, 8.89.

### Example 55

### N-[3-(4-benzyl-1-piperidinyl)propyl]-5-oxo-1-phenethyl-N-phenyl-3-pyrrolidinecarboxamide

By reactions and purification similar to those in Example 31 using the compounds obtained in Reference Example 10 and Reference Example 47, the title compound was obtained, yield 59% (oil).
¹H NMR (CDCl₃) δ 1.12-1.37 (2H, m), 1.38-1.90 (7H, m), 2.13-2.31 (3H, m), 2.51 (2H, d, J = 6.6 Hz), 2.61-2.85 (5H, m), 2.92-3.06 (2H, m), 3.44 (2H, t like, J = 7.4 Hz), 3.54-3.59 (1H, m), 3.69 (2H, t like, J = 7.4 Hz), 7.07-7.44 (15H, m).

### Example 56

### N-[3-(4-benzyl-1-piperidinyl)propyl]-5-oxo-N-phenyl-1-(3-phenylpropyl)-3-pyrrolidinecarboxamide

By reactions and purification similar to those in Example 31 using the compounds obtained in Reference Example 10 and Reference Example 48, the title compound was obtained, yield 84% (oil).
¹H NMR (CDCl₃) δ 1.10-1.31 (2H, m), 1.35-1.91 (9H, m), 2.13-2.32 (3H, m), 2.49-2.71 (5H, m), 2.80-3.03 (3H, m), 3.13 (1H, t, J = 9.0 Hz), 3.22-3.43 (2H, m), 3.59-3.74 (3H, m), 7.10-7.48 (15H, m).

### Example 57

### N-[3-(4-benzyl-1-piperidinyl)propyl]-1-(4-methoxybenzyl)-5-oxo-N-phenyl-3-pyrrolidinecarboxamide

By reactions and purification similar to those in Example 31 using the compounds obtained in Reference Example 10 and Reference Example 49, the title compound was obtained, yield 81% (oil).
¹H NMR (CDCl₃) δ 1.15-1.85 (9H, m), 2.05-2.34 (3H, m), 2.50 (2H, d, J = 6.6 Hz), 2.65-2.83 (3H, m), 2.94-3.10 (2H, m), 3.51 (1H, dd, J = 8.0, 5.8 Hz), 3.64-3.72 (2H, m), 3.78 (3H, s), 4.27 (1H, d, J = 14.8 Hz), 4.36 (1H, d, J = 14.8 Hz), 6.80-6.86 (2H, m), 7.07-7.45 (12H, m).

### Example 58

### N-[3-(4-benzyl-1-piperidinyl)propyl]-5-oxo-N-phenyl-3-pyrrolidinecarboxamide

To a mixed solution of the compound (65 mg, 0.12 mmol) obtained in Example 57 in acetonitrile/water (1.5 mL/0.5 mL) was added CAN (132 mg, 0.24 mmol) at 0°C and the mixture was stirred at room temperature for 1 h. CAN (66 mg, 0.12 mmol) was added and the mixture was stirred at room temperature for 14 h. Water (5 mL) was added to the reaction mixture and the mixture was extracted with ethyl acetate (10 mL×2). The organic layer was washed with saturated aqueous sodium hydrogencarbonate solution (10 mL), dried over anhydrous magnesium sulfate, filtrated and concentrated under reduced pressure. The obtained oil was purified by column chromatography (basic alumina activity III, 20 g, eluted with ethyl acetate/methanol = 9/1) to give the title compound (25 mg, 50%, oil).
¹H NMR (CDCl₃) δ 1.10-1.33 (2H, m), 1.38-1.87 (7H, m), 2.08-2.32 (3H, m), 2.51 (2H, d, J = 6.6 Hz), 2.59-2.85 (3H, m), 3.09-3.28 (2H, m), 3.55-3.75 (3H, m), 5.42 (1H, br), 7.10-7.49 (10H, m).
MS m/z = 420 (MH⁺).

### Example 59

### 1-benzyl-N-[3-(4-benzyl-1-piperidinyl)propyl]-N-(3,4-dichlorophenyl)-5-oxo-3-pyrrolidinecarboxamide

By reactions and purification similar to those in Example 31 using the compounds obtained in Reference Example 18 and Reference Example 44, the title compound was obtained, yield 58% (oil).
¹H NMR (CDCl₃) δ 1.10-1.38 (2H, m), 1.38-1.86 (7H, m), 2.22-2.40 (3H, m), 2.50 (2H, d, J = 6.6 Hz), 2.66-2.82 (3H, m), 2.90-3.15 (2H, m), 3.45-3.70 (3H, m), 4.34 (1H, d, J = 14.8 Hz), 4.46 (1H, d, J = 14.8 Hz), 6.97 (1H, dd, J = 8.6, 2.6 Hz), 7.10-7.40 (11H, m), 7.49 (1H, d, J = 8.6 Hz).

### Example 60

### N-[3-(4-benzyl-1-piperidinyl)propyl]-N-(3,4-dichlorophenyl)-5-oxo-1-phenethyl-3-pyrrolidinecarboxamide

By reactions and purification similar to those in Example 31 using the compounds obtained in Reference Example 18 and Reference Example 47, the title compound was obtained, yield 40% (oil).
¹H NMR (CDCl₃) δ 1.10-1.35 (2H, m), 1.37-1.87 (7H, m), 2.17-2.30 (3H, m), 2.51 (2H, d, J = 6.6 Hz), 2.61-3.04 (6H, m), 3.41-3.55 (4H, m), 3.62-3.69 (2H, m), 6.96 (1H, dd, J = 8.8, 2.6 Hz), 7.11-7.31 (11H, m), 7.51 (1H, d, J = 8.8 Hz).

### Example 61

### N-[3-(4-benzyl-1-piperidinyl)propyl]-N-(3,4-dichlorophenyl)-5-oxo-1-(3-phenylpropyl)-3-pyrrolidinecarboxamide

By reactions and purification similar to those in Example 31 using the compounds obtained in Reference Example 18 and Reference Example 48, the title compound was obtained, yield 75% (oil).
¹H NMR (CDCl₃) δ 1.10-1.37 (2H, m), 1.38-1.85 (9H, m), 2.15-2.30 (3H, m), 2.49-2.68 (5H, m), 2.78-2.98 (3H, m), 3.16 (1H, t, J = 9.0 Hz), 3.29 (2H, t, J = 7.0 Hz), 3.58-3.71 (3H, m), 7.00 (1H, dd, J = 8.4, 2.6 Hz), 7.03-7.31 (11H, m), 7.53 (1H, d, J = 8.4 Hz).

### Example 62

### N-benzyl-N-[3-(4-benzyl-1-piperidinyl)propyl]-1-methyl-5-oxo-3-pyrrolidinecarboxamide

To a solution of the compound (200 mg, 0.62 mmol) obtained in Reference Example 32 in acetonitrile (6 mL) were added 1-methyl-5-oxo-3-pyrrolidinecarboxylic acid (89 mg, 0.62 mmol) and, 1-hydroxybenzotriazole monohydrate (104 mg, 0.68 mmol), and dicyclohexylcarbodiimide (141 mg, 0.68 mmol) was added. This mixture was stirred at 80°C for 1 h. After cooling, the reaction mixture was concentrated under reduced pressure, and ethyl acetate (20 mL) was added, and an insoluble material was filtered off. The mother liquor was washed with 2N aqueous sodium hydroxide solution (5 mL), dried over anhydrous magnesium sulfate and concentrated under reduced pressure. The obtained oil was purified by column chromatography (basic alumina activity III, 35 g, eluted with ethyl acetate ) to give the title compound (125 mg, 45%, oil).
¹H NMR (CDCl₃) (ca. 1:1 isomer mixture) δ 1.10-1.40 (2H, m) , 1.41-1.88 (7H, m), 2.19-2.78 (8H, m), 2.80 (1.5H, s), 2.88 (1.5H, s), 3.21-3.82 (5H, m), 4.48-4.73 (2H, m), 7.11-7.37 (10H, m).
Anal. Calcd for C₂₈H₃₇N₃O₂·0.25H₂O: C, 74.38; H, 8.36; N, 9.29. Found: C, 74.38; H, 8.49; N, 9.09.

### Example 63

### N-[3-(4-benzyl-1-piperidinyl)propyl]-N-(4-hydroxybenzyl)-1-methyl-5-oxo-3-pyrrolidinecarboxamide

By reactions and purification similar to those in Example 62 using the compounds obtained in Reference Example 33, the title compound was obtained, yield 45% (oil).
¹H NMR (CDCl₃) δ 1.10-2.00 (11H, m), 2.18-2.90 (9H, m), 3.20-3.83 (5H, m), 4.32 (1H, d, J = 14.4 Hz), 4.41 (1H, s), 4.69 (1H, d, J = 14.4 Hz), 6.69-6.76 (2H, m), 6.90 (1H, d, J = 8.4 Hz), 7.02 (1H, d, J = 8.4 Hz), 7.11-7.32 (5H, m).

### Example 64

### N-[3-(4-benzyl-1-piperidinyl)propyl]-1-methyl-N-(1-naphthylmethyl)-5-oxo-3-pyrrolidinecarboxamide

By reactions and purification similar to those in Example 31 using the compounds obtained in Reference Example 34, the title compound was obtained, yield 87% (oil).
¹H NMR (CDCl₃) (ca. 0.4:0.6 isomer mixture) δ 1.10-1.38 (2H, m), 1.39-1.93 (7H, m), 2.17 (0.60×2H, t like, J = 6.8 Hz), 2.32 (0.40×2H, t like, J = 7.4 Hz), 2.49-3.00 (9H, m), 3.10-3.83 (5H, m), 5.00-5.23 (2H, m), 7.11-7.60 (9H, m), 7.80-8.00 (3H, m) .

### Example 65

### N-[3-(4-benzyl-1-piperidinyl)propyl]-1-methyl-N-(2-naphthylmethyl)-5-oxo-3-pyrrolidinecarboxamide

By reactions and purification similar to those in Example 62 using the compounds obtained in Reference Example 35, the title compound was obtained, yield 64% (oil).
¹H NMR (CDCl₃) (ca. 1:1 isomer mixture) δ 1.06-2.00 (9H, m), 2.17-2.34 (2H, m), 2.41-2.56 (3H, m), 2.60-2.89 (6H, m), 3.20-3.84 (5H, m), 4.66-4.89 (2H, m), 7.11-7.88 (12H, m).

### Example 66

### N-[3-(4-benzyl-1-piperidinyl)propyl]-N-(2,3-dihydro-lH-indene-2-yl)-1-methyl-5-oxo-3-pyrrolidinecarboxamide

By reactions and purification similar to those in Example 31 using the compounds obtained in Reference Example 41, the title compound was obtained, yield 54% (oil).
¹H NMR (CDCl₃) (ca. 1:1 isomer mixture) δ 1.00-1.90 (9H, m), 2.14-2.30 (2H, m), 2.50 (2H, d, J = 6.2Hz), 2.59-2.80 (4H, m), 2.86 (0.5×3H, s), 2.87 (0.5×3H, s), 2.98-3.17 (4H, m), 3.20-3.30 (2H, m), 3.40-3.59 (2H, m), 3.69-3.82 (1H, m), 4.60-4.80 (0.5H, m), 5.01-5.16 (0.5H, m), 7.10-7.27 (9H, m).

### Example 67

### N-benzyl-N-{3-[4-(4-chlorophenyl)-4-hydroxy-1-piperidinyl]propyl}-1-methyl-5-oxo-3-pyrrolidinecarboxamide

By reactions and purification similar to those in Example 62 using the compounds obtained in Reference Example 36, the title compound was obtained, yield 54% (oil).
¹H NMR (CDCl₃) (ca. 0.4:0.6 isomer mixture) δ 1.60-1.90 (5H, m), 1.90-2.20 (2H, m), 2.30-2.53 (5H, m), 2.60-2.80 (3H, m), 2.82 (0.6×3H, s), 2.87 (0.4×3H, s), 3.27-3.90 (5H, m), 4.54-4.75 (2H, m) , 7.13-7.46 (9H, m).

### Example 68

### N-{3-[4-(4-chlorophenyl)-4-hydroxy-1-piperidinyl]propyl}-N-isopropyl-1-methyl-5-oxo-3-pyrrolidinecarboxamide

By reactions and purification similar to those in Example 62 using the compounds obtained in Reference Example 37, the title compound was obtained, yield 11% (oil).
¹H NMR (CDCl₃) (ca. 0.35:0.65 isomer mixture) δ 1.18 (0.35×6H, d, J = 7.0 Hz), 1.24 (0.65×6H, d, J = 7.0 Hz), 1.60-1.90 (4H, m), 2.00-2.23 (2H, m), 2.40-2.95 (11+0.65H, m), 3.24 (2H, dd, J = 10.0, 6.0 Hz), 3.38-3.55 (2+0.35H, m), 3.60-3.85 (1H, m), 3.90-4.10 (0.65H, m), 4.55-4.70 (0.35H, m), 7.28-7.50 (4H, m).

### Example 69

### N-{3-[4-(4-chlorophenyl)-4-hydroxy-1-piperidinyl]propyl}-N-cyclohexyl-1-methyl-5-oxo-3-pyrrolidinecarboxamide

By reactions and purification similar to those in Example 31 using the compounds obtained in Reference Example 38, the title compound was obtained, yield 57% (oil).
¹H NMR (CDCl₃) δ 1.00-2.20 (15H, m), 2.37-3.00 (12H, m), 3.15-4.40 (7H, m), 7.29-7.48 (4H, m).

### Example 70

### N-{3-[4-(4-chlorophenyl)-4-hydroxy-1-piperidinyl)propyl}-N-cyclopentyl-1-methyl-5-oxo-3-pyrrolidinecarboxamide

By reactions and purification similar to those in Example 31 using the compounds obtained in Reference Example 39, the title compound was obtained, yield 77% (oil).
¹H NMR (CDCl₃) (ca. 0.3:0.7 isomer mixture) δ 0.80-2.00 (11H, m), 2.02-2.20 (2H, m), 2.30-2.80 (9H, m), 2.85 (3H, s), 3.15-3.35 (2H, m), 3.37-3.55 (3H, m), 3.57-3.85 (1H, m), 3.95-4.20 (0.7H, m), 4.35-4.60 (0.3H, m) , 7.29-7.50 (4H, m).

### Example 71

### N-{3-[4-(4-fluorobenzoyl)-1-piperidinyl]-2-hydroxypropyl}-1-methyl-5-oxo-N-phenyl-3-pyrrolidinecarboxamide

By reactions and purification similar to those in Example 31 using the compounds obtained in Reference Example 42, the title compound was obtained, yield 50% (oil).
¹H NMR (CDCl₃) δ 1.76-2.50 (9H, m), 2.61-3.26 (6H, m), 2.78 (3H, s), 3.51-4.01 (5H, m) , 7.10-7.46 (7H, m), 7.92-8.00 (2H, m). Mass : MH⁺ = 482

### Example 72

### 1-benzyl-N-[3-(4-benzyl-1-piperidinyl)propyl]-N-(1-naphthylmethyl)-5-oxo-3-pyrrolidinecarboxamide

By reactions and purification similar to those in Example 31 using the compound obtained in Reference Example 44, the title compound was obtained, yield 82% (oil).
¹H NMR (CDCl₃) (ca. 0.4:0.6 isomer mixture) δ 1.00-1.35 (2H, m), 1.36-1.90 (7H, m), 2.14 (0.60×2H, t like, J = 6.6 Hz), 2.29 (0.40×2H, t like, J = 7.5 Hz), 2.49 (2H, d, J = 6.6 Hz), 2.55-2.97 (4H, m), 3.09-3.70 (5H, m), 4.30-4.67 (2H, m), 5.02 (0.8H, s), 5.09 (1.2H, s), 7.11-7.60 (14H, m), 7.78-7.95 (3H, m).

### Example 73

### N-[3-(4-benzyl-1-piperidinyl)propyl]-1-(cyclohexylmethyl)-5-oxo-N-phenyl-3-pyrrolidinecarboxamide

By reactions and purification similar to those in Example 31 using the compounds obtained in Reference Example 10 and Reference Example 52, the title compound was obtained, yield 70% (oil).
¹H NMR (CDCl₃) δ 0.80-1.03 (2H, m), 1.04-1.38 (5H, m), 1.39-1.90 (13H, m), 2.16-2.32 (3H, m), 2.51 (2H, d, J = 6.6 Hz), 2.61-3.20 (7H, m), 3.63-3.75 (3H, m), 7.10-7.50 (10H, m).

### Example 74

### N-[3-(4-benzyl-1-piperidinyl)propyl]-1-(4-fluorobenzyl)-5-oxo-N-phenyl-3-pyrrolidinecarboxamide

By reactions and purification similar to those in Example 31 using the compounds obtained in Reference Example 10 and Reference Example 51, the title compound was obtained, yield 82% (oil).
¹H NMR (CDCl₃) δ 1.10-1.38 (2H, m), 1.39-1.85 (7H, m), 2.23-2.36 (3H, m), 2.50 (2H, d, J = 6.6 Hz), 2.66-2.80 (3H, m), 2.96-3.10 (2H, m), 3.45-3.72 (3H, m), 4.35 (2H, s), 6.94-7.50 (14H, m).

### Example 75

### N-[3-(4-benzyl-1-piperidinyl)propyl]-5-oxo-N-phenyl-1-(4-pyridylmethyl)-3-pyrrolidinecarboxamide

By reactions and purification similar to those in Example 31 using the compounds obtained in Reference Example 10 and Reference Example 50, the title compound was obtained, yield 63% (oil).
¹H NMR (CDCl₃) δ 1.00-1.86 (9H, m), 2.24-2.41 (3H, m), 2.50 (2H, d, J = 6.2 Hz), 2.70-2.90 (3H, m), 3.02-3.15 (2H, m), 3.50-3.74 (3H, m), 4.40 (2H, s), 7.05-7.50 (12H, m), 8.55 (2H, d, J = 5.8 Hz).

### Example 76

### N-[3-(4-benzyl-1-piperidinyl)propyl]-1-(2-chlorobenzyl)-5-oxo-N-phenyl-3-pyrrolidinecarboxamide

By reactions and purification similar to those in Example 31 using the compounds obtained in Reference Example 10 and Reference Example 53, the title compound was obtained, yield 72% (oil).
¹H NMR (CDCl₃) δ 1.1-1.35 (2H, m), 1.35-1.85 (7H, m), 2.23-2.37 (3H, m), 2.50 (2H, d, J = 6.6 Hz), 2.69-2.90 (3H, m), 2.96-3.18 (2H, m), 3.58 (1H, dd, J = 8.4, 6.2 Hz), 3.69 (2H, t, J = 7.8 Hz), 4.48 (1H, d, J = 15.2 Hz), 4.58 (1H, d, J = 15.2 Hz), 7.10-7.64 (14H, m).

### Example 77

### N-[3-(4-benzyl-1-piperidinyl)propyl]-1-(3-chlorobenzyl)-5-oxo-N-phenyl-3-pyrrolidinecarboxamide

By reactions and purification similar to those in Example 31 using the compounds obtained in Reference Example 10 and Reference Example 54, the title compound was obtained, yield 81% (oil).
¹H NMR (CDCl₃) δ 1.1-1.9 (9H, m), 2.23-2.37 (3H, m), 2.50 (2H, d, J = 6.6 Hz), 2.67-2.83 (3H, m), 2.98-3.12 (2H, m), 3.5-3.6 (1H, m), 3.69 (2H, t like, J = 7.6 Hz), 4.30 (1H, d, J = 14.6 Hz), 4.41 (1H, d, J = 14.6 Hz), 7.0-7.5 (14H, m).

### Example 78

### N-[3-(4-benzyl-1-piperidinyl)propyl]-1-(4-chlorobenzyl)-5-oxo-N-phenyl-3-pyrrolidinecarboxamide

By reactions and purification similar to those in Example 31 using the compounds obtained in Reference Example 10 and Reference Example 55, the title compound was obtained, yield 78% (oil).
¹H NMR (CDCl₃) δ 1.1-1.9 (9H, m), 2.23-2.36 (3H, m), 2.50 (2H, d, J = 6.6 Hz), 2.67-2.83 (3H, m), 2.96-3.10 (2H, m), 3.5-3.6 (1H, m), 3.69 (2H, t like, J = 7.4 Hz), 4.35 (2H, s), 7.0-7.5 (14H, m).

### Example 79

### N-[3-(4-benzyl-1-piperidinyl)propyl]-5-oxo-N-phenyl-1-[4-(trifluoromethyl)benzyl]-3-pyrrolidinecarboxamide

By reactions and purification similar to those in Example 31 using the compounds obtained in Reference Example 10 and Reference Example 56, the title compound was obtained, yield 63% (oil).
¹H NMR (CDCl₃) δ 1.15-1.30 (2H, m), 1.35-1.85 (7H, m), 2.23-2.38 (3H, m), 2.50 (2H, d, J = 6.6 Hz), 2.68-2.90 (3H, m), 2.99-3.13 (2H, m), 3.50-3.73 (3H, m), 4.44 (2H, s), 7.08-7.50 (12H, m), 7.57 (2H, d, J = 8.4 Hz).

### Example 80

### N-[3-(4-benzyl-1-piperidinyl)propyl]-1-(2-morpholinoethyl)-5-oxo-N-phenyl-3-pyrrolidinecarboxamide

By reactions and purification similar to those in Example 31 using the compounds obtained in Reference Example 10 and Reference Example 57, the title compound was obtained, yield 74% (oil).
¹H NMR (CDCl₃) δ 1.0-1.9 (11H, m), 2.16-2.52 (10H, m), 2.68 (1H, dd, J = 17.0, 8.8 Hz), 2.82 (2H, br d, J = 11.4 Hz), 2.97-3.10 (1H, m), 3.22-3.50 (3H, m), 3.50-3.80 (6H, m), 7.0-7.6 (10H, m).

### Example 81

### N-[3-(4-benzyl-1-piperidinyl)propyl]-1-(2-furylmethyl)-5-oxo-N-phenyl-3-pyrrolidinecarboxamide

By reactions and purification similar to those in Example 31 using the compounds obtained in Reference Example 10 and Reference Example 58, the title compound was obtained, yield 18% (oil).
¹H NMR (CDCl₃) δ 1.15-1.33 (2H, m), 1.40-1.86 (7H, m), 2.19-2.31 (3H, m), 2.50 (2H, d, J = 6.6 Hz), 2.68 (1H, t, J = 8.8 Hz), 2.81 (2H, br d, J = 11.4 Hz), 2.92-3.10 (1H, m), 3.18 (1H, t, J = 8.8 Hz), 3.57-3.73 (3H, m), 4.31 (1H, d, J = 15.4 Hz), 4.44 (1H, d, J = 15.4 Hz), 6.20-6.30 (2H, m), 7.10-7.50 (11H, m) .

### Example 82

### N-[3-(4-benzyl-1-piperidinyl)propyl]-1-(4-methylbenzyl)-5-oxo-N-phenyl-3-pyrrolidinecarboxamide

By reactions and purification similar to those in Example 31 using the compounds obtained in Reference Example 10 and Reference Example 59, the title compound was obtained, yield 40% (oil).
¹H NMR (CDCl₃) δ 1.1-1.37 (2H, m), 1.37-1.88 (7H, m), 2.32 (3H, s), 2.21-2.37 (3H, m), 2.50 (2H, d, J = 6.6 Hz), 2.66-2.88 (3H, m), 2.95-3.15 (2H, m), 3.45-3.60 (1H, m), 3.65 (2H, t like, J = 8.0 Hz), 4.44 (2H, s), 7.05-7.60 (14H, m).

### Example 83

### N-{3-[4-(4-fluorobenzyl)-1-piperidinyl]propyl}-1-methyl-5-oxo-N-phenyl-3-pyrrolidinecarboxamide hydrochloride

By reactions and purification similar to those in Example 31 using the compound obtained in Reference Example 64, the title compound was obtained, yield 43% (oil).
¹H NMR (CDCl₃) δ 1.3-1.7 (2H, m), 1.75-2.10 (5H, m), 2.31 (1H, dd, J = 17.2, 9.6 Hz), 2.56-2.71 (3H, m), 2.77 (3H, s), 2.92 (2H, t like, J = 12.4 Hz), 3.09-3.36 (4H, m), 3.53-3.70 (3H, m), 3.70-3.90 (2H, m), 6.97-7.10 (2H, m), 7.17-7.24 (2H, m), 7.34-7.60 (5H, m).

### Example 84

### N-(3,4-dichlorophenyl)-N-{3-[4- (4-fluorobenzyl)-1-piperidinyl]propyl}-1-methyl-5-oxo-3-pyrrolidinecarboxamide hydrochloride

By reactions and purification similar to those in Example 31 using the compound obtained in Reference Example 65, the title compound was obtained, yield 65% (oil).
¹H NMR (CD₃OD) δ 1.4-1.7 (2H, m), 1.70-2.10 (5H, m), 2.36 (1H, dd, J = 17.2, 9.8 Hz), 2.50-2.70 (3H, m), 2.78 (3H, s), 2.92 (2H, t like, J = 12.0 Hz), 3.08-3.60 (4H, m), 3.50-3.70 (3H, m), 3.70-3.90 (2H, m), 7.02 (2H, t, J = 8.8 Hz), 7.17-7.24 (2H, m), 7.35 (1H, dd, J = 8.4, 2.2 Hz), 7.68-7.72 (2H, m).

### Example 85

### 1-benzyl-N-[3-(4-benzyl-1-piperidinyl)propyl]-N-(3-chlorophenyl)-5-oxo-3-pyrrolidinecarboxamide

By reactions and purification similar to those in Example 31 using the compounds obtained in Reference Example 17 and Reference Example 44, the title compound was obtained, yield 39% (oil).
¹H NMR (CDCl₃) δ 1.10-1.30 (2H, m), 1.30-1.85 (7H, m), 2.23-2.38 (3H, m), 2.50 (2H, d, J = 6.6 Hz), 2.68-2.85 (3H, m), 2.96-3.13 (2H, m), 3.48-3.70 (3H, m), 4.48 (2H, s), 7.08-7.60 (14H, m).

### Example 86

### N-[3-(4-benzyl-1-piperidinyl)propyl]-1-(2,6-difluorobenzyl)-5-oxo-N-phenyl-3-pyrrolidinecarboxamide

By reactions and purification similar to those in Example 31 using the compounds obtained in Reference Example 10 and Reference Example 60, the title compound was obtained, yield 76% (oil).
¹H NMR (CDCl₃) δ 1.2-1.9 (9H, m), 2.23-2.30 (3H, m), 2.51 (2H, d, J = 6.6 Hz), 2.60-2.73 (1H, m), 2.81 (2H, br d, J = 11.0 Hz), 2.95-3.14 (2H, m), 3.55 (1H, t, J = 7.7 Hz), 3.68 (2H, t like, J = 7.5 Hz), 4.52 (2H, s), 6.88 (2H, t, J = 7.0 Hz), 7.09-7.40 (11H, m).

### Example 87

### 1-benzyl-N-[3-(4-benzyl-1-piperidinyl)propyl]-N-(2,3-dihydro-1H-inden-1-yl)-5-oxo-3-pyrrolidinecarboxamide

By reactions and purification similar to those in Example 31 using the compounds obtained in Reference Example 62 and Reference Example 44, the title compound was obtained, yield 67% (oil).
¹H NMR (CDCl₃) (ca. 1:1 isomer mixture) δ 1.0-2.2 (11H, m), 2.3-3.8 (13H, m), 2.49 (2H, d, J = 6.6 Hz), 4.30-4.70 (2H, m), 5.25-5.40 (0.5H, m), 6.00-6.10 (0.5H, m), 6.91-7.50 (14H, m).

### Example 88

### 1-benzyl-N-[3-(4-benzyl-1-piperidinyl)propyl]-5-oxo-N-(1,2,3,4-tetrahydro-1-naphthyl)-3-pyrrolidinecarboxamide

By reactions and purification similar to those in Example 31 using the compounds obtained in Reference Example 63 and Reference Example 44, the title compound was obtained, yield 73% (oil).
¹H NMR (CDCl₃) (ca. 0.4:0.6 isomer mixture) δ 1.0-2.2 (12H, m), 2.25-2.40 (1H, m), 2.52 (2H, d, J = 5.8 Hz), 2.60-3.80 (13H, m), 4.30-4.60 (2H, m), 4.80-4.95 (0.6H, m), 5.60-5.80 (0.4H, m), 6.76-7.40 (14H, m).

### Example 89

### 1-benzyl-N-[3-(4-benzyl-1-piperidinyl)propyl]-N-(3,4-dichlorophenyl)-6-oxo-3-piperidine carboxamide

By reactions and purification similar to those in Example 31 using the compounds obtained in Reference Example 18 and Reference Example 61, the title compound was obtained, yield 77% (oil).
¹H NMR (CDCl₃) δ 1.20-1.35 (2H, m), 1.35-1.9 (7H, m), 1.9-2.2 (2H, m), 2.2-2.3 (3H, m), 2.4-2.65 (4H, m), 2.79 (2H, br d, J = 11.8 Hz), 2.95-3.10 (1H, m), 3.38-3.70 (3H, m), 4.31 (1H, d, J = 9.0 Hz), 4.74 (1H, d, J = 9.0 Hz), 6.85-6.95 (1H, m), 7.1-7.31 (11H, m), 7.40 (1H, d, J = 8.4 Hz).

### Example 90

### N-[3-(4-benzyl-1-piperidinyl)propyl]-5-oxo-N-phenyl-1-propargyl-3-pyrrolidinecarboxamide

To a solution of the compound (100 mg, 0.24 mmol) obtained in Example 58 in DMF (1.5 ml), was added sodium hydride (60%, 12.4 mg, 0.31 mmol) under ice-cooling and the mixture was stirred at room temperature for 30 min. Then propargyl bromide (34 mg, 0.29 mmol) was added and the mixture was stirred at room temperature for 1 h. DMF was evaporated under reduced pressure and the mixture was extracted with ethyl acetate (10 ml×2) and 1N-aqueous sodium hydroxide solution (10 ml). The organic layer was dried over anhydrous magnesium sulfate and concentrated under reduced pressure. The residue was subjected to column chromatography (basic alumina activity III, 3 g, ethyl acetate/hexane=1/1→1/0). The objective fraction was concentrated under reduced pressure to give the title compound (77 mg, 71%) as a colorless oil.
¹H NMR (CDCl₃) δ 1.1-1.9 (9H, m), 2.19 (1H, t, J = 2.6 Hz), 2.24-2.37 (3H, m), 2.51 (2H, d, J = 6.6 Hz), 2.71 (1H, dd, J = 16.8, 8.8 Hz), 2.87 (2H, br d, J = 11.0 Hz), 3.00-3.17 (1H, m), 3.34 (1H, t, J = 8.8 Hz), 3.65-3.76 (3H, m), 3.94 (1H, dd, J = 17.6, 2.6 Hz), 4.12 (1H, dd, J = 17.6, 2.6 Hz), 7.11-7.50 (10H, m) .

### Example 91

### N-[3-(4-benzyl-1-piperidinyl)propyl]-1-(2-methylbenzyl)-5-oxo-N-phenyl-3-pyrrolidinecarboxamide

By reactions and purification similar to those in Example 90 using 2-methylbenzylbromide, the title compound was obtained, yield 63% (oil).
¹H NMR (CDCl₃) δ 1.1-1.9 (9H, m), 2.25 (3H, s), 2.2-2.36 (3H, m), 2.50 (2H, d, J = 6.4 Hz), 2.67-2.85 (3H, m), 2.95-3.10 (2H, m), 3.4-3.6 (1H, m), 3.67 (2H, t like, J = 7.8 Hz), 4.40 (2H, s), 7.0-7.5 (14H, m).

### Example 92

### N-[3-(4-benzyl-1-piperidinyl)propyl]-1-(2-fluorobenzyl)-5-oxo-N-phenyl-3-pyrrolidinecarboxamide

By reactions and purification similar to those in Example 90 using 2-fluorobenzylbromide, the title compound was obtained, yield 83% (oil).
¹H NMR (CDCl₃) δ 1.1-2.0 (9H, m), 2.21-2.34 (3H, m), 2.50 (2H, d, J = 6.6 Hz), 2.66-2.89 (3H, m), 2.9-3.1 (2H, m), 3.13 (2H, t, J = 8.8 Hz), 3.58 (1H, dd, J = 8.6, 6.8 Hz), 4.45 (2H, s), 6.97-7.50 (14H, m).

### Example 93

### N-[3-(4-benzyl-1-piperidinyl)propyl]-5-oxo-N-phenyl-1-(2,2,2-trifluoroethyl)-3-pyrrolidinecarboxamide

By reactions and purification similar to those in Example 90 using 2,2,2-trifluoroethyl triflate, the title compound was obtained, yield 30% (oil).
¹H NMR (CDCl₃) δ 1.15-1.35 (2H, m), 1.4-1.85 (7H, m), 2.22-2.36 (3H, m), 2.51 (2H, d, J = 6.2 Hz), 2.65-2.90 (3H, m), 3.03-3.20 (1H, m), 3.37 (1H, t, J = 8.4 Hz), 3.60-3.80 (4H, m) , 3.85-4.02 (1H, m), 7.10-7.30 (8H, m), 7.32-7.50 (2H, m).

### Example 94

### N-[3-(4-benzyl-1-piperidinyl)propyl]-N-(3-chlorophenyl)-5-oxo-1-[2-(trifluoromethyl)benzyl]-3-pyrrolidinecarboxamide

To a mixture of 1-(2,4-dimethoxybenzyl)-5-oxo-3-pyrrolidinecarboxylic acid (698 mg, 2.5 mmol) synthesized by reactions and purification similar to those in Example 43 using 2,4-dimethoxybenzylamine, DMF (0.024 ml) and dichloromethane (10 ml) was added oxalyl chloride (0.256 ml, 3.0 mmol) under ice-cooling and the mixture was stirred at the same temperature for 15 min and for 1 h while allowing the mixture to warm to room temperature. The obtained solution was added to a mixture of the compound (416 mg, 1.0 mmol) obtained in Reference Example 17, triethylamine (1.39 ml, 10 mmol) and dichloromethane (15 ml) at -20°C with stirring and the mixture was stirred for 1 h while allowing to warm to 0°C. A saturated aqueous sodium hydrogencarbonate solution (15 ml) was added, and the organic solvent was evaporated under reduced pressure and extracted with ethyl acetate (20 ml×2). The organic layer was washed successively with saturated aqueous sodium hydrogencarbonate solution (10 ml×2) and saturated brine (10 ml), dried over anhydrous magnesium sulfate and concentrated under reduced pressure. The residue was subjected to column chromatography (basic alumina activity III, 30 g, eluted with ethyl acetate/hexane=1/1). The objective fraction was concentrated under reduced pressure and the residue (200 mg) was dissolved in trifluoroacetic acid (4 ml) and the mixture was stirred at 70°C for 4 h. After concentration under reduced pressure, saturated aqueous sodium hydrogencarbonate solution (15 ml) was added and the mixture was extracted with ethyl acetate (20 ml×2). The organic layer was washed with saturated brine (20 ml), dried over anhydrous magnesium sulfate and concentrated under reduced pressure. The residue was subjected to column chromatography (basic alumina activity III, 10 g, ethyl acetate). The objective fraction was concentrated under reduced pressure to give N-[3-(4-benzyl-1-piperidinyl)propyl]-N-(3-chlorophenyl)-5-oxo-3-pyrrolidinecarboxamide (75 mg, 50%). By reactions and purification similar to those in Example 90 using this compound and 2-trifluorobenzylbromide, the title compound was obtained, yield 76% (oil).
¹H NMR (CDCl₃) δ 1.1-2.1 (9H, m), 2.26 (2H, t, J = 7.4 Hz), 2.31-2.44 (1H, m), 2.50 (2H, d, J = 6.6 Hz), 2.72-2.90 (3H, m), 2.96-3.16 (2H, m), 3.53 (1H, dd, J = 8.4, 5.8 Hz), 3.67 (2H, t, J = 7.8 Hz), 4.52 (1H, d, J = 15.8 Hz), 4.70 (1H, d, J = 15.8 Hz), 6.98-7.04 (1H, m), 6.98-7.04 (1H, m) , 7.10-7.39 (9H, m), 7.48-7.65 (2H, m).

### Example 95

### N-[3-(4-benzyl-1-piperidinyl)propyl]-1-methyl-5-oxo-N-[3-(trifluoromethyl)phenyl]-3-pyrrolidinecarboxamide hydrochloride

To a mixture of 1-methyl-5-oxo-3-pyrrolidinecarboxylic acid (358 mg, 2.5 mmol), DMF (0.023 ml) and dichloromethane (10 ml) was added oxalyl chloride (0.256 ml, 3.0 mmol) under ice-cooling and the mixture was stirred at the same temperature for 15 min and for 1 h while allowing the mixture to warm to room temperature. The obtained solution was added to a mixture of the compound (449 mg, 1.0 mmol) obtained in Reference Example 66, triethylamine (1.39 ml, 10 mmol) and dichloromethane (15 ml) at -20°C with stirring and the mixture was stirred for 1 h while allowing to warm to 0°C. A saturated aqueous sodium hydrogencarbonate solution (15 ml) was added, and the organic solvent was evaporated under reduced pressure and extracted with ethyl acetate (15 m1×3). The organic layer was washed successively with saturated aqueous sodium hydrogencarbonate solution (5 ml×3) and saturated brine (5 ml), dried over anhydrous magnesium sulfate and concentrated under reduced pressure. The residue was subjected to column chromatography (silica gel 10 g, ethyl acetate/methanol=1/0→9/1). The objective fraction was concentrated under reduced pressure to give a free base (383 mg) of the title compound.
¹H NMR (CDCl₃) δ 1.05-1.95 (9H, m), 2.15-2.35 (3H, m), 2.51 (2H, d, J=6.6Hz), 2.6-3.1 (4H, m) , 2.78 (3H, s), 3.19 (1H, t, J=9.1Hz), 3.6-3.8 (3H, m), 7.05-7.45 (7H, m), 7.55-7.75 (2H, m).

The free base (383 mg) was dissolved in methanol, 1N hydrogen chloride diethyl ether solution (2 ml) was added, and the mixture was concentrated under reduced pressure. Diethyl ether was added to the residue and the precipitate was collected by filtration. The precipitate was washed with diethyl ether and dried under reduced pressure to give the title compound (376 mg, 0.70 mmol, yield 70%) as a hygroscopic amorphous.
Anal. Calcd for C₂₈H₃₄F₃N₃O₂·HCl·0.6H₂O: C, 61.27; H, 6.65; Cl, 6.46; F, 10.38; N, 7.66. Found: C, 61.29; H, 6.60; Cl, 6.37; F, 10.44; N, 7.69.

### Example 96

### N-[3-(4-benzyl-1-piperidinyl)propyl]-1-methyl-N-(3-methylphenyl) -5-oxo-3-pyrrolidinecarboxamide hydrochloride

By reactions and purification similar to those in Example 95 using the compound (395 mg) obtained in Reference Example 67, a free base (420 mg) of the title compound was obtained.
¹H NMR (CDCl₃) δ 1.05-1.95 (9H, m), 2.1-2.4 (3H, m), 2.38 (3H, s), 2.51 (2H, d, J=6.6Hz), 2.55-2.9 (3H, m), 2.76 (3H, s), 2.95-3.25 (2H, m), 3.55-3.75 (3H, m), 6.85-7.0 (2H, m), 7.05-7.35 (7H, m).

The free base (420 mg) was converted to the title compound (405 mg) by a method similar to the method of Example 95.
Anal. Calcd for C₂₈H₃₇N₃O₂·HCl·0.5H₂O: C, 68.20; H, 7.97; Cl, 7.19; N, 8.52. Found: C, 68.18; H, 8.12; Cl, 7.10; N, 8.63.

### Example 97

### N-[3-(4-benzyl-1-piperidinyl)propyl]-1-methyl-N-(2-methylphenyl)-5-oxo-3-pyrrolidinecarboxamide hydrochloride

By reactions and purification similar to those in Example 95 using the compound (395 mg) obtained in Reference Example 68, a free base (318 mg) of the title compound was obtained.
¹H NMR (CDCl₃) δ 1.05-1.95 (9H, m), 2.05-2.35 (3H, m), 2.21 (3H, s), 2.45-3.25 (6H, m), 2.51 (2H, d, J=6.6Hz), 2.75 (0.5×3H, s), 2.76 (0.5×3H, s), 3.4-3.8 (1H, m) , 4.0-4.25 (1H, m) , 7.0-7.35 (9H, m).

The free base (318 mg) was converted to the title compound (283 mg) by a method similar to the method of Example 95.
Anal. Calcd for C₂₈H₃₇N₃O₂·HCl·0.7H₂O: C, 67.71; H, 8.00; Cl, 7.14; N, 8.46. Found: C, 67.68; H, 7.97; Cl, 7.36; N, 8.50.

### Example 98

### N-[3-(4-benzyl-1-piperidinyl)propyl]-N-(4-cyanophenyl)-1-methyl-5-oxo-3-pyrrolidinecarboxamide

By reactions and purification similar to those in Example 31 using the compound obtained in Reference Example 69, the title compound was obtained.
IR (KBr) 2230 cm⁻¹.
¹H NMR (CDCl₃) δ 1.21-1.99 (9H, m), 2.03-2.54 (6H, m), 2.78 (3H, s), 2.58-3.15 (4H, m), 3.58-3.78 (3H, m), 7.10-7.36 (7H, m), 7.77 (2H, d, J=8.0Hz).

### Example 99

### N- [3- (4-benzyl-1-piperidinyl)propyl]-N-(3-cyanophenyl) -1-methyl-5-oxo-3-pyrrolidinecarboxamide

By reactions and purification similar to those in Example 31 using the compound obtained in Reference Example 70, the title compound was obtained.
IR (KBr) 2232 cm⁻¹.
¹H NMR (CDCl₃) δ 1.16-2.00 (9H, m), 2.10-2.59 (5H, m), 2.78 (3H, s), 2.59-3.09 (3H, m), 3.09-3.40 (2H, m), 3.54-3.81 (3H, m), 7.09-7.32 (5H, m), 7.41-7.70 (4H, m).

### Example 100

### N-[3-(2-benzyl-4-morpholinyl)propyl]-1-methyl-5-oxo-N-phenyl-3-pyrrolidinecarboxamide

By reactions and purification similar to those in Example 31 using the compound obtained in Reference Example 71, the title compound was obtained.
¹H NMR (CDCl₃) δ 2.78 and 2.81 (3H, s×2), 2.19-3.15 (14H, m), 3.28-3.90 (6H, m), 7.10-7.32 (10H, m).

### Reference Example 1

### 1-methyl-5-oxo-N-phenyl-3-pyrrolidinecarboxamide

To a solution of 1-methyl-5-oxo-3-pyrrolidinecarboxylic acid (8.59 g, 60 mmol), aniline (5.59 g, 60 mmol) and 1-hydroxybenzotriazole (8.92 g, 66 mmol) in DMF (60 ml) was added *N*-ethyl-*N*'-(3-dimethylaminopropyl)carbodiimide hydrochloride (17.25 g, 90 mmol) and the mixture was stirred at room temperature for 4 h. The reaction mixture was concentrated under reduced pressure and saturated aqueous sodium hydrogencarbonate solution (120 ml) was added to the residue. The mixture was extracted with dichloromethane (120 ml×5). The organic layer was dried over anhydrous magnesium sulfate and concentrated under reduced pressure. The residue was subjected to column chromatography (silica gel 170 g, ethyl acetate/methanol=1/0→9/1). The objective fraction was concentrated under reduced pressure and diethyl ether was added to the residue. The precipitate was collected by filtration. The precipitate was washed with diethyl ether and dried under reduced pressure to give the title compound (11.04 g, 51 mmol, 84%) as white crystals.
mp 163-165°C
¹H NMR (CDCl₃) δ 2.67 (1H, dd, J=9.9, 17.1Hz), 2.81 (1H, dd, J=8.4, 17.1Hz), 2.88 (3H, s), 3.15-3.31 (1H, m), 3.58 (1H, dd, J=9.6, 9.6Hz), 3.77 (1H, dd, J=7.0, 9.6Hz), 7.14 (1H, t, J=7.3Hz), 7.34 (2H, dd, J=7.3, 8.0Hz), 7.53 (2H, d, J=8.0Hz), 7.60 (1H, br s).
Anal. Calcd for C₁₂H₁₄N₂O₂: C, 66.04; H, 6.47; N, 12.84. Found: C, 66.00; H, 6.44; N, 12.89.

### Reference Example 2

### N-(3,4-dichlorophenyl)-1-methyl-5-oxo-3-pyrrolidinecarboxamide

By reactions and purification similar to those in Reference Example 1 using 3,4-dichloroaniline, the title compound was obtained, yield 58%.
mp 164-166°C
¹H NMR (CDCl₃) δ 2.67 (1H, dd, J=10.0, 17.0Hz), 2.78 (1H, dd, J=7.8, 17.0Hz), 2.89 (3H, s), 3.16-3.33 (1H, m), 3.59 (1H, dd, J=9.6, 9.6Hz), 3.78 (1H, dd, J=6.6, 9.6Hz), 7.38 (1H, s), 7.39 (1H, s), 7.80 (1H, s), 7.97 (1H, br s).
Anal. Calcd for C₁₂H₁₂Cl₂N₂O₂: C, 50.19; H, 4.21; Cl, 24.69; N, 9.76. Found: C, 50.22; H, 4.26; Cl, 24.54; N, 9.94.

### Reference Example 3

### N-(3-chloropropyl)-1-methyl-5-oxo-N-phenyl-3-pyrrolidinecarboxamide

The compound (2.00g, 9.2 mmol) obtained in Reference Example 1 was dissolved in DMF (20 ml) and sodium hydride (60%, 733 mg, 18 mmol) was added under ice-cooling. The mixture was stirred at the same temperature for 1 h. Then, 1-bromo-3-chloropropane (1.81 ml, 18 mmol) was added and the mixture was stirred for 30 min under ice-cooling and for 1 h while allowing the mixture to warm to room temperature. Water (100 ml) was added under ice-cooling, and the mixture was extracted with ethyl acetate (15 ml×3). The organic layer was dried over anhydrous magnesium sulfate and concentrated under reduced pressure. The residue was subjected to column chromatography (silica gel 60 g, ethyl acetate/methanol=1/0→9/1). The objective fraction was concentrated under reduced pressure to give the title compound (2.43g, purity about 80% from ¹H NMR) as a colorless oil.
¹H NMR (CDCl₃) δ 1.95-2.15 (2H, m), 2.24 (1H, dd, J=9.3, 17.0Hz), 2.68 (1H, dd, J=8.5, 17.0Hz), 2.77 (3H, s), 2.95-3.25 (1H, m), 3.19 (1H, t, J=8.8Hz), 3.56 (2H, t, J=6.6Hz), 3.65 (1H, dd, J=7.0, 8.8Hz), 3.8-3.9 (2H, m), 7.1-7.25 (2H, m), 7.35-7.55 (3H, m).

### Reference Example 4

### N-(4-chlorobutyl)-1-methyl-5-oxo-N-phenyl-3-pyrrolidinecarboxamide

By reactions and purification similar to those in Reference Example 3 using 1-bromo-4-chlorobutane, the title compound was obtained.
¹H NMR (CDCl₃) δ 1.58-1.89 (4H, m), 2.23 (1H, dd, J=9.3, 16.7Hz), 2.60-2.80 (4H, m), 2.97-3.25 (2H, m), 3.50-3.81 (5H, m), 7.11-7.20 (2H, m), 7.36-7.53 (3H, m).

### Reference Example 5

### N-(5-chloropentyl)-1-methyl-5-oxo-N-phenyl-3-pyrrolidinecarboxamide

By reactions and purification similar to those in Reference Example 3 using 1-bromo-5-chloropentane, the title compound was obtained.
¹H NMR (CDCl₃) δ 1.35-1.87 (6H, m), 2.23 (1H, dd, J=9.3, 16.3Hz), 2.60-2.80 (4H, m), 2.95-3.24 (2H, m), 3.52 (2H, t, J=6.4Hz), 3.59-3.77 (3H, m), 7.10-7.20 (2H, m), 7.38-7.53 (3H, m).

### Reference Example 6-1

### 2-{[(1-methyl-5-oxo-3-pyrrolidinyl)carbonyl]anilino}ethyl acetate

The compound (2.00 g, 9.2 mmol) obtained in Reference Example 1 was dissolved in DMF (20 ml) and sodium hydride (60%, 916 mg, 23 mmol) was added under ice-cooling. The mixture was stirred at the same temperature for 1 h. Then bromoethyl acetate (3.05 ml, 28 mmol) was added and the mixture was stirred for 30 min under ice-cooling and at room temperature for 6 h. The reaction mixture was poured into 0.5N hydrochloric acid (100 ml) under ice-cooling and the mixture was extracted with ethyl acetate (50 ml×3). The organic layer was dried over anhydrous magnesium sulfate and concentrated under reduced pressure. The residue was subjected to column chromatography (silica gel 70 g, ethyl acetate/methanol=1/0→95/5). The objective fraction was concentrated under reduced pressure to give the title compound (2.43 g, 8.0 mmol, 87%), mp 72-74°C.
¹H NMR (CDCl₃) δ 1.28 (3H, t, J=7.2Hz), 2.28 (1H, dd, J=9.4, 16.4Hz), 2.75 (1H, dd, J=7.8, 16.4Hz), 2.78 (3H, s), 3.1-3.35 (2H, m), 3.6-3.8 (1H, m), 4.22 (2H, q, J=7.2Hz), 4.26 (1H, d, J=17.1Hz), 4.45 (1H, d, J=17.1Hz), 7.3-7.55 (5H, m).

### Reference Example 6-2

### 2-{[(1-methyl-5-oxo-3-pyrrolidinyl)carbonyl]anilino}acetic acid

The compound (1.83 g, 6.0 mmol) obtained in Reference Example 6-1 was dissolved in methanol (20 ml) and 8N aqueous sodium hydroxide solution (1.5 ml) was added. The mixture was stirred at room temperature for 10 h. 1N Hydrochloric acid (13 ml) was added and the mixture was concentrated under reduced pressure. Ethyl acetate was added to the residue and the mixture was dried over anhydrous magnesium sulfate. An insoluble material was filtrated and the filtrate was concentrated under reduced pressure to give the title compound (1.54 g, 5.6 mmol, 93%).
¹H NMR (CDCl₃) δ 2.35 (1H, dd, J=9.0, 17.0Hz), 2.75-2.95 (1H, m) , 2.80 (3H, s), 3.1-3.35 (2H, m), 3.65-3.8 (1H, m), 4.31 (1H, d, J=17.4Hz), 4.45 (1H, d, J=17.4Hz), 7.3-7.55 (5H, m).

### Reference Example 6-3

### N-(2-hydroxyethyl)-1-methyl-5-oxo-N-phenyl-3-pyrrolidinecarboxamide

The compound (829 mg, 3.0 mmol) obtained in Reference Example 6-2 and triethylamine (0.627 ml, 4.5 mmol) were dissolved in THF (15 ml) and ethyl chloroformate (0.43 ml, 4.5 mmol) was added at -15°C. The mixture was stirred at from -15°C to -10°C for 30 min. Then, a solution of sodium borohydride (227 mg, 6.0 mmol) in water (1.5 ml) was added at -10°C, and the mixture was stirred at from -10°C to 0°C for 1 h. 1N Hydrochloric acid was added at 0°C and the organic solvent was evaporated under reduced pressure. The residue was extracted with dichloromethane. The organic layer was dried over anhydrous magnesium sulfate and concentrated under reduced pressure. The residue was subjected to column chromatography (silica gel 10 g, ethyl acetate/methanol=1/0→95/5). The objective fraction was concentrated under reduced pressure to give the title compound (662 mg, 2.5 mmol, 84%) as a colorless oil.
¹H NMR (CDCl₃) δ 2.27 (1H, dd, J=9.5, 16.9Hz), 2.71 (1H, dd, J=8.4, 16.9Hz), 2.78 (3H, s), 3.0-3.25 (1H, m), 3.22 (1H, t, J=8.9Hz), 3.66 (1H, dd, J=6.6, 8.9Hz), 3.7-4.1 (4H, m), 7.15-7.3 (2H, m), 7.3-7.55 (3H, m).

### Reference Example 6-4

### N-(2-chloroethyl)-N-phenyl-1-methyl-5-oxo-3-pyrrolidinecarboxamide

A mixture of the compound (659 mg, 2.5 mmol) obtained in Reference Example 6-3, triphenylphosphine (857 mg, 3.3 mmol) and carbon tetrachloride (10 ml) was stirred with reflux under heating for 1 h. An insoluble material was filtrated and the insoluble material was washed with ethyl acetate . The filtrate was concentrated under reduced pressure and the residue was subjected to column chromatography (silica gel 40 g, ethyl acetate/methanol=1/0→95/5). The objective fraction was concentrated under reduced pressure and diethyl ether was added to the residue. The precipitate was collected by filtration. The precipitate was washed with diethyl ether and dried under reduced pressure to give the title compound (366 mg, 1.3 mmol, 52%).
¹H NMR (CDCl₃) δ 2.25 (1H, dd, J=9.3, 16.9Hz), 2.70 (1H, dd, J=8.2, 16.9Hz), 2.78 (3H, s), 2.95-3.25 (1H, m), 3.21 (1H, t, J=8.9Hz), 3.55-3.75 (3H, m), 4.00 (1H, dt, J=13.9, 6.2Hz), 4.11 (1H, dt, J=13.9, 6.6Hz), 7.2-7.3 (2H, m), 7.35-7.55 (3H, m).

### Reference Example 7

### N-(3-chloropropyl)-N-(3,4-dichlorophenyl)-1-methyl-5-oxo-3-pyrrolidinecarboxamide

By reactions and purification similar to those in Reference Example 3 using the compound obtained in Reference Example 2, the title compound was obtained, purity about 50% from ¹H NMR.
¹H NMR (CDCl₃) δ 1.95-2.15 (2H, m), 2.28 (1H, dd, J=9.7, 17.1Hz), 2.6-2.8 (1H, m), 2.80 (3H, s), 2.95-3.2 (1H, m), 3.24 (1H, t, J=9.2Hz), 3.56 (2H, t, J=6.4Hz), 3.66 (1H, dd, J=7.0, 9.2Hz), 3.75-3.9 (2H, m), 7.05 (1H, dd, J=2.4, 8.6Hz), 7.31 (1H, d, J=2.4Hz), 7.57 (1H, d, J=8.6Hz).

### Reference Example 8-1

### N-[2-(1,3-dioxolan-2-yl)ethyl]-1-methyl-5-oxo-N-phenyl-3-pyrrolidinecarboxamide

The compound (2.40 g, 11 mmol) obtained in Reference Example 1 was dissolved in DMF (22 ml) and sodium hydride (60%, 880 mg, 22 mmol) was added under ice-cooling. The mixture was stirred at the same temperature for 1 h. Then, 2-(2-bromoethyl)-1,3-dioxolane (2.58 ml, 22 mmol) was added and the mixture was stirred at 80°C for 12 h. The reaction mixture was concentrated under reduced pressure and water (45 ml) was added. The mixture was extracted with dichloromethane (45 ml×3). The organic layer was dried over anhydrous magnesium sulfate and concentrated under reduced pressure. The residue was subjected to column chromatography (silica gel 70 g, ethyl acetate/methanol=1/0→9/1). The objective fraction was concentrated under reduced pressure and the residue was recrystallized from a mixed solvent of diisopropyl ether and ethyl acetate. The precipitate was collected by filtration, and the precipitate was washed with diisopropyl ether and dried under reduced pressure to give the title compound (2.47 g, 7.8 mmol, 70%) as pale-yellow crystals, mp 108-110°C.
¹H NMR (CDCl₃) δ 1.91 (2H, dt, J=4.4, 7.3Hz), 2.23 (1H, dd, J=9.1, 16.9Hz), 2.70 (1H, dd, J=8.0, 16.9Hz), 2.77 (3H, s), 2.95-3.15 (1H, m), 3.18 (1H, t, J=9.1Hz), 3.66 (1H, dd, J=6.9, 9.1Hz), 3.75-4.0 (6H, m), 4.93 (1H, t, J=4.4Hz), 7.15-7.25 (2H, m), 7.35-7.55 (3H, m).

### Reference Example 8-2

### N-[2-formylethyl]-1-methyl-5-oxo-N-phenyl-3-pyrrolidinecarboxamide

The compound (1.95 g, 6.1 mmol) obtained in Reference Example 8-1 was dissolved in 1N hydrochloric acid (10 ml) and the mixture was stirred at room temperature for 18 h. The mixture was extracted with dichloromethane (20 ml×3) and the organic layer was dried over anhydrous magnesium sulfate and concentrated under reduced pressure to give the title compound (1.66 g, 6.1 mmol, 99%) as a pale-yellow oil.
¹H NMR (CDCl₃) δ 2.23 (1H, dd, J=9.4, 16.6Hz), 2.6-2.8 (3H, m), 2.77 (3H, s), 2.95-3.15 (1H, m), 3.18 (1H, t, J=9.1Hz), 3.61 (1H, dd, J=6.9, 9.1Hz), 3.98 (1H, dt, J=14.0, 6.6Hz), 4.14 (1H, dt, J=14.0, 6.9Hz), 7.1-7.25 (2H, m), 7.35-7.55 (3H, m), 9.77 (1H, t, J=1.9Hz).

### Reference Example 9

### N-[3-(4-benzyl-1-piperidyl)propyl]-4-methylaniline dihydrochloride

To a solution of 4-benzylpiperidine (3.51 g, 20 mmol) and DBU (0.030 ml, 0.2 mmol) in THF (40 ml) was added dropwise with stirring a solution of acrolein (90%, 1.49 ml, 20 mmol) in THF (5 ml) at -20°C over 5 min. The mixture was stirred for 1 h while raising the temperature of the mixture from -20°C to-10°C. Then, p-toluidine (2.14g, 20 mmol) and sodium triacetoxyborohydride (8.48 g, 40 mmol) were successively added at -10°C and the mixture was stirred for 23 h while raising the temperature of the mixture to room temperature. A saturated aqueous sodium hydrogencarbonate solution (160 ml) and water were added and the mixture was extracted with ethyl acetate (60 ml×3). The organic layer was dried over anhydrous magnesium sulfate and concentrated under reduced pressure. The residue was subjected to column chromatography (silica gel 100g, ethyl acetate/methanol=1/0→9/1→4/1). The objective fraction was concentrated under reduced pressure to give *N*-[3-(4-benzyl-1-piperidyl)propyl]-4-methylaniline (4.07 g, 12.6 mmol, 63%) as an oil.
¹H NMR (CDCl₃) δ 1.15-1.95 (9H, s), 2.23 (3H, s), 2.42 (2H, t, J=6.8Hz), 2.55 (2H, d, J=6.6Hz), 2.85-3.0 (2H, m), 3.13 (2H, t, J=6.4Hz), 6.51 (2H, d, J=8.4Hz), 6.98 (2H, d, J=8.4Hz), 7.1-7.35 (5H, m).

2-Propanol(20 ml) and 4N hydrogen chloride (ethyl acetate solution, 8 ml) were added to *N*-[3-(4-benzyl-1-piperidyl)propyl]-4-methylaniline (4.07 g, 12.6 mmol) and the precipitate was collected by filtration. The precipitate was washed with 2-propanol and dried under reduced pressure to give the title compound (4.52 g, 11 mmol, 57%) as white crystals.
mp 182-192°C (dec)
¹H NMR (DMSO-*d*₆) δ 1.4-1.9 (5H, m), 2.0-2.25 (2H, m), 2.31 (3H, s), 2.45-2.6 (2H, m), 2.7-2.95 (2H, m), 2.95-3.55 (6H, m), 7.1-7.45 (9H, m).
Anal. Calcd for C₂₂H₃₀N₂·2HCl·0.5H₂O: C, 65.34; H, 8.22; Cl, 17.53; N, 6.93. Found: C, 65.24; H, 8.38; Cl, 17.37; N, 6.98.

### Reference Example 10

### N-[3-(4-benzyl-1-piperidyl)propyl]aniline dihydrochloride

By reactions and purification similar to those in Reference Example 9 using aniline, the title compound was obtained, yield 47%.
mp 217°C (dec)
¹H NMR (D₂O) δ 1.44-1.56 (2H, m), 1.81-1.84 (3H, m), 2.08-2.24 (2H, m), 2.62 (2H, d, J=6.6Hz), 2.85-2.96 (2H, m), 3.12-3.20 (2H, m), 3.48-3.56 (4H, m), 7.25-7.65 (10H, m).
Anal. Calcd for C₂₁H₂₈N₂·2HCl·0.5H₂O: C, 64.61; H, 8.00; N, 7.18. Found: C, 64.71; H, 7.92; N, 7.32.

### Reference Example 11

### N-[3-(4-benzyl-1-piperidyl)propyl] -4-tert-butylaniline dihydrochloride

By reactions and purification similar to those in Reference Example 9 using 4-*tert*-butylaniline, the title compound was obtained, yield 51%.
mp 203-213°C (dec)
¹H NMR (DMSO-*d*₆) δ 1.27 (9H, s), 1.4-1.9 (5H, m), 2.0-2.2 (2H, m), 2.45-2.6 (2H, m), 2.75-2.95 (2H, m), 3.0-3.7 (6H, m), 7.1-7.4 (7H, m), 7.44 (2H, d, J=8.4Hz).
Anal. Calcd for C₂₅H₃₆N₂·2HCl·0.2H₂O: C, 68.07; H, 8.77; Cl, 16.07; N, 6.35. Found: C, 68.10; H, 8.80; Cl, 15.85; N, 6.35.

### Reference Example 12

### N-[3-(4-benzyl-1-piperidyl)propyl]-5-indanylamine dihydrochloride

By reactions and purification similar to those in Reference Example 9 using 5-aminoindan, the title compound was obtained, yield 28%.
mp 175°C (dec)
¹H NMR (D₂O) δ 1.42-1.50 (2H, m), 1.87-1.93 (3H, m), 2.08-2.15 (4H, m), 2.61 (2H, d, J=6.6Hz), 2.82-2.94 (6H, m), 3.10-3.18 (2H, m), 3.26-3.54 (4H, m), 7.12 (1H, d, J=7.8Hz), 7.24-7.41 (7H, m) .
Anal. Calcd for C₂₄H₃₂N₂·2HCl·0.25H₂O: C, 67.67; H, 8.25; N, 6.57. Found: C, 67.73; H, 7.97; N, 6.50.

### Reference Example 13

### N-[3-(4-benzyl-1-piperidyl)propyl]-4-methoxyaniline dihydrochloride

By reactions and purification similar to those in Reference Example 9 using 4-methoxyaniline, the title compound was obtained, yield 38%.
mp 154-159°C (dec)
¹H NMR (DMSO-*d*₆) δ 1.4-1.95 (5H, m), 1.95-2.2 (2H, m), 2.45-2.65 (2H, m), 2.7-3.0 (2H, m), 3.0-3.55 (6H, m), 3.76 (3H, s), 7.02 (2H, d, J=8.8Hz), 7.1-7.45 (7H, m).
Anal. Calcd for C₂₂H₃₀N₂O·2HCl·0.4H₂O: C, 63.12; H, 7.90; Cl, 16.94; N, 6.69. Found: C, 63.12; H, 7.84; Cl, 16.71; N, 6.78.

### Reference Example 14

### N-[3-(4-benzyl-1-piperidyl)propyl]-3,4-dimethoxyaniline dihydrochloride

By reactions and purification similar to those in Reference Example 9 using 3,4-dimethoxyaniline, the title compound was obtained, yield 61%.
mp 149-159°C (dec)
¹H NMR (DMSO-*d*₆) δ 1.4-1.9 (5H, m), 2.0-2.25 (2H, m), 2.45-2.6 (2H, m), 2.75-3.0 (2H, m), 3.0-3.65 (6H, m), 3.77 (3H, s), 3.79 (3H, s), 7.03 (2H, s), 7.05-7.4 (6H, m).
Anal. Calcd for C₂₃H₃₂N₂O₂·2HCl·1.0H₂O: C, 60.13; H, 7.90; Cl, 15.43; N, 6.10. Found: C, 60.13; H, 7.72; Cl, 15.26; N, 6.06.

### Reference Example 15

### N-[3-(4-benzyl-1-piperidyl)propyl]-3,4-diethoxyaniline dihydrochloride

By reactions and purification similar to those in Reference Example 9 using 3,4-diethoxyaniline, the title compound was obtained, yield 24%.
mp 160°C (dec)
¹H NMR (D₂O) δ 1.38-1.51 (8H, m), 1.89-1.96 (3H, m), 2.10-2.19 (2H, m), 2.63 (2H, d, J=6.6Hz), 2.86-2.94 (2H, m), 3.12-3.20 (2H, m), 3.45-3.55 (4H, m), 4.13-4.23 (4H, m), 7.02-7.39 (8H, m) .
Anal. Calcd for C₂₅H₃₆N₂O₂·2HCl·0.6H₂O: C, 62.51; H, 8.23; N, 5.83. Found: C, 62.30; H, 8.10; N, 5.84.

### Reference Example 16

### N-[3-(4-benzyl-1-piperidyl)propyl] -4-chloroaniline dihydrochloride

By reactions and purification similar to those in Reference Example 9 using 4-chloroaniline, the title compound was obtained, yield 70%.
mp 155-159°C (dec)
¹H NMR (DMSO-*d*₆) δ 1.4-1.9 (5H, m), 1.9-2.1 (2H, m), 2.45-2.6 (2H, m), 2.7-2.95 (2H, m), 2.95-3.5 (6H, m), 6.85 (2H, d, J=9.2Hz), 7.1-7.4 (7H, m).
Anal. Calcd for C₂₁H₂₇ClN₂·2HCl: C, 60.66; H, 7.03; Cl, 25.58; N, 6.74. Found: C, 60.85; H, 6.81; Cl, 25.33; N, 6.79.

### Reference Example 17

### N-[3-(4-benzyl-1-piperidyl)propyl]-3-chloroaniline dihydrochloride

By reactions and purification similar to those in Reference Example 9 using 3-chloroaniline, the title compound was obtained, yield 41%.
mp 202°C (dec)
¹H NMR (DMSO-*d*₆) δ 1.53-2.01 (7H, m), 2.50-2.55 (2H, m), 2.66-2.92 (2H, m), 3.08-3.20 (4H, m), 3.38-3.44 (2H, m), 6.61-6.69 (3H, m), 7.07-7.30 (6H, m).
Anal. Calcd for C₂₁H₂₇ClN₂·2HCl·0.1H₂O: C, 60.39; H, 7.04; N, 6.71. Found: C, 60.33; H, 6.93; N, 6.84.

### Reference Example 18

### N-[3-(4-benzyl-1-piperidyl)propyl] -3,4-dichloroaniline dihydrochloride

By reactions and purification similar to those in Reference Example 9 using 3,4-dichloroaniline, the title compound was obtained, yield 53%.
mp 203°C (dec)
¹H NMR (DMSO-*d*₆) δ 1.49-1.76 (5H, m), 1.91-1.96 (2H, m), 2.50-2.55 (2H, m), 2.79-3.17 (6H, m), 3.38-3.44 (2H, m), 6.68 (1H, dd, J=2.8, 8.8Hz), 6.75 (1H, d, J=2.6Hz), 7.17-7.30 (6H, m).
Anal. Calcd for C₂₁H₂₆Cl₂N₂·2HCl·0.5H₂O: C, 54.92; H, 6.36; N, 6.10. Found: C, 55.11; H, 6.64; N, 6.37.

### Reference Example 19

### N-[3-(4-benzyl-1-piperidyl)propyl]-3,4-difluoroaniline dihydrochloride

By reactions and purification similar to those in Reference Example 9 using 3,4-difluoroaniline, the title compound was obtained, yield 53%.
mp 177°C (dec)
¹H NMR (DMSO-*d*₆) δ 1.53-1.75 (5H, m), 1.94-1.98 (2H, m), 2.51-2.54 (2H, m), 2.66-2.84 (2H, m), 3.06-3.10 (4H, m), 3.38-3.44 (2H, m), 6.51-6.55 (1H, m), 6.67-6.77 (1H, m), 7.11-7.34 (6H, m) .
Anal. Calcd for C₂₁H₂₆F₂N₂·2HCl: C, 60.43; H, 6.76; N, 6.71. Found: C, 59.93; H, 6.67; N, 6.74.

### Reference Example 20

### N-[3-(4-benzyl-1-piperidyl)propyl]-2,4-difluoroaniline dihydrochloride

By reactions and purification similar to those in Reference Example 9 using 2,4-difluoroaniline, the title compound was obtained, yield 43%.
mp 181°C (dec)
¹H NMR (DMSO-*d*₆) δ 1.53-1.75 (5H, m), 1.95-2.02 (2H, m), 2.50-2.54 (2H, m), 2.66-2.84 (2H, m), 3.05-3.18 (4H, m), 3.37-3.43 (2H, m), 6.72-6.94 (2H, m), 7.04-7.34 (6H, m).
Anal. Calcd for C₂₁H₂₆F₂N₂·2HCl·1.0H₂O: C, 57.93; H, 6.95; N, 6.43. Found: C, 57.46; H, 7.04; N, 6.14.

### Reference Example 21

### N-[3-(4-benzyl-1-piperidyl)propyl]-2,6-difluoroaniline dihydrochloride

By reactions and purification similar to those in Reference Example 9 using 2,6-difluoroaniline, the title compound was obtained, yield 15%.
mp 168°C (dec)
¹H NMR (D₂O) δ 1.41-1.50 (2H, m), 1.83-2.08 (5H, m), 2.61 (2H, d, J=6.4Hz), 2.82-2.94 (2H, m), 3.12-3.55 (6H, m), 7.06-7.42 (8H, m).
Anal. Calcd for C₂₁H₂₆F₂N₂·2HCl: C, 60.43; H, 6.66; N, 6.71. Found: C, 60.27; H, 6.66; N, 6.64.

### Reference Example 22

### N-[3-(4-benzyl-1-piperidyl)propyl]-3-chloro-4-fluoroaniline dihydrochloride

By reactions and purification similar to those in Reference Example 9 using 3-chloro-4-fluoroaniline, the title compound was obtained, yield 40%.
mp 197°C (dec)
¹H NMR (DMSO-*d*₆) δ 1.53-1.75 (5H, m), 1.94-2.02 (2H, m), 2.50-2.55 (2H, m), 2.80-2.85 (2H, m), 3.07-3.10 (4H, m), 3.38-3.45 (2H, m), 6.67-6.73 (1H, m), 6.84 (1H, dd, J=3.0, 6.0Hz), 7.13-7.34 (6H, m).
Anal. Calcd for C₂₁H₂₆ClFN₂·2HCl·0.5H₂O: C, 56.96; H, 6.60; N, 6.33. Found: C, 57.12; H, 6.43; N, 6.46.

### Reference Example 23

### N-[3-(4-benzyl-1-piperidyl)propyl]-4-(trifluoromethyl)aniline dihydrochloride

By reactions and purification similar to those in Reference Example 9 using 4-(trifluoromethyl)aniline, the title compound was obtained, yield 36%.
mp 168°C (dec)
¹H NMR (DMSO-*d*₆) δ 1.56-1.75 (5H, m), 1.95-2.06 (2H, m), 2.50-2.55 (2H, m), 2.80-2.90 (2H, m), 3.04-3.18 (4H, m), 3.38-3.45 (2H, m), 6.70 (2H, d, J=8.6Hz), 7.16-7.40 (7H, m).
Anal. Calcd for C₂₂H₂₇F₃N₂·2HCl: C, 58.80; H, 6.50; N, 6.23. Found: C, 58.64; H, 6.47; N, 6.32.

### Reference Example 24

### N-[3-(4-benzyl-1-piperidyl)propyl]-3,5-bis(trifluoromethyl)aniline dihydrochloride

By reactions and purification similar to those in Reference Example 9 using 3,5-bis(trifluoromethyl)aniline, the title compound was obtained, yield 19%.
mp 185°C (dec)
¹H NMR (DMSO-*d*₆) δ 1.50-1.76 (5H, m), 1.91-1.97 (2H, m), 2.50-2.55 (2H, m), 2.80-2.86 (2H, m), 3.08-3.24 (4H, m), 3.40-3.47 (2H, m), 7.05-7.34 (8H, m).
Anal. Calcd for C₂₃H₂₆F₆N₂·2HCl·1.0H₂O: C, 51.60; H, 5.65; N, 5.23. Found: C, 51.69; H, 5.54; N, 5.43.

### Reference Example 25

### N-[3-(4-benzyl-1-piperidyl)propyl]-4-(trifluoromethoxy)aniline dihydrochloride

By reactions and purification similar to those in Reference Example 9 using 4-(trifluoromethoxy)aniline, the title compound was obtained, yield 35%.
mp 175°C (dec)
¹H NMR (DMSO-*d*₆) δ 1.54-1.75 (5H, m), 1.98-2.06 (2H, m), 2.50-2.55 (2H, m), 2.80-2.90 (2H, m), 3.12-3.19 (4H, m), 3.39-3.45 (2H, m), 6.68 (2H, d, J=8.8Hz), 7.16-7.34 (7H, m).
Anal. Calcd for C₂₂H₂₇F₃N₂O·2HCl·1.1H₂O: C, 54.45; H, 6.48; N, 5.77. Found: C, 54.26; H, 6.17; N, 5.97.

### Reference Example 26

### N-[3-(4-benzyl-1-piperidyl)propyl]-1-naphthylamine dihydrochloride

By reactions and purification similar to those in Reference Example 9 using 1-aminonaphthalene, the title compound was obtained, yield 48%.
mp 175°C (dec)
¹H NMR (DMSO-*d*₆) δ 1.55-1.75 (5H, m), 2.10-2.20 (2H, m), 2.50-2.55 (2H, m), 2.80-2.90 (2H, m), 3.10-3.18 (2H, m), 3.33-3.45 (4H, m), 6.82-6.86 (1H, m), 7.16-7.37 (7H, m), 7.46-7.50 (2H, m), 7.81-7.86 (1H, m), 8.21-8.26 (1H, m).
Anal. Calcd for C₂₅H₃₀N₂·2HCl·1.0H₂O: C, 66.81; H, 7.62; N, 6.23. Found: C, 66.60; H, 7.53; N, 6.25.

### Reference Example 27

### N-[3-(4-benzyl-1-piperidyl)propyl]-3-phenylaniline dihydrochloride

By reactions and purification similar to those in Reference Example 9 using 3-aminobiphenyl, the title compound was obtained, yield 55%.
mp 164-169°C (dec)
¹H NMR (DMSO-*d*₆) δ 1.4-1.9 (5H, m), 1.9-2.2 (2H, m) , 2.45-2.6 (2H, m), 2.7-3.0 (2H, m), 3.0-3.55 (6H, m), 6.95-7.1 (1H, m), 7.1-7.55 (11H, m), 7.64 (2H, d, J=7.0Hz).
Anal. Calcd for C₂₇H₃₂N₂·2HCl·0.9H₂O: C, 68.46; H, 7.62; Cl, 14.97; N, 5.91. Found: C, 68.55; H, 7.62; Cl, 14.87; N, 5.96.

### Reference Example 28

### 3-(benzyloxy)-N-[3-(4-benzyl-1-piperidyl)propyl]aniline dihydrochloride

By reactions and purification similar to those in Reference Example 9 using 3-(benzyloxy)aniline, the title compound was obtained, yield 58%.
mp 134-139°C (dec)
¹H NMR (DMSO-*d*₆) δ 1.4-1.9 (5H, m), 1.9-2.15 (2H, m), 2.45-2.6 (2H, m), 2.7-2.95 (2H, m), 2.95-3.5 (6H, m), 5.08 (2H, s), 6.6-6.85 (3H, m), 7.1-7.5 (11H, m).
Anal. Calcd for C₂₆H₃₄N₂O·2HCl: C, 68.98; H, 7.44; Cl, 14.54; N, 5.75. Found: C, 68.90; H, 7.37; Cl, 14.23; N, 5.74.

### Reference Example 29

### 4-(benzyloxy)-N-[3-(4-benzyl-1-piperidyl)propyl]aniline dihydrochloride

By reactions and purification similar to those in Reference Example 9 using 4-(benzyloxy)aniline, the title compound was obtained, yield 72%.
mp 160-170°C (dec)
¹H NMR (DMSO-*d*₆) δ 1.4-1.95 (5H, m), 2.0-2.25 (2H, m), 2.45-2.6 (2H, m), 2.7-2.95 (2H, m), 2.95-3.5 (6H, m), 5.12 (2H, s), 7.05-7.5 (14H, m).
Anal. Calcd for C₂₈H₃₄N₂O·2HCl: C, 68.98; H, 7.44; Cl, 14.54; N, 5.75. Found: C, 68.73; H, 7.41; Cl, 14.24; N, 5.64.

### Reference Example 30

### 3-(4-benzyl-1-piperidinyl)propylamine

To a solution of 4-benzylpiperidine (24.6 g, 140 mmol) in *N,N*'-dimethylformamide (250 mL) were added N-(3-bromopropyl)phthalimide (37.5 g, 140 mmol) and then potassium carbonate (38.7 g, 280 mmol) and the mixture was stirred at room temperature for 14 h. Water (200 mL) was added to the reaction mixture and the mixture was extracted with ethyl acetate (300 mL×2). The organic layer was washed with water (400 mL) and saturated sodium chloride solution (400 mL), dried over anhydrous magnesium sulfate, filtered (eluted with ethyl acetate) through silica gel (100 g) and concentrated under reduced pressure. The obtained crude crystals were recrystallized from ethyl acetate-hexane to give 2-[3-(4-benzyl-1-piperidinyl)propyl]-1*H*-isoindole-1,3(2*H*)-dione (27.4 g, yield 69%). To a solution of this compound (500 mg, 1.38 mmol) in ethanol (5 mL) was added hydrazine monohydrate (345 mg, 6.9 mmol) and the mixture was refluxed under heating at 90°C for 2 h. After cooling, an insoluble material was filtrated and the mother liquor was concentrated under reduced pressure. A 2N aqueous sodium hydroxide solution (10 mL) was added to the residue and the mixture was extracted with a mixed solvent of ethyl acetate/tetrahydrofuran = 1/1 (20 mL×3). The organic layer was dried over anhydrous sodium sulfate and concentrated under reduced pressure. The residue was crystallized from acetonitrile to give the title compound (329 mg, yield 95%).
mp 59-61°C
¹H NMR (CDCl₃+D₂O) δ 1.20-1.38 (2H, m), 1.40-1.70 (5H, m) , 1.71-1.89 (2H, m), 2.26-2.43 (2H, m), 2.53 (2H, d, J = 6.6 Hz), 2.72 (2H, t, J = 7.0 Hz), 2.90-3.00 (2H, m), 7.10-7.30 (5H, m).

### Reference Example 31

### 1-(3-aminopropyl)-4-(4-chlorophenyl)-4-piperidinol

By reactions and purification similar to those in Reference Example 30 using 4-(4-chlorophenyl)-4-hydroxypiperidine, the title compound was obtained, yield 67%.
mp 102-104°C
¹H NMR (CDCl₃) δ 1.60-1.80 (5H, m), 2.00-2.20 (2H, m), 2.30-2.50 (4H, m), 2.72 (2H, t, J = 7.0 Hz), 2.75-2.90 (2H, m), 4.80 (2H, br), 7.20-7.50 (4H, m).

### Reference Example 32

### N-benzyl-3-(4-benzyl-1-piperidinyl)-1-propaneamine

To a solution of the compound (500 mg, 2.15 mmol) obtained in Reference Example 30 in tetrahydrofuran (3 mL) was added dropwise a solution of benzaldehyde (323 mg, 2.20 mmol) in tetrahydrofuran (2 mL) at 0°C and the mixture was stirred at room temperature for 1 h. To this solution was added dropwise a solution of acetic acid (168 mg, 2.80 mmol) in tetrahydrofuran (5 mL) at 0°C, and sodium triacetoxyborohydride (593 mg, 2.80 mmol) was added. The mixture was stirred at room temperature for 14 h. The reaction mixture was concentrated under reduced pressure and a mixed solvent of ethyl acetate/tetrahydrofuran = 1/1 (10 mL) was added. An insoluble material was filtrated and the mother liquor was concentrated. The obtained oil was purified by column chromatography (basic alumina activity III, 50 g, eluted with ethyl acetate - ethyl acetate/methanol = 4/1) to give the title compound (340 mg, 49%, oil).
¹H NMR (CDCl₃) δ 1.10-1.88 (10H, m), 2.35 (2H, t, J = 7.5 Hz), 2.52 (2H, d, J = 6.6 Hz), 2.66 (2H, t, J = 6.8 Hz), 2.88-3.00 (2H, m), 3.78 (2H, s), 7.11-7.36 (10H, m).

### Reference Example 33

### 4- ({[3- (4-benzyl-1-piperidinyl)propyl] amino }methyl) phenol

By reactions and purification similar to those in Reference Example 32 using 4-hydroxybenzaldehyde, the title compound was obtained, yield 59% (oil).
¹H NMR (CDCl₃) δ 1.20-2.00 (9H, m) , 2.40 (2H, t like, J = 7.0 Hz), 2.50 (2H, d, J = 6.2 Hz), 2.68 (2H, t like, J = 7.0 Hz), 2.88-3.00 (2H, m), 3.65 (2H, s), 3.80-4.66 (2H, br), 6.57 (2H, d, J = 8.4 Hz), 7.03 (2H, d, J = 8.4 Hz), 7.10-7.31 (5H, m).

### Reference Example 34

### 3-(4-benzyl-1-piperidinyl)-N-(1-naphthylmethyl)-1-propaneamine

By reactions and purification similar to those in Reference Example 32 using 1-naphthoaldehyde, the title compound was obtained, yield 57% (oil).
¹H NMR (CDCl₃) δ 1.05-1.35 (2H, m) , 1.37-1.93 (7H, m) , 2.22 (1H, br s), 2.37 (2H, t, J = 7.3 Hz), 2.47 (2H, d, J = 6.8 Hz), 2.79 (2H, t, J = 6.8 Hz), 2.85-2.95 (2H, m), 4.24 (2H, s), 7.10-7.32 (4H, m), 7.39-7.57 (4H, m), 7.76-7.90 (2H, m), 8.09-8.13 (2H, m).

### Reference Example 35

### 3-(4-benzyl-1-piperidinyl)-N-(2-naphthylmethyl)-1-propaneamine

By reactions and purification similar to those in Reference Example 32 using 2-naphthaldehyde, the title compound was obtained, yield 43% (oil).
¹H NMR (CDCl₃) δ 1.15-1.35 (2H, m), 1.40-1.93 (8H, m), 2.36 (2H, t, J = 7.4 Hz), 2.49 (2H, d, J = 6.6 Hz), 2.70 (2H, t, J = 7.0 Hz), 2.80-3.00 (2H, m), 3.95 (2H, s), 7.09-7.32 (5H, m), 7.40-7.51 (3H, m), 7.76-7.84 (4H, m) .

### Reference Example 36

### 1-[3-(benzylamino)propyl]-4-(4-chlorophenyl)-4-piperidinol

By reactions and purification similar to those in Reference Example 32 using the compound obtained in Reference Example 31, the title compound was obtained, yield 48% (oil).
¹H NMR (CDCl₃) δ 1.60-1.90 (6H, m), 2.06 (2H, td, J = 13.4, 4.4 Hz), 2.33-2.52 (4H, m), 2.73 (2H, t, J = 6.8 Hz), 2.80-2.86 (2H, m), 3.80 (2H, m), 7.20-7.50 (9H, m).

### Reference Example 37

### 4-(4-chlorophenyl)-1-[3-(isopropylamino)propyl]-4-piperidinol

By reactions and purification similar to those in Reference Example 32 using the compound obtained in Reference Example 31 and acetone, the title compound was obtained, yield 45%.
¹H NMR (DMSO-*d*₆) δ 1.24 (6H, d, J = 6.6 Hz), 1.50-1.70 (2H, m), 1.70-2.00 (4H, m) , 2.40-2.60 (5H, m), 2.70-2.90 (2H, m), 2.95 (2H, t, J = 7.3 Hz), 3.20-3.40 (2H, m), 7.37 (2H, d, J = 8.7 Hz), 7.49 (2H, d, J = 8.7 Hz).

### Reference Example 38

### 4-(4-chlorophenyl)-1-[3-(cyclohexylamino)propyl]-4-piperidinol

By reactions and purification similar to those in Reference Example 32 using the compound obtained in Reference Example 31 and cyclohexanone, the title compound was obtained, yield 58%.
¹H NMR (CDCl₃) δ 1.10-1.40 (6H, m), 1.50-1.96 (10H, m), 2.08 (2H, td, J = 11.6, 4.4 Hz), 2.38-2.60 (4H, m), 2.77-2.92 (4H, m), 2.80-3.40 (1H, br), 7.31 (2H, d, J = 8.8 Hz), 7.44 (2H, d, J = 8.8 Hz).

### Reference Example 39

### 4-(4-chlorophenyl)-1-[3-(cyclopentylamino)propyl]-4-piperidinol

By reactions and purification similar to those in Reference Example 32 using the compound obtained in Reference Example 31 and cyclopentanone, the title compound was obtained, yield 57%.
¹H NMR (DMSO-*d*₆) δ 1.40-2.20 (13H, m), 2.30-2.60 (2H, m), 3.00-3.60 (8H, m), 5.62 (1H, s), 7.43 (2H, d, J = 9.2 Hz), 7.50 (2H, d, J = 9.2 Hz), 9.06 (1H, br s).

### Reference Example 40

### 4-benzyl-1-(3-chloropropyl)piperidine

To a solution of 4-benzylpiperidine (100 mg, 0.57 mmol) in N,N'-dimethylformamide (2 mL) were added 1-chloro-3-iodopropane (117 mg, 0.57 mmol) and then triethylamine (58 mg, 0.57 mmol) and the mixture was stirred at room temperature for 14 h. Water (10 mL) was added to the reaction mixture and the mixture was extracted with ethyl acetate (20 mL×2). The organic layer was washed with water (20 mL) and dried over anhydrous magnesium sulfate, filtrated and concentrated under reduced pressure. The obtained oil was purified by column chromatography (basic alumina activity III, 50 g, eluted with ethyl acetate/N-hexane = 1/20) to give the title compound (86 mg, 60%, oil).
¹H NMR (CDCl₃) δ 1.15-2.05 (9H, m), 2.43 (2H, t, J = 7.0Hz), 2.53 (2H, d, J = 6.6 Hz), 2.80-3.00 (2H, m), 3.58 (2H, t, J = 6.6Hz), 7.12-7.33 (5H, m).

### Reference Example 41

### N-[3-(4-benzyl-1-piperidinyl)propyl]-2-indanamine

To a solution of the compound (755 mg, 3 mmol) obtained in Reference Example 40 in acetonitrile (5 mL) were added a solution of 2-aminoindan (266 mg, 2 mmol) in acetonitrile (5 mL) and triethylamine (304 mg, 3 mmol) and the mixture was stirred with heating at 80°C for 5 h. The solvent was concentrated under reduced pressure and the residue was purified by column chromatography (basic alumina activity III, 60 g, eluted with ethyl acetate) to give the title compound (150 mg, 22%, oil).
¹H NMR (CDCl₃) δ 1.10-1.32 (2H, m), 1.38-1.88 (8H, m), 2.36 (2H, t, J = 7.3Hz), 2.51 (2H, d, J = 6.8 Hz), 2.67-3.00 (6H, m), 3.16 (2H, dd, J = 15.4, 7.0 Hz), 3.61 (1H, qui., J = 7.0 Hz), 7.12-7.32 (9H, m).

### Reference Example 42

### [1-(3-anilino-2-hydroxypropyl)-4-piperidinyl]-(4-fluorophenyl)methanone

(4-Fluorophenyl) (4-piperidinyl)methanone hydrochloride (1.05 g, 4.3 mmol) was added to a mixture of ethyl acetate (50 mL) and 1N aqueous sodium hydroxide solution (10 mL), and the mixture was extracted with ethyl acetate. The organic layer was washed with water (20 mL), dried over anhydrous magnesium sulfate and concentrated under reduced pressure. The residue was dissolved in acetonitrile (30 mL) and *N*-(2-oxiranylmethyl)aniline (700 mg, 4.7 mmol) was added. The mixture was refluxed under heating for 24 h. After cooling, the reaction mixture was concentrated under reduced pressure and the residue was purified by silica gel chromatography (silica gel 100 g, ethyl acetate/methanol = 9/1) to give the title compound (510 mg, 33%, oil).
¹H NMR (DMSO-*d*₆) δ 1.57-1.86 (4H, m), 2.11-2.52 (4H, m), 2.86-3.33 (5H, m), 3.78-3.81 (1H, m), 4.62-4.64 (1H, m), 5.64 (1H, br), 6.47-6.60 (3H, m), 7.02-7.09 (2H, m), 7.29-7.37 (2H, m) , 8.02-8.09 (2H, m).

### Reference Example 43

### 5-oxo-1-phenyl-3-pyrrolidinecarboxylic acid

Aniline (18 g, 190 mmol) was added to itaconic acid (25 g, 190 mmol) and the mixture was refluxed under heating at 150°C for 1 h. After cooling, the obtained crude crystals were recrystallized from methanol (200 mL) to give the title compound (35 g, 90%).
mp 188-189°C (methanol).
¹H NMR (CDCl₃) δ 2.60-2.86 (2H, m), 3.20-3.50 (1H, m), 3.92-4.10 (2H, m), 7.14 (1H, t, J = 7.6 Hz), 7.37 (2H, t, J = 7.6 Hz), 7.64 (2H, d, J = 7.6 Hz), 12.80 (1H, br s).
Anal. Calcd for C₁₁H₁₁NO₃: C, 64.38; H, 5.40; N, 6.83. Found: C, 64.34; H, 5.53; N, 6.91.

### Reference Example 44

### 1-benzyl-5-oxo-3-pyrrolidinecarboxylic acid

By reactions and purification similar to those in Reference Example 43 using benzylamine, the title compound was obtained, yield 76%.
mp 192-193°C (methanol).
¹H NMR (CDCl₃) δ 2.69-2.92 (2H, m), 3.14-3.30 (1H, m), 3.43-3.59 (2H, m), 4.39 (1H, d, J = 14.6 Hz), 4.53 (1H, d, J = 14.6 Hz), 7.19-7.38 (5H, m), 10.29 (1H, br s).
Anal. Calcd for C₁₂H₁₃NO₃: C, 65.74; H, 5.98; N, 6.39. Found: C, 65.80; H, 5.84; N, 6.48.

### Reference Example 45

### 1-cyclohexyl-5-oxo-3-pyrrolidinecarboxylic acid

By reactions and purification similar to those in Reference Example 43 using cyclohexylamine, the title compound was obtained, yield 62%.
mp 186-187°C (methanol-diethyl ether).
¹H NMR (CDCl₃) δ 1.00-1.77 (10H, m), 2.34-2.57 (2H, m), 3.08-3.23 (1H, m), 3.30-4.00 (4H, m).

### Reference Example 46

### 1-butyl-5-oxo-3-pyrrolidinecarboxylic acid

By reactions and purification similar to those in Reference Example 43 using N-butylamine, the title compound was obtained, yield 67% (oil).
¹H NMR (CDCl₃) δ 0.93 (3H, t, J = 7.0 Hz), 1.23-1.59 (4H, m), 2.64-2.88 (2H, m), 3.19-3.40 (3H, m), 3.56-3.74 (2H, m), 7.20-7.60 (1H, br).

### Reference Example 47

### 5-oxo-1-phenethyl-3-pyrrolidinecarboxylic acid

By reactions and purification similar to those in Reference Example 43 using phenethylamine, the title compound was obtained, yield 60%.
mp 185-186°C (methanol).
¹H NMR (CDCl₃) δ 2.54-2.88 (4H, m), 3.05-3.21 (1H, m) , 3.40-3.62 (4H, m), 7.19-7.40 (5H, m), 7.70-8.20 (1H, br).

### Reference Example 48

### 5-oxo-1-(3-phenylpropyl)-3-pyrrolidinecarboxylic acid

By reactions and purification similar to those in Reference Example 43 using 3-phenylpropylamine, the title compound was obtained, yield 51%.
mp 88-90°C (ethyl acetate ).
¹H NMR (CDCl₃) δ 1.78-1.93 (2H, m), 2.57-2.80 (4H, m), 3.09-3.69 (5H, m), 7.15-7.32 (5H, m), 8.34 (1H, br s).

### Reference Example 49

### 1-(4-methoxybenzyl)-5-oxo-3-pyrrolidinecarboxylic acid

By reactions and purification similar to those in Reference Example 43 using 4-methoxybenzylamine, the title compound was obtained, yield 83%.
mp 153-155°C (methanol).
¹H NMR (CDCl₃) δ 2.61-2.86 (2H, m), 3.08-3.24 (1H, m), 3.39-3.55 (2H, m), 3.80 (3H, s), 4.33 (1H, d, J = 14.2 Hz), 4.46 (1H, d, J = 14.2 Hz), 6.82-6.89 (2H, m), 7.13-7.20 (2H, m), 7.50-9.00 (1H, br).

### Reference Example 50

### 5-oxo-1-(4-pyridylmethyl)-3-pyrrolidinecarboxylic acid

By reactions and purification similar to those in Reference Example 43 using 4-(aminomethyl)pyridine, the title compound was obtained, yield 15%.
mp 190-191°C (water-methanol).
¹H NMR (DMSO-*d*₆) δ 2.25-2.71 (2H, m), 3.15-3.57 (3H, m), 4.36 (1H, d, J = 16.0 Hz), 4.47 (1H, d, J = 16.0 Hz), 7.23 (2H, d, J = 5.6 Hz), 8.53 (2H, d, J = 5.6 Hz).

### Reference Example 51

### 1-(4-fluorobenzyl)-5-oxo-3-pyrrolidinecarboxylic acid

By reactions and purification similar to those in Reference Example 43 using 4-fluorobenzylamine, the title compound was obtained, yield 72%.
mp 142-143°C (methanol).
¹H NMR (CDCl₃) δ 2.64-2.88 (2H, m), 3.11-3.27 (1H, m), 3.41-3.57 (2H, m), 4.43 (2H, s), 6.97-7.32 (4H, m), 9.40-10.40 (1H, br).

### Reference Example 52

### 1-(cyclohexylmethyl)-5-oxo-3-pyrrolidinecarboxylic acid

By reactions and purification similar to those in Reference Example 43 using (aminomethyl)cyclohexane, the title compound was obtained, yield 50%.
mp 96-97°C (methanol-diethyl ether).
¹H NMR (CDCl₃) δ 0.80-1.32 (5H, m), 1.50-1.80 (6H, m), 2.66-2.89 (2H, m), 3.04-3.35 (3H, m), 3.55-3.73 (2H, m), 6.40-7.20 (1H, br).

### Reference Example 53

### 1-(2-chlorobenzyl)-5-oxo-3-pyrrolidinecarboxylic acid

By reactions and purification similar to those in Reference Example 43 using 2-chlorobenzylamine, the title compound was obtained, yield 77%.
¹H NMR (CDCl₃+DMSO-d₆) δ 2.62-2.87 (2H, m) , 3.14-3.30 (1H, m) , 3.42-3.58 (2H, m), 4.60 (2H, s), 7.22-7.40 (4H, m).

### Reference Example 54

### 1-(3-chlorobenzyl)-5-oxo-3-pyrrolidinecarboxylic acid

By reactions and purification similar to those in Reference Example 43 using 3-chlorobenzylamine, the title compound was obtained, yield 69%.
¹H NMR (CDCl₃+DMSO-*d*₆) δ 2.60-2.90 (2H, m), 3.10-3.28 (1H, m), 3.45-3.60 (2H, m), 4.58 (2H, s), 7.20-7.45 (4H, m).

### Reference Example 55

### 1-(4-chlorobenzyl)-5-oxo-3-pyrrolidinecarboxylic acid

By reactions and purification similar to those in Reference Example 43 using 4-chlorobenzylamine, the title compound was obtained, yield 66%.
¹H NMR (CDCl₃+DMSO-*d*₆) δ 2.65-2.90 (2H, m), 3.10-3.30 (1H, m), 3.45-3.61 (2H, m), 4.53 (2H, s), 7.34 (2H, d, J = 7.5Hz), 7.58 (2H, d, J = 7.5Hz), 7.6-8.5 (1H, br).

### Reference Example 56

### 5-oxo-1-[4-(trifluoromethyl)benzyl]-3-pyrrolidinecarboxylic acid

By reactions and purification similar to those in Reference Example 43 using 4-(trifluoromethyl)benzylamine, the title compound was obtained, yield 69%.
¹H NMR (CDCl₃) δ 2.80-2.84 (2H, m) , 3.19-3.35 (1H, m) , 3.46-3.61 (2H, m), 4.53 (2H, s), 7.36 (2H, d, J = 7.6Hz), 7.60 (2H, d, J = 7.6Hz), 7.6-8.2 (1H, br).

### Reference Example 57

### 1-(2-morpholinoethyl)-5-oxo-3-pyrrolidinecarboxylic acid

By reactions and purification similar to those in Reference Example 43 using 2-morpholinoethylamine, the title compound was obtained, yield 44%.
¹H NMR (CDCl₃+DMSO-*d*₆) δ 2.45-2.81 (8H, m), 3.13-3.76 (9H, m), 9.2-9.6 (1H, br).

### Reference Example 58

### 1-(2-furylmethyl)-5-oxo-3-pyrrolidinecarboxylic acid

By reactions and purification similar to those in Reference Example 43 using furfurylamine, the title compound was obtained, yield 63%.
mp 155-156°C (ethanol).
¹H NMR (CDCl₃) δ 2.60-2.85 (2H, m), 3.12-3.28 (1H, m), 3.51-3.68 (2H, m), 4.39 (1H, d, J = 15.4 Hz), 4.53 (1H, d, J = 15.4 Hz), 6.26 (1H, d, J = 3.6 Hz), 6.31-6.34 (1H, m), 7.36 (1H, d, J = 1.8 Hz), 8.30-10.00 (1H, br).

### Reference Example 59

### 1-(4-methylbenzyl)-5-oxo-3-pyrrolidinecarboxylic acid

By reactions and purification similar to those in Reference Example 43 using 4-methylbenzylamine, the title compound was obtained, yield 79%.
¹H NMR (CDCl₃+DMSO-*d*₆) δ 2.33 (3H, s) , 2.61-2.87 (2H, m), 3.09-3.25 (1H, m), 3.40-3.55 (2H, m), 4.34 (1H, d, J = 14.6 Hz), 4.48 (1H, d, J = 14.6 Hz), 7.12 (4H, s), 7.2-7.8 (1H, br).

### Reference Example 60

### 1-(2,6-difluorobenzyl)-5-oxo-3-pyrrolidinecarboxylic acid

By reactions and purification similar to those in Reference Example 43 using 2,6-difluorobenzylamine, the title compound was obtained, yield 62%.
¹H NMR (DMSO-*d*₆) δ 2.40-2.60 (2H, m), 3.10-3.60 (3H, m), 4.46 (2H, s), 7.05-7.16 (2H, m), 7.37-7.50 (1H, m), 12.4-12.8 (1H, br).

### Reference Example 61

### 1-benzyl-6-oxo-3-piperidinecarboxylic acid

Diethyl 2-methylenepentanedioate (Tetrahedron Lett. 1989, 30, 7381)(1.00 g, 5.0 mmol) was dissolved in ethanol (1.5 ml) and benzylamine (0.546 ml, 5.0 mmol) was added. The mixture was stirred at 60°C for 6 days. The reaction mixture was concentrated under reduced pressure and the residue was subjected to column chromatography (silica gel 25 g, ethyl acetate/hexane=1/1→1/0). The objective fraction was concentrated under reduced pressure to give ethyl 1-benzyl-6-oxo-3-piperidinecarboxylate (1.01 g, 3.9 mmol, yield 77%).
¹H NMR (CDCl₃) δ 1.22 (3H, t, J=7.2Hz), 1.85-2.25 (2H, m), 2.35-2.85 (3H, m), 3.3-3.55 (2H, m), 4.12 (2H, qd, J=7.2Hz, 2.0Hz), 4.52 (1H, d, J=14.8Hz), 4.71 (1H, d, J=14.8Hz), 7.2-7.4 (5H, m).

Ethyl 1-benzyl-6-oxo-3-piperidinecarboxylate (261 mg, 1 mmol) was dissolved in methanol (1 ml) and 1N aqueous sodium hydroxide solution (1.2 ml) was added. The mixture was stirred at room temperature for 1 h. To the reaction mixture was added 1N hydrochloric acid (1.5 ml) and the resulting precipitate was collected by filtration washed with water and dried under reduced pressure to give the title compound (200 mg, 86%).
¹H NMR (CDCl₃) δ 1.90-2.30 (2H, m), 2.43-2.90 (3H, m), 3.34-3.52 (2H, m), 4.46 (1H, d, J = 14.6 Hz), 4.77 (1H, d, J = 14.6 Hz), 7.23-7.36 (5H, m), 8.6-9.4 (1H, br).

### Reference Example 62

### N-[3-(4-benzyl-1-piperidinyl)propyl]-1-indanamine dihydrochloride

By reactions and purification similar to those in Reference Example 41 using 1-indanamine, the title compound was obtained, yield 33%.
¹H NMR (DMSO-*d*₆) δ 1.4-1.9 (6H, m) , 2.0-2.3 (3H, m) , 2.3-2.6 (2H, m), 2.6-3.6 (11H, m), 4.74 (1H, br s), 7.17-7.4 (8H, m), 7.7-7.9 (1H, m) , 9.2-9.8 (2H, br).

### Reference Example 63

### N-[3-(4-benzyl-1-piperidinyl)propyl]-1,2,3,4-tetrahydro-1-naphthylamine dihydrochloride

By reactions and purification similar to those in Reference Example 41 using 1,2,3,4-tetrahydro-1-naphthylamine hydrochloride, the title compound was obtained, yield 56%.
¹H NMR (DMSO-*d*₆) δ 1.4-3.4 (24H, m), 4.46 (1H, br s), 7.0-7.5 (8H, m), 7.71 (1H, br d, J = 6.2 Hz), 9.2-10.0 (2H, br).

### Reference Example 64

### N-{3-[4-(4-fluorobenzyl) -1-piperidinyl]propyl}aniline dihydrochloride

By reactions and purification similar to those in Reference Example 9 using 4-(4-fluorobenzyl)piperidine and aniline, the title compound was obtained, yield 54%.
mp 230°C (dec.)
¹H NMR (DMSO-*d*₆) δ 1.35-1.9 (5H, m), 1.95-2.2 (2H, m), 2.45-2.6 (2H, m), 2.83 (2H, br t, J=11.5Hz), 3.11 (2H, br t, J=7.4Hz), 3.24 (2H, br t, J=6.8Hz), 3.42 (2H, br d, J=10.6Hz), 6.9-7.2 (9H, m).
Anal. Calcd for C₂₁H₂₇FN₂·2HCl·0.8H₂O: C, 60.96; H, 7.45; N, 6.77; Cl, 17.14; F, 4.59. Found: C, 61.02; H, 7.37; N, 6.76; Cl, 17.04; F, 4.30.

### Reference Example 65

### 3,4-dichloro-N-{3-[4-(4-fluorobenzyl)-1-piperidinyl]propyl}aniline dihydrochloride

By reactions and purification similar to those in Reference Example 9 using 4-(4-fluorobenzyl)piperidine and 3,4-dichloroaniline, the title compound was obtained, yield 48%.
mp 203-209°C (dec.)
¹H NMR (DMSO-*d*₆) δ 1.35-2.05 (7H, m) , 2.45-2.6 (2H, m) , 2.6-3.3 (6H, m), 3.41 (2H, br d, J=10.6Hz), 6.57 (1H, dd, J=2.7, 8.8Hz), 6.75 (1H, d, J=2.7Hz), 7.05-7.3 (5H, m).
Anal. Calcd for C₂₁H₂₅Cl₂FN₂·2HCl·0.5H₂O: C, 52.85; H, 5.91; N, 5.87. Found: C, 52.90; H, 6.12; N, 5.94.

### Reference Example 66

### N-[3-(4-benzyl-1-piperidinyl)propyl]-3-(trifluoromethyl)aniline dihydrochloride

By reactions and purification similar to those in Reference Example 9 using 3-(trifluoromethyl)aniline, the title compound was obtained, yield 56%.
mp 167-173°C (dec.)
¹H NMR (DMSO-*d*₆) δ 1.4-2.1 (7H, m) , 2.45-2.6 (2H, m), 2.6-2.95 (2H, m), 2.95-3.3 (2H, m), 3.13 (2H, t, J=6.6Hz), 3.41 (2H, br d, J=11.6Hz), 6.75-6.95 (3H, m), 7.1-7.4 (6H, m).
Anal. Calcd for C₂₂H₂₇F₃N₂·2HCl·0.8H₂O: C, 56.97; H, 6.65; N, 6.04. Found: C, 56.87; H, 6.64; N, 6.10.

### Reference Example 67

### N-[3-(4-benzyl-1-piperidinyl)propyl]-3-methylaniline dihydrochloride

By reactions and purification similar to those in Reference Example 9 using m-toluidine, the title compound was obtained, yield 67%.
¹H NMR (DMSO-*d*₆) δ 1.4-2.25 (7H, m), 2.31 (3H, s), 2.45-3.5 (10H, m), 6.95-7.4 (9H, m).
Anal. Calcd for C₂₂H₃₀N₂·2HCl·0.2H₂O: C, 66.22; H, 8.18; N, 7.02; Cl, 17.77. Found: C, 66.30; H, 8.12; N, 6.99; Cl, 17.56.

### Reference Example 68

### N-[3-(4-benzyl-1-piperidinyl)propyl] -2-methylaniline dihydrochloride

By reactions and purification similar to those in Reference Example 9 using o-toluidine, the title compound was obtained, yield 69%.
¹H NMR (DMSO-*d*₆) δ 1.4-2.25 (7H, m), 2.32 (3H, s), 2.45-3.5 (10H, m), 6.9-7.4 (9H, m).
Anal. Calcd for C₂₂H₃₀N₂·2HCl·1.0H₂O: C, 63.91; H, 8.29; N, 6.78; Cl, 17.15. Found: C, 64.01; H, 8.18; N, 6.74; Cl, 16.93.

### Reference Example 69

### N-[3-(4-benzyl-1-piperidinyl)propyl]-4-cyanoaniline

By reactions and purification similar to those in Reference Example 9 using 4-cyanoaniline, the title compound was obtained.
¹H NMR (CDCl₃) δ 1.19-1.39 (2H, m), 1.45-1.96 (7H, m), 2.42-2.49 and 2.56-2.60 (2H and 2H, m), 2.90-2.97 and 3.15-3.24 (2H and 2H, m), 6.17-6.30 (1H, br s), 6.45 (2H, d, J=9.0Hz), 7.14-7.42 (7H, m).

### Reference Example 70

### N-[3-(4-benzyl-1-piperidinyl)propyl]-3-cyanoaniline

By reactions and purification similar to those in Reference Example 9 using 3-cyanoaniline, the title compound was obtained.
¹H NMR (CDCl₃) δ 1.20-1.40 (2H, m), 1.41-1.95 (7H, m), 2.42-2.49 and 2.56-2.60 (2H and 2H, m), 2.91-2.98 and 3.11-3.19 (2H and 2H, m), 6.68-6.74 (2H, m), 6.89-6.93 (1H, m), 7.14-7.30 (6H, m) .

### Reference Example 71

### N-[3-(2-benzyl-4-morpholino)propyl]aniline

By reactions and purification similar to those in Reference Example 9 using 2-benzylmorpholine (J. Pharm. Pharmacol. 1990, 42, 797) and aniline, the title compound was obtained.
¹H NMR (CDCl₃) δ 1.62-2.10 (4H, m), 2.45 (2H, t, J=6.6Hz), 2.61-2.93 (4H, m), 3.16 (2H, t, J=6.2Hz), 3.58-3.93 (3H, m), 6.54-6.75 (3H, m), 7.11-7.29 (7H, m).

### Experimental Example

### (1) Cloning of human CCR5 chemokine receptor

Cloning of CCR5 gene was carried out by PCR (polymerase chain reaction) from human spleen cDNA. With using 0.5 ng of spleen cDNA (Toyobo, QUICK-Clone cDNA) as template, PCR was performed in DNA Thermal Cycler 480 (Perkin-Elmer) (reaction conditions: 30 cycles of 95°C for 1 minute, 60°C for 1 minute, and 75°C for 5 minutes) by adding primer set, 5'-CAGGATCCGATGGATTATCAAGTGTCAAGTCCAA-3' (25pmol) and 5'-TCTAGATCACAAGCCCACAGATATTTCCTGCTCC-3' (25pmol), which were designed referring to nucleotide sequence of CCR5 gene reported by Samson et al. (Biochemistry, 35(11), 3362-3367 (1996)) and by using TaKaRa EX Taq (Takara Shuzo). The resultant PCR product was subjected to agarose gel electrophoresis to collect about 1.0 kb DNA fragment, which was subjected to Original TA Cloning Kit (Funakoshi) to carry out cloning of CCR5 gene.

### (2) Preparation of plasmid for expression of human CCR5

The plasmid obtained in the above was digested with restriction enzymes XbaI (Takara Shuzo) and BamHI (Takara Shuzo) and subjected to agarose gel electrophoresis to collect about 1.0kb DNA fragment. The DNA fragment was mixed with plasmid pcDNA3.1 (Funakoshi) for expression in animal cells, said plasmid being digested with XbaI and BamHI, and they were ligated with DNA Ligation Kit Ver. 2 (Takara Shuzo). The resulting plasmid was subjected to transformation of competent cell of E. coli JM109 (Takara Shuzo) to obtain plasmid pCKR5.

### (3) Introduction of plasmid for expression of human CCR5 into CHO-K1 cell and Expression of said plasmid in CHO-K1 cell

CHO-K1 cells were grown in 750ml of tissue culture flask (Becton Dickinson) using Ham's F12 medium (Nihon Pharmaceutical) containing 10% fetal calf serum (Life Tech Oriental) and took off with 0.5 g/L trypsin-0.2 g/L EDTA (Life Tech Oriental). The cells were washed with PBS (Life Tech Oriental), centrifuged (1000 rpm, 5 minutes), and suspended in PBS. With using Gene Pulser (Bio-Rad Laboratories), DNA was introduced into the cells under the conditions shown below. That is, to the cuvette of 0.4 cm gap were added 8×10⁶ cells and 10 µg of plasmid pCKR5 for expression of human CCR5, and electroporation was carried out under 0.25 kV of voltage and 960 µF of capacitance. The cells were transferred into Ham's F12 medium containing 10% fetal calf serum, and cultivated for 24 hours. The cells were again took off and centrifuged, and suspended in Ham's F12 medium containing 10% fetal calf serum and 500 µg/ml of geneticin (Life Tech Oriental). The suspension was diluted to give 10⁴ cells/ml of the suspension, which was inoculated on 96 well plate (Becton Dickinson) to give resistant cells. The resulting geneticin resistant cells were cultivated in 96 well plate (Becton Dickinson), and cells expressing CCR5 were selected from the geneticin resistant cells. That is, in assay buffer (Ham's F12 medium containing 0.5% BSA and 20 mM HEPES (Wako Pure Chemical, pH 7.2)) to which was added 200 pM of [¹²⁵I]-RANTES (Amersham) as ligand, binding reaction was carried out at room temperature for 40 minutes, and the buffer was washed with cooled PBS. To the buffer was added 50 µl/well of 1M NaOH, and the mixture was stirred. Radioactivity was determined with γ-counter to select CHO/CCR5 cells which specifically bind to the ligand.

### (4) Evaluation of Test Compounds based on CCR5 antagonistic activity

The CHO/CCR5 were inoculated on 96 well microplate (5×10⁴ cells/well) and cultivated for 24 hours. The medium was removed by means of suction, and to each well was added assay buffer containing Test Compound (1 µM) and then 100 pM of [¹²⁵I]-RANTES (Amersham) as ligand. Binding assay was carried out at room temperature for 40 minutes, and assay buffer was removed by means of suction. Each well was washed twice with cooled PBS, and 200 µl of Microscint-20 (Packard Instrument, Inc.) was added to each well. Radio-activity was determined with Top-Count (Packard Instrument, Inc.).

According to the method described above, inhibition rate of Test Compound to CCR5 binding.

The results are shown in Table 1.

**Table 1**

| Example No. | Inhibitory rate (%) at 1.0 µM |
|---|---|
| 1 | 57 |
| 8 | 24 |
| 13 | 40 |
| 17 | 22 |
| 23 | 95 |
| 38 | 82 |
| 51 | 92 |
| 52 | 76 |
| 62 | 67 |
| 76 | 91 |
| 84 | 92 |
| 93 | 90 |

A CCR5 antagonist (e.g., an agent for the prophylaxis and treatment of HIV infectious diseases, an agent for the prophylaxis and treatment of AIDS etc.) containing the compound (I) of the present invention as an active ingredient can be produced to have, for example, the following formulations.

### Formulation Example

### 1. capsules

(1) Compound obtained in Example 51 40 mg
(2) Lactose 70 mg
(3) Microcrystalline cellulose 9 mg
(4) Magnesium stearate 1 mg
1 capsule 120 mg

(1), (2), (3), 2/3 of (4) and 1/2 of (5) are mixed and then granulated. To the granules are added the remainders of (4) and (5), followed by subjecting the mixture to compression molding.

### 2. tablets

(1) Compound obtained in Example 51 40 mg
(2) Lactose 58 mg
(3) Corn starch 18 mg
(4) Microcrystalline cellulose 3.5 mg
(5) Magnesium stearate 0.5 mg
1 tablet 120 mg

(1), (2), (3) and 1/2 of (4) are mixed and then granulated. To the granules is added the remainder of (4), and the whole is filled into a gelatin capsule.

### Industrial Applicability

The compound of the formula (I) and a salt thereof of the present invention have a superior CCR5 antagonistic activity. Therefore, they can be advantageously used for the prophylaxis and treatment of various HIV infectious diseases in human, such as AIDS.

## Claims

1. A compound of the formula: 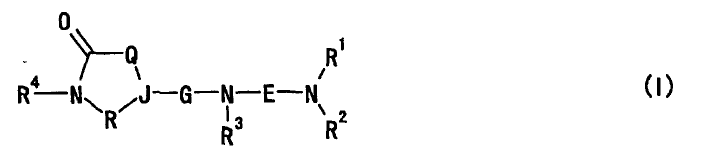 wherein
R¹ is a hydrocarbon group;
R² is a hydrocarbon group having 2 or more carbon atoms, or R¹ and R² may in combination form, together with an adjacent nitrogen atom, a ring optionally having a substituent or substituents;
R³ is a hydrocarbon group optionally having a substituent or substituents or a heterocyclic group optionally having a substituent or substituents;
R⁴ is a hydrogen atom, a hydrocarbon group optionally having a substituent or substituents or a heterocyclic group optionally having a substituent or substituents;
E is a divalent chain hydrocarbon group optionally having a substituent or substituents other than an oxo group;
G is CO or SO₂;
J is a nitrogen atom or a methine group optionally having a substituent or substituents; and
Q and R are each a bond or a divalent chain C₁₋₃ hydrocarbon group optionally having a substituent or substituents,
or a salt thereof.

2. The compound of claim 1, wherein R¹ is a C₁₋₆ alkyl group or a C₃₋₈ cycloalkyl group; R² is a C₂₋₆ alkyl group or a C₃₋₈ cycloalkyl group, or R¹ and R² in combination form, together with an adjacent nitrogen atom, a ring optionally having a substituent or substituents; R³ is a C₁₋₆ alkyl group optionally having a substituent or substituents, a C₃₋₈ cycloalkyl group optionally having a substituent or substituents, an aryl group optionally having a substituent or substituents or a heterocyclic group optionally having a substituent or substituents; R⁴ is a hydrogen atom, alkyl group optionally having a substituent or substituents, a C₃₋₈ cycloalkyl group optionally having a substituent or substituents, an aryl group optionally having a substituent or substituents or a heterocyclic group optionally having a substituent or substituents; E is a C₂₋₅ alkylene group optionally having a substituent or substituents other than oxo group; G is CO or SO₂; J is a nitrogen atom or a methine group optionally having a substituent or substituents; and Q and R are each a bond or a C₁₋₃ alkylene group optionally having a substituent or substituents.

3. The compound of claim 1 or claim 2, wherein R¹ and R² in combination form, together with an adjacent nitrogen atom, a ring optionally having a substituent or substituents.

4. The compound of claim 3, wherein the ring optionally having a substituent or substituents is a 1-piperidinyl group or a 1-piperazinyl group each optionally having a substituent or substituents.

5. The compound of claim 4, wherein the substituent of the 1-piperidinyl group or 1-piperazinyl group is (1) phenyl-C₁₋₄ alkyl optionally having halogen on a benzene ring, (2) diphenylmethyl optionally having hydroxy, (3) benzoyl optionally having halogen on a benzene ring, (4) 2-phenylethen-1-yl, (5) phenyl optionally having halogen, (6) hydroxy, (7) phenoxy or (8) benzyloxy.

6. The compound of claim 3, wherein the ring optionally having a substituent or substituents is a 1-piperidinyl group optionally having a substituent or substituents.

7. The compound of claim 6, wherein the substituent of the 1-piperidinyl group is a benzyl group optionally having halogen on a benzene ring.

8. The compound of claim 1 or claim 2, wherein R³ is (1) a C₁₋₆ alkyl group, (2) a C₃₋₈ cycloalkyl group, (3) a benzyl group optionally having a hydroxy group, (4) a naphthylmethyl group, (5) a phenyl group optionally having, as a substituent, (a) C₁₋ ₄ alkyl optionally having halogen, (b) C₁₋₄ alkoxy optionally having halogen, (c) phenyl, (d) cyano, (e) benzyloxy or (f) a halogen atom, (6) a naphthyl group, (7) an indanyl group or (8) a tetrahydronaphthyl group.

9. The compound of claim 1 or claim 2, wherein R³ is a phenyl group optionally having, as a substituent, C₁₋₄ alkyl or halogen.

10. The compound of claim 1 or claim 2, wherein E is C₂₋₆ polymethylene optionally having hydroxy.

11. The compound of claim 1 or claim 2, wherein R⁴ is (1) a hydrogen atom, (2) C₁₋₆ alkyl optionally having (a) halogen, (b) pyridyl, (c) morpholino, (d) furyl, (e) ethynyl or (f) C₃₋₈ cycloalkyl, (3) phenyl-C₁₋₄ alkyl optionally having (a) halogen, (b) C₁₋₄ alkyl, (c) halogeno-C₁₋₄ alkyl or (d) C₁₋₄ alkoxy on a benzene ring, or (4) C₃₋₈ cycloalkyl.

12. The compound of claim 1 or claim 2, wherein R⁴ is (a) C₁₋₄ alkyl group optionally having, as a substituent, halogen or furyl or (b) a benzyl group optionally having halogen on a benzene ring.

13. The compound of claim 1, wherein -N(R¹)R² is a 1-piperidinyl group optionally having a substituent or substituents, E is a trimethylene group , R³ is a phenyl group optionally having a substituent or substituents, G is CO, J is CH, and Q and R are each a methylene group.

14. A compound selected from *N*-[3-(4-benzyl-1-piperidinyl)propyl]-*N*-(3,4-dichlorophenyl)-1-methyl-5-oxo-3-pyrrolidinecarboxamide, 1-benzyl-*N*-[3-(4-benzyl-1-piperidinyl)propyl]-5-oxo-N-phenyl-3-pyrrolidinecarboxamide, 1-(2-chlorobenzyl)-*N*-[3-(4-benzyl-1-piperidinyl)propyl]-5-oxo-*N*-phenyl-3-pyrrolidinecarboxamide, *N*-{3-[4-(4-fluorobenzyl)-1-piperidinyl]propyl}-*N*-(3,4-dichlorophenyl)-1-methyl-5-oxo-3-pyrrolidinecarboxamide and *N*-[3-(4-benzyl-1-piperidinyl)propyl]-5-oxo-*N*-phenyl-1-(2,2,2-trifluoroethyl)-3-pyrrolidinecarboxamide, or a salt thereof.

15. A prodrug of the compound of claim 1.

16. A pharmaceutical composition containing the compound of claim 1 or a prodrug thereof.

17. The composition of claim 16, which is a chemokine receptor antagonist.

18. The composition of claim 16, which is a CCR5 antagonist.

19. The composition of claim 16, which is an agent for the prophylaxis or treatment of HIV infectious diseases.

20. The composition of claim 16, which is an agent for the prophylaxis or treatment of AIDS.

21. The composition of claim 16, which is an agent for suppressing the progress of a disease state of AIDS.

22. The composition of claim 19, which further contains a protease inhibitor and/or a reverse transcriptase inhibitor in combination.

23. The composition of claim 22, wherein the reverse transcriptase inhibitor is zidovudine, didanosine, zalcitabine, lamivudine, stavudine, abacavir, nevirapine, delavirdine or efavirenz.

24. The composition of claim 22, wherein the protease inhibitor is saquinavir, ritonavir, indinavir, amprenavir or nelfinavir.

25. Use of the compound of claim 1 or a prodrug thereof, and a protease inhibitor and/or a reverse transcriptase inhibitor for the prophylaxis or treatment of HIV infectious diseases.

26. A method for producing a compound of the formula: 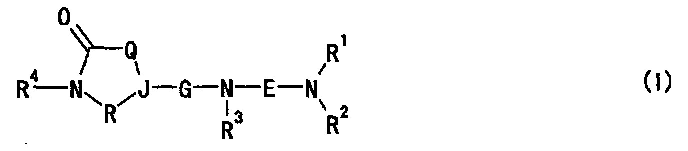 wherein
R¹ is a hydrocarbon group;
R² is a hydrocarbon group having 2 or more carbon atoms, or R¹ and R² may in combination form, together with an adjacent nitrogen atom, a ring optionally having a substituent or substituents;
R³ is a hydrocarbon group optionally having a substituent or substituents or a heterocyclic group optionally having a substituent or substituents;
R⁴ is a hydrogen atom, a hydrocarbon group optionally having a substituent or substituents or a heterocyclic group optionally having a substituent or substituents;
E is a divalent chain hydrocarbon group optionally having a substituent or substituents other than an oxo group;
G is CO or SO₂;
J is a nitrogen atom or a methine group optionally having a substituent or substituents; and
Q and R are each a bond or a divalent chain C₁₋₃ hydrocarbon group optionally having a substituent or substituents,
or a salt thereof, which method comprises reacting a compound of the formula: 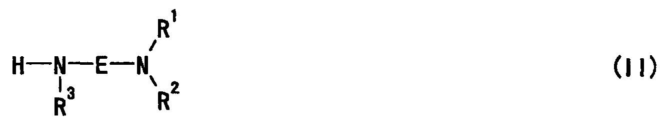 wherein each symbol is as defined above, or a salt thereof, and a compound of the formula: 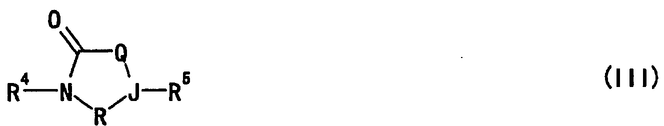 wherein R⁵ is a carboxyl group or a sulfonic acid group, a salt thereof or a reactive derivative thereof, and other symbols are as defined above, or a salt thereof.

27. A method for producing a compound of the formula: 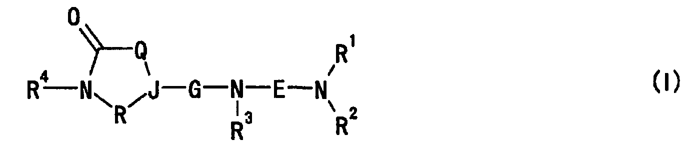 wherein
R¹ is a hydrocarbon group;
R² is a hydrocarbon group having 2 or more carbon atoms, or R¹ and R² may in combination form, together with an adjacent nitrogen atom, a ring optionally having a substituent or substituents;
R³ is a hydrocarbon group optionally having a substituent or substituents or a heterocyclic group optionally having a substituent or substituents;
R⁴ is a hydrogen atom, a hydrocarbon group optionally having a substituent or substituents or a heterocyclic group optionally having a substituent or substituents;
E is a divalent chain hydrocarbon group optionally having a substituent or substituents other than an oxo group;
G is CO or SO₂;
J is a nitrogen atom or a methine group optionally having a substituent or substituents; and
Q and R are each a bond or a divalent chain C₁₋₃ hydrocarbon group optionally having a substituent or substituents,
or a salt thereof, which method comprises reacting, in the presence of a base, a compound of the formula: 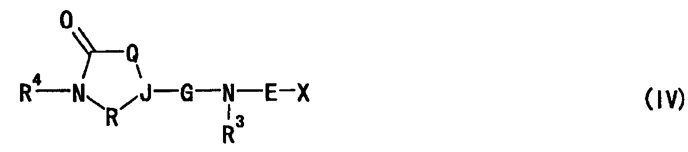 wherein X is a leaving group, and other symbols are as defined above, or a salt thereof and a compound of the formula: 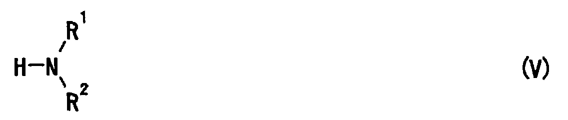 wherein each symbol is as defined above, or a salt thereof.

28. A method for suppressing a chemokine receptor activity, which method comprises administering an effective amount of the compound of claim 1 to a mammal.

29. Use of a compound of claim 1 for the production of a pharmaceutical agent that suppresses a chemokine receptor activity.
